(19)

Europäisches
Patentamt

European
Patent Office

Office européen
des brevets

(11)  EP 3 838 288 A1

(12)  **EUROPEAN PATENT APPLICATION**

(43) Date of publication:
23.06.2021  Bulletin 2021/25

(21) Application number: 20209411.6

(22) Date of filing: 11.06.2015

(51) Int Cl.:
*A61K 38/20* (2006.01)  *A61K 38/21* (2006.01)
*A61K 39/00* (2006.01)  *A61K 35/14* (2015.01)
*A61K 35/26* (2015.01)  *C12N 5/07* (2010.01)
*G01N 33/50* (2006.01)  *A61P 35/00* (2006.01)
*A61P 37/00* (2006.01)  *A61P 31/00* (2006.01)
*C12N 5/0783* (2010.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority:  11.06.2014  DE 102014211167

(62) Document number(s) of the earlier application(s) in
accordance with Art. 76 EPC:
15737995.9 / 3 154 567

(71) Applicant: polybiocept GmbH
63755 Alzenau (DE)

(72) Inventor: **Mäurer, Markus**
**18444 Äkersberga (SE)**

(74) Representative: **Patent- und Rechtsanwälte**
**Ullrich & Naumann**
**PartG mbB**
**Schneidmühlstrasse 21**
**69115 Heidelberg (DE)**

Remarks:
This application was filed on 24.11.2020 as a
divisional application to the application mentioned
under INID code 62.

(54)  **EXPANSION OF LYMPHOCYTES WITH A CYTOKINE COMPOSITION FOR ACTIVE CELLULAR IMMUNOTHERAPY**

(57)  The present invention relates to a composition for expanding lymphocytes comprising at least two types of cytokines selected from interleukin 2 (IL-2), interleukin 15 (IL-15) and interleukin 21 (IL-21). It further relates to a Method of preparing a population of clinically relevant lymphocytes, comprising the steps of: obtaining a body sample from a mammal in particular a tissue sample or body liquid sample, comprising at least one lymphocyte and optionally separating the cells in the body sample, culturing the body sample *in-vitro* to expand and/or stimulate lymphocytes in the sample wherein the culturing comprises using IL-2, IL-15 and/or IL-21, and optionally determining the presence of clinically relevant lymphocyte in the cultured sample. The present invention also relates to an immunotherapy and the population of clinically relevant lymphocytes.

EP 3 838 288 A1

**Description**

FIELD OF THE INVENTION

The present invention relates to active cellular immunotherapy including a method of preparing a population of clinically relevant lymphocytes using a composition of predefined cytokines. The invention further relates to the composition of cytokines and the generated clinically relevant lymphocytes.

BACKGROUND OF THE INVENTION

Cancer remains to be one of the most common causes of death in the developed countries. For example in the United States and Germany it is the second most common cause of death with a mortality of 560,000 (2009) and 218,000 (2010), respectively. Survival rates remain poor for many cancers despite improvement in the ability to detect and treat this constellation of diseases.

Among the cancer diseases, pancreatic cancer is the fourth leading cause of cancer death in the United States and Sweden without signs of improvement. By the time of diagnosis of pancreatic cancer the majority of patients is incurable with a locally advanced cancer or metastatic disease only allowing for a palliative treatment. The medium survival is about six months. Only about 15 to 20 % of the patients have a resectable tumor and therefore a potentially curable disease. Like most cancer, pancreatic cancer is a systemic disease that requires early and systemic intervention. In comparison with many other types of cancer, pancreatic cancer is highly chemotherapy resistant and radiation resistant. In order to achieve a significant breakthrough in improving the dismal prognosis of pancreatic cancer finding new alternatives and more efficient treatment concepts is imperative. The biology of pancreatic cancer is associated with both local and systemic immunosuppression enabling tumor progression and metastasizing.

The situation is similar for glioblastoma, which is the most frequent and progressive glioma with an incidence of 2-3/100.000 in the United States. Glioblastoma make up 12 to 15 % of all intracranial and 50 to 60 % of histiocytic tumors. New treatment regimens have increased a medium overall survival (14.6 months with radio plus temozolomide as compared to 12.1 months with radio therapy alone). So far intents to develop robust and clinically effective immunotherapeutic protocols have been frustrating for patients with glioblastoma or patients with pancreatic cancer. One approach for treating such cancers is to overcome the tumor induced suppression and/or to induce anti-tumor directed cellular and humoral immune responses.

One of the most promising advances is a new therapeutic class called active cellular immunotherapy (ACI). Cancer immunotherapies can be either passive or active. Passive therapy is based on the adoptive transfer of immunomodulators including cytokines, tumor specific antibodies or immune cells. These substances or cells are then administered to the patient to initiate an anti-tumor action. In general these therapies do not generate immunologic memory and therefore require chronic infusion based treatment. Active immunotherapies, on the other hand, stimulate the patient's immune system with the intent of promoting an antigen specific anti-tumor effect using the body's own immune cells. In addition active immunotherapies seek to create durable anti-tumor response that can protect against minimal residual disease and tumor recurrences.

Clinically relevant and a long-term remission using T-cells directed against tumors (tumor reactive T-cells) have been achieved in patients with melanoma (2, 3). A landmark article showed recently that the best and durable response in cancer treatment is achieved if the patient's own T-cells are directed against the patient's own tumor cells, i.e. the patient's own "private" mutations (4). Such promising results were also obtained for patients with epithelial tumors, i.e. by adaptive transfer of T-cells targeting mutant epitopes in epithelial cancer (5). These approaches usually rely on the harvesting of tumor infiltrating lymphocytes (TIL) from tumor lesions or T-cells from peripheral blood.

A recent report of the CTEP Subcommittee on Adaptive Cell Therapy summarized protocols on the expansion of tumor reactive T-cells from peripheral blood and TILs.

This study compiled the roadmap for using TIL therapy or T-cell based therapy with a particular focus on product consistency and effective yield of the T-cell products. Both consistency and yield for anti-melanoma directed T-cells appear to be achievable with current methodologies that would enable T-cell based strategies to enter the mainstream of cancer treatment, along with the biologicals, i.e. anti-CD40L or PD-1 directed therapies.

Minimally cultured TIL appears to provide the most effective phenotype and profile for clinical use (11). The most successful approach up to date has been the use of autologous ex vivo activated T-cells that were grown in 24 well plates, tested for immune effector functions and further expansion using IL-2, allogeneic feeder cells and OKT3 (9, 12, 13).

CD4+ or CD8+ tumor antigen (TAA) directed T-cells have been generated under GMP conditions from peripheral blood and formulated to subsequent treatment of patients. This has been achieved with autologous CD4+ T-cells (14-16) or CD8+ T-cells(17), some directed against the NY-ESO-1 antigen(18), which is also possible in PBMCs from healthy patients without cancer, since sufficient T-cell precursors are present in the peripheral circulation. Various methods for expansion of T-cells generating CD8+ T-cell clones for target directed therapy (19) have been described. This if great interest since clonal repopulation of the patient's immune system with anti-tumor lymphocytes has been shown to induce cancer regression but also autoimmunity (20).

The growth medium formulation may also be relevant for successful active immunotherapy. Studies showed that starvation impacts on T-cell mediated immune responses and may induce starvation- induced immunosuppression, yet also the expansion of certain T-cell subsets This mechanism appears to be mediated by Leptin (21) which modulates also B-cell development and subsequent B-cell responses (22). This lead to the discovery of the nutrient sensor pathways (i.e. the GCN2 in dendritic cells) that enhances antigen presentation (23). More recent studies showed that cytokine-driven T-cell expansion (such as those in *ex vivo* expansion of TIL or ACT) are dependent on exogenous amino acids and that cytokines, i.e. IL-7, upregulated genes associated with amino acid transporter expression. Tailoring growth media requirements will therefore be shaped by the respective cytokine cocktail used for T- cell expansion (24) as well as for amino acids in the medium; both factors will impact on T-cell maturation and differentiation which is clinically meaningful.

Clinical (antitumor) efficacy appears to be mediated by CD8+ and central memory cells, defined by CD45RA-CCR7+, defined ex vivo from patients responding to T-cell based therapy. The phenotype of such T-cells is determined by the ex vivo expanded T-cell population, as well as by host factors after adoptive transfer. A diverse population of T-cells targeting cancer cells may be advantageous for effective immune responses, including long-term memory T-cells, yet also T-cells that can immediately react to (cancer) target cells and produce anti-tumor directed immune responses, including terminally differentiated T-cells that express cytolytic molecules, such as granzyme and perforin (25, 26). Long term-memory immune memory is in part determined by the increased proliferation potential and half-life that can be measured by the telomere length (27, 28).

Relatively little is known about what stages of (melanoma)-specific TIL or T-cell clones are best for *in vivo* transfer, due to gene expression differences in vitro and in vivo, as well as the differential cytokine milieu in individual patients. Not only an individual phenotype, and also rather different phenotypes associated with the fast delivery of immune effector functions (terminally differentiated CD45RA+CCR7-) T-cells along with the provision of long-term immunologically memory of central memory T-cells that would replenish the differentiated T-cell pool could represent a good choice for expansion of T-cells.

Similarly relevant appear to be the expression of activation/exhaustion markers, i.e. LAG-3, PD-1 and or 4-1BB on T-cells that may indicate a higher change for T-cell exhaustion and loss of function, and also an enrichment of tumor-antigen specific T-cells (which tend to be PD1+, and/or LAG-3, 4-1BB+ (29)).

NY-ESO-1 was known to be an eligible target for tumor- antigen specific T-cells. NY-ESO-1 is a cancer testing antigen (36, 37) and expressed in a high number of tumors. For example, at Karolinska 50 glioblastoma lesions were screened for NY-ESO-1 protein expression and it was found that 35% of the grade 3 and 4 GB are positive for NY-ESO-1. Screening of pancreatic cancer lesions showed a lower number of NY-ESO-1 protein + cancer lesions in the range of 20%, particularly in metastatic lesions. Targeting NY-ESO-1 for the expansion of tumor-reactive T-cells from peripheral blood appears to be a 'safe choice' of target, since NY-ESO-1 appears to be expressed only in malignant cells and testis without overt 'off-target' reactivity in anti-NY-ESO-1 directed T-cells (36). This is of great interest since clonal repopulation of the patients immune system with antitumor lymphocytes has been shown to induce cancer regression, and autoimmunity(20), a potential risk. NYESO-1 has been tested in a number of studies as a potential target in GB, as well as in GB-stem cells (38), along with the use of DNA- methylating agents to increase NY-ESO-1 reactivity (39, 40).

In view of this state of the art it is an objective of the present invention to provide improved methods in immunotherapy.

SUMMARY OF THE INVENTION

The present invention is *inter alia* based on the finding that a composition comprising cytokines interleukin-2 (IL-2), interleukin-15 (IL-15) and/or interleukin-21 (IL-21) leads to a superior stimulation and expansion of lymphocytes in particular clinically relevant lymphocytes. The expansion and stimulation procedure with the cytokine mixture is highly sensitive and allows the preparation of the population of clinically relevant lymphocytes even if the starting concentration in the sample is very low.

Thus, according to a first aspect the invention provides a composition for expanding lymphocytes comprising at least two types of cytokines selected from interleukin 2 (IL-2), interleukin 15 (IL-15) and interleukin 21 (IL-21).

With this cytokine composition the inventors were able to determine a novel method for the preparation of population of antigen edited lymphocytes. Therefore according to a second aspect the present invention provides a method of preparing a population of clinically relevant lymphocytes, comprising the steps of:

- obtaining a body sample from a mammal, in particular a tissue sample or body liquid sample, comprising at least one lymphocyte and optionally separating the cells in the body sample,

- culturing the body sample *in-vitro* to expand and/or stimulate lymphocytes in the sample, wherein the culturing comprises using IL-2, IL-15 and/or IL-21,
- and optionally determining the presence of clinically relevant lymphocyte in the cultured sample.

The method of the second aspect of the invention leads to the formation of population of lymphocytes, which includes a population of clinically relevant lymphocytes.

According a third aspect, the invention provides a clinically relevant lymphocyte obtained by the method according to the second aspect wherein the clinically relevant lymphocyte is selected from a B-cell, an NK cell and T-cell.

According to a fourth aspect the present invention provides a population of lymphocytes obtained by the second aspect of the invention comprising a population of clinically relevant lymphocytes.

The population of clinically relevant lymphocytes obtained with the method according to the second aspect of the invention is in particular advantageous for cellular immunotherapy.

According to a fifth aspect the invention provides an immunotherapy for treating or preventing a tumor disease, an infectious disease or an autoimmune disease in a mammal comprising the steps of generating a clinically relevant lymphocyte population according to the second aspect of the invention wherein the body sample is obtained from said mammal and administering the clinically relevant lymphocyte population to said mammal.

According to a sixth aspect the invention provides a composition according to the first aspect of the invention for use in the medical treatment, in particular for treating and preventing an infectious disease, an autoimmune disease or a tumor disease.

Therefore, according to a seventh aspect the invention provides a kit for use in medical treatment, in particular for treating or preventing an infectious disease, an autoimmune disease or a tumor disease, wherein the kit comprises IL-2, IL-15 and IL-21, and optionally at least one of a component that stimulates the TCR, in particular OKT3, costimulatory molecules, feeder cells and a peptide comprising the amino acid sequence of at least clinically relevant antigen

FIGURES

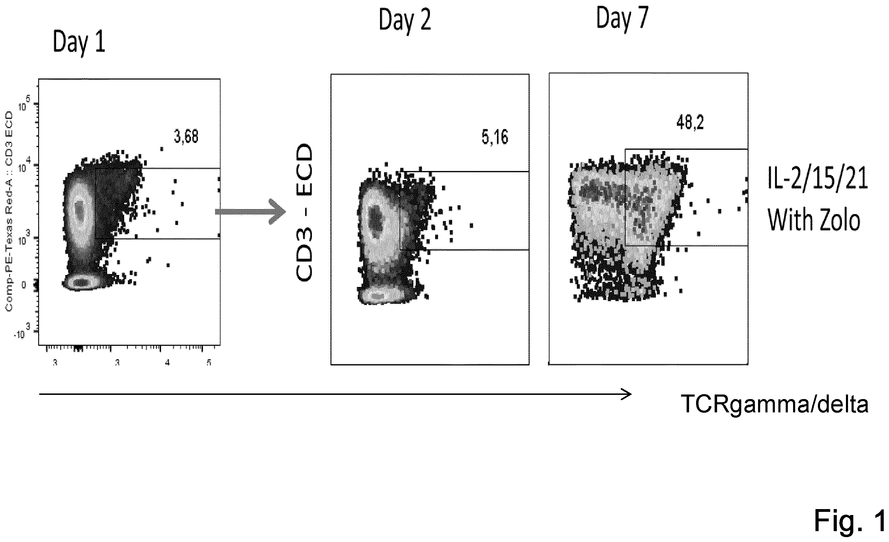

Fig. 1   shows three graphs representing the results of flow cytometry analysis of samples from an expansion of PBMCs with the cytokine cocktail IL-2, IL-15 and IL-21 in combination zoledronic acid. The samples were taken at different time points as indicated above the graphs. The measured signals are a CD3 signal in direction of the y-axis and a TCR gamma delta signal in the direction of the x-axis. Gamma delta T-cells are found in the rectangle area. The originally colored images show intensities of overlapping signals in grey scale. The percentage of cells in the rectangle area is shown above.

Fig. 2   shows the result of a flow cytometry analysis of lymphocytes from PBMCs expanded with the cytokine cocktail in the presence of PRDM2 peptides. The larger left panel shows the result of the sample at the beginning of the lymphocyte expansion, the right panel the result of the sample after 18 days of stimulation. Cell signals are separated based on the CD4/CD8 markers. The small panels on the right show the gating for lymphocytes and CD3+ cells.

Fig. 3   shows the flow cytometry analysis of the same samples as Fig. 2 Separation of the cell signals via IFN-y marker and size (side-scattered light SSC).

Fig. 4   shows the results of a flow cytometry on samples of an expansion of PBMCs with a cytokine cocktail and stimulation with INO80E and UCHL3. The cell signals are gated for lymphocytes (4a), CD3+ (4b) and then separated based on the CD8 and CD4 signal (4c).

Fig. 5   shows the IFN-y production on the double negative and CD8+ population after stimulation with INO80E or UCHL3.

Fig. 6   shows the analysis of PBMC cells expanded with the cytokine cocktail and INO80E and UCHL3 peptides. Cells stimulated with INO80E were analyzed for the production of cytokines CD107a (6d), CD127 (6e) and CD117 (6f).

Fig. 7a   to 7f show the results of the expansion of PBMCs with the cytokine cocktail and stimulation with CMVpp65.

Fig. 8   shows results of IFN-y analysis after the stimulation of expanded cells with NY-ESO-1: Fig. 8a and 8c unstimulated on day 0 and day 18, respectively. Fig. 8b and 8d stimulated with NY-ESO-1 on day 0 and day 18, respectively.

Fig. 9   shows the cytokine production of cells expanded from PBMCs obtained from a patient with glioblastoma upon stimulation with survivin again on day 0 and day 18. The measured cytokines are IL-2, IFN-$\gamma$ and TNF-$\alpha$. Fig. 9a shows the results for the subset of CD4+ T-cells, Fig. 9b for the subset of double negative T-cells and Fig. 9c for the subset of CD8+ T-cells.

Fig. 10   shows the analysis of the phenotypes CD45RA and CCR7 of lymphocytes using flow cytometry. Again lymphocytes are measured on day 0 and after 18 days of expansion with the cytokine cocktail.

Fig. 11   shows the analysis of the effect of expansion on the phenotypes of CD4+ cells (TH1 / TH2) and CD8+ T-cells.

Fig. 12   shows the analysis of cytokine CD107a expression by cells expanded from peripheral blood of a patient with HPV. Fig. 12a shows the CD107a expression upon HPV L1 peptide stimulation, Fig. 12b the positive control and Fig. 12c represents the result without stimulation (medium). The gating process for CD8+ T-cells is shown in Fig. 12d to 12f.

Fig. 13   shows two graphs representing the IFN-y production of lymphocytes expanded with either no cytokine, IL-2, IL-15, IL-21 or IL-7 and IL-2 and stimulation with NY-ESO-1 or survivin.

Fig. 14   shows three graphs representing the IFN-y production of lymphocytes expanded with either no cytokine, IL-2, IL-15, IL-21 or IL-7 and IL-2 and stimulation with EBNA-1, EBNA-3a, or CMVpp65.

Fig. 15   shows a flow cytometry analysis determining $T_{reg}$ (regulatory T-cells) were identified prior to and after expansion of T-cells with the cytokine cocktail.. From left to right: T-cells were gated on CD4+ T-cells and then on CD25high designating the high expression of the IL-2 receptor on activated T-cells. Then the cells were gated on II-2R (high CD125) cells and tested for expression of the IL-7Receptor (CD127) and Foxp3 (intracellularly).

Fig 16   shows a flow cytometry analysis determining the percentage of PD-1+ T-cells in the CD8+ subset.

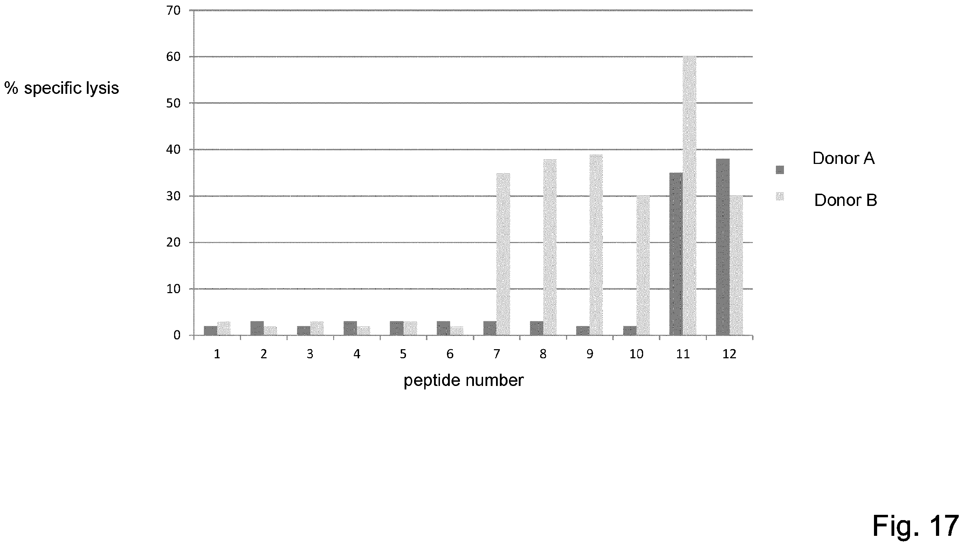

Fig. 17   shows the specific lysis of autologous B-cells pulsed with and pulsed with peptides 1-12 by expanded lymphocytes.

Fig 18   shows a flow cytometric analysis of PBMCs prior to IL-2/IL-15/IL-21 driven expansion in the presence of the tumor -associated antigen NY-ESO-1. First, CD3+ T-cells are gated, then the CD3+ T-cells are gated on CD4+ and CD8+ T-cells.

Fig 19   shows a flow cytometric analysis of PBMCs prior to IL-2/IL-15/IL-21 driven expansion in the presence of the tumor -associated antigen NY-ESO-1. First, CD3+ T-cells are gated, then the CD3+ T-cells are gated on CD4+ and CD8+ T-cells.

Fig. 20   shows images of tumor-infiltrating cultured in-vitro with cytokines IL-2, IL-15, and IL-21 lymphocyte culture of the one week of incubation.

Fig. 21   shows an overview of the functional scheme of the cytotoxicity assay of expanded lymphocytes against autologous tumor cells using radioactivity (Cr 51) labelling and release.

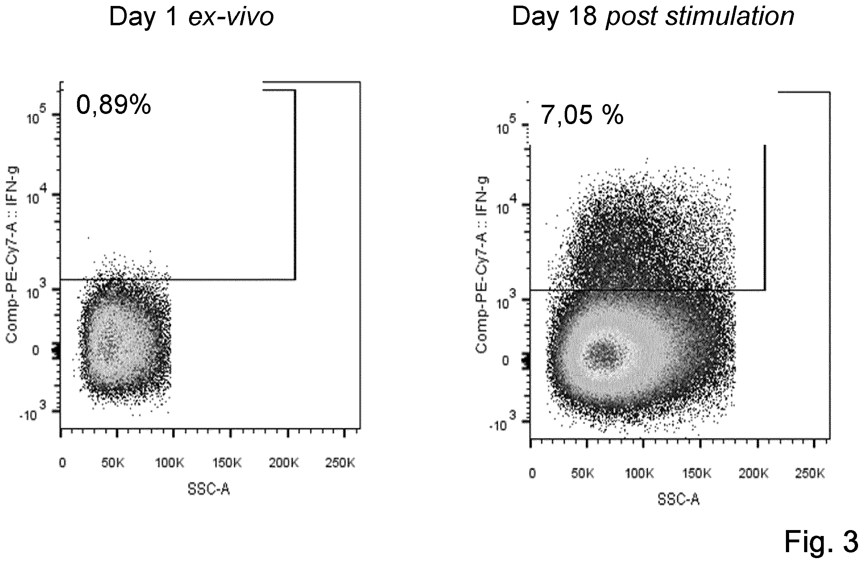

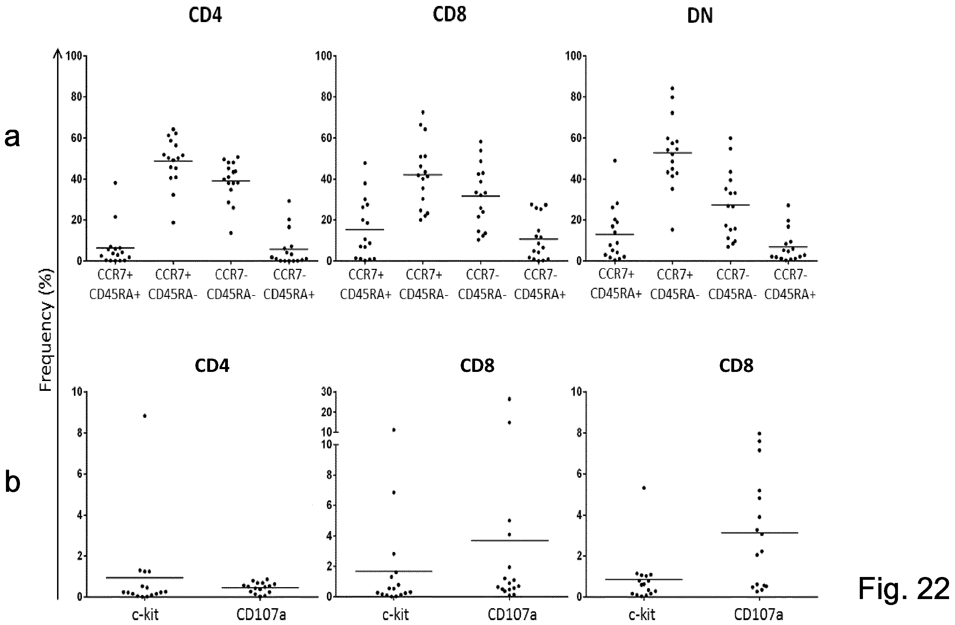

Fig. 22   shows the results of a flow cytometric analysis of lymphocytes expanded from TIL obtained from patients with Glioblastoma. Figure 3 (A) shows the distribution of T-cell phenotypes in expanded TIL from 16 TIL into specific phenotypes: precursor (CD45RA+CCR7+), central memory (CD45RA-CCR7+), peripheral memory (CD45RA-CCR7-), and differentiated effector (CD45RA+CCR7-) T-cells separately for base phenotypes CD8+ (left panel), CD4+ (right panel) and double negative T-cells (right panel). The individual data points represent the percentage of the specific phenotype based on the base phenotype. The data shows that IL-2, IL-15 and IL-21 expand TIL with a long-term memory phenotype, as well as T-cell precursors - that may provide long-term immune protection. Fig. 22 (B) shows the expression of T-cell activation and exhaustion markers. The results are grouped like in (A) according to the base phenotype CD8+ (left panel), CD4+ (right panel) and double negative T-cells (right panel). The individual data points represent the percentage of cells expressing the marker indicated on the X-axis based on the base phenotype. CD117 (c-kit) is a 'stem-cell' associated marker and designates T-cells with a long-term memory, CD107a represents a marker for recent T-cell degranulation. The data show that TIL expanded in IL-2, IL-15 and IL-21 express markers (e.g. c-kit) that enables them for long-term immune cell memory and immune-surveillance.

Fig. 23    shows the results of a flow cytometric analysis of lymphocytes (TIL) expanded from tumor tissue of patients with pancreas cancer. The left panel shows the distribution of CD4+ T-cells into precursor (CD45RA+CCR7+), central memory (CD45RA-CCR7+), peripheral memory (CD45RA-CCR7-), and differentiated effector (CD45RA+CCR7-). The right panel the distribution of CD 8+ cells. The data shows that IL-2, IL-15 and IL-21 expand TIL with a long-term memory phenotype, as well as T-cell precursors - that may provide long-term immune protection.

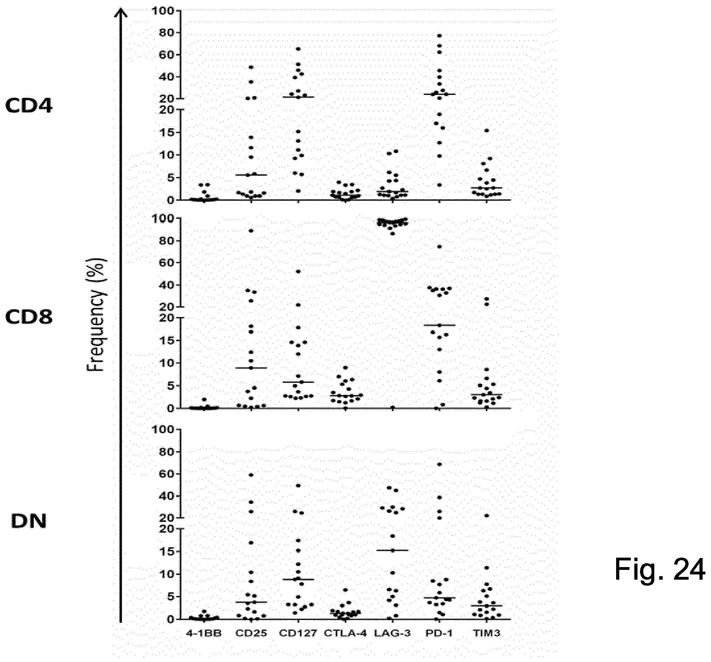

Fig. 24    shows the results of a flow cytometric analysis of lymphocytes (TIL) expanded from tumor tissue of patients with pancreas cancer with respect to T-cell activation and exhaustion markers (4-1BB, LAG-3, TIM-3 et seq.). The results are grouped according to the CD4+/CD8+ phenotypes CD4+ (upper panel), CD 8+ (middle panel), DN (lower panel). The individual data points represent the percentage of cells expressing the marker indicated on the X-axis based on the base phenotype. The data show TIL that express a wide range of markers indicative for strong anti-tumor responses and recent antigen-exposure. The CD127 molecule (IL-7R) mediates strong T-cell survival factors.

Fig. 25    shows the TCR length distribution of T-cells expanded from tumor tissue of patients with pancreatic cancer determined by a PCR-based approach.

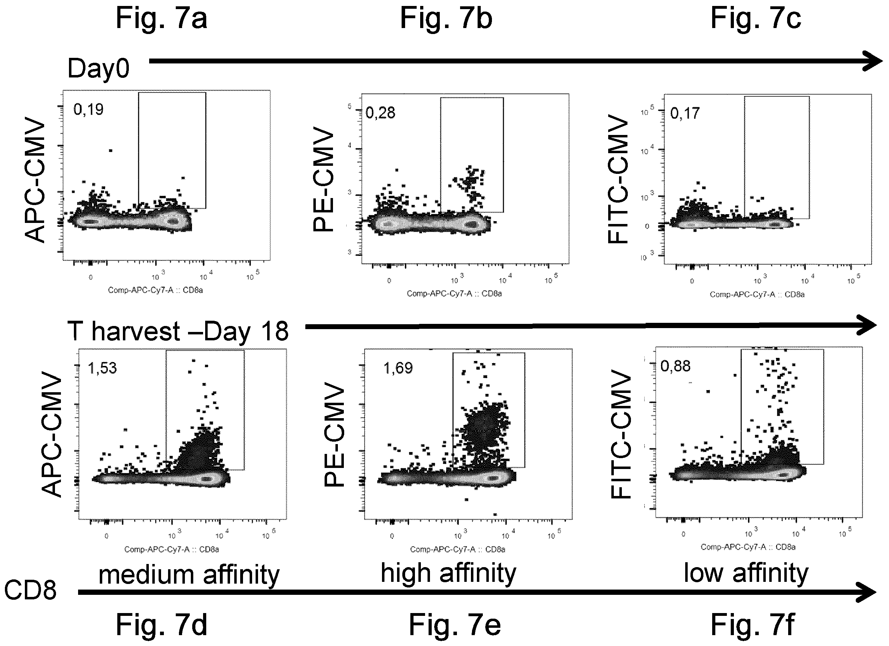

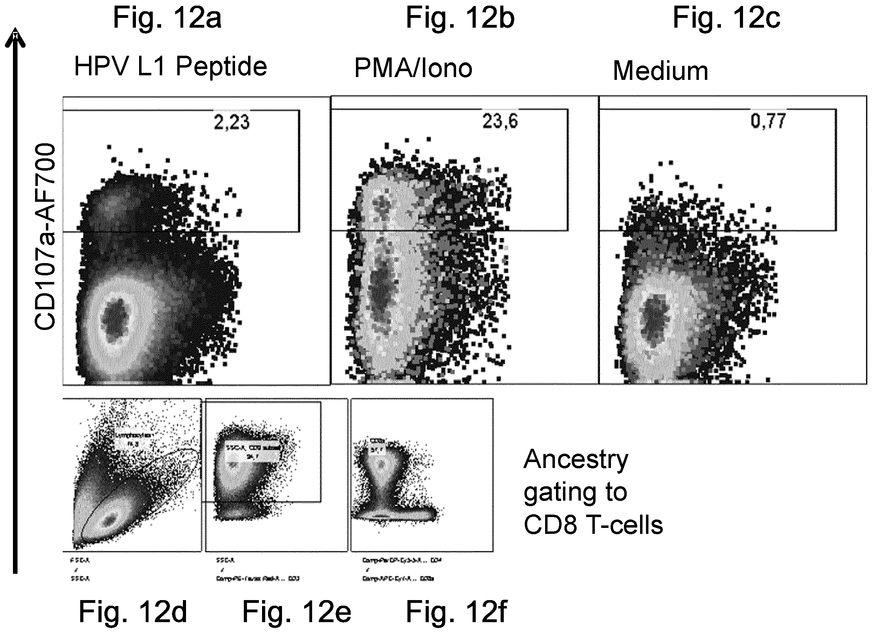

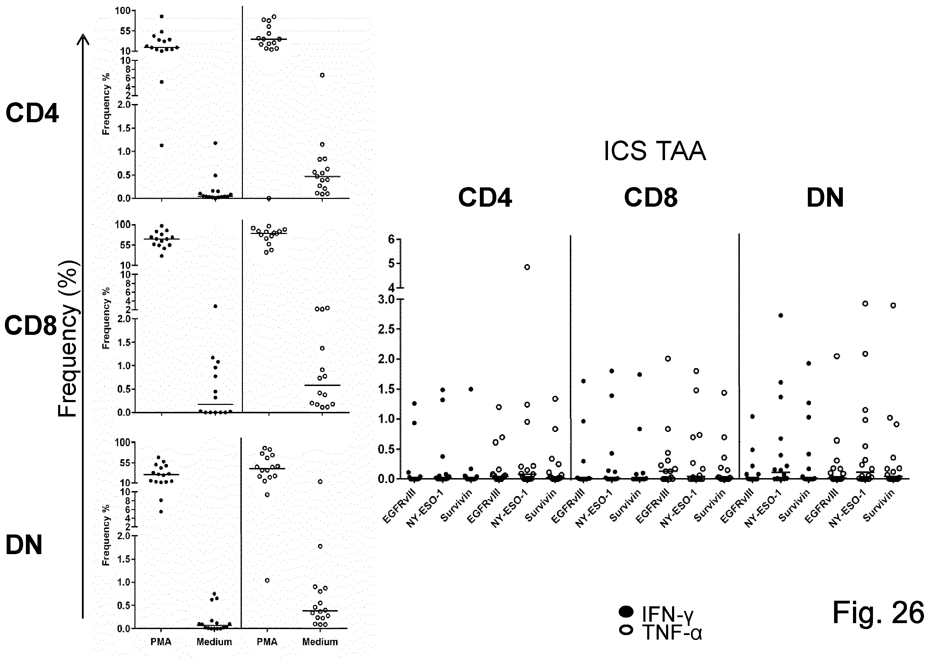

Fig. 26    shows the results of an intracellular cytokine production assay in CD4+, CD8 or DN T-cells in expanded lymphocytes from Glioblastoma lesions. The graphs in Fig. 7 B show the percentage of T-cells producing the cytokines IFNγ and TNFα after stimulation. Fig 12 A shows maximal stimulation by PMA/ionomycin (positive control) and background by medium only. Fig. 7 B shows the results of stimulation by synthetic peptides derived from tumor - associated antigens, i.e. the EGRvrlll, NY-ESO-1 or survivin. The data show that IL-2, IL-15 and IL-21 expanded TIL from patients with glioblastoma contain T-cells that react at low frequency to commonly shared tumor-associated antigens.

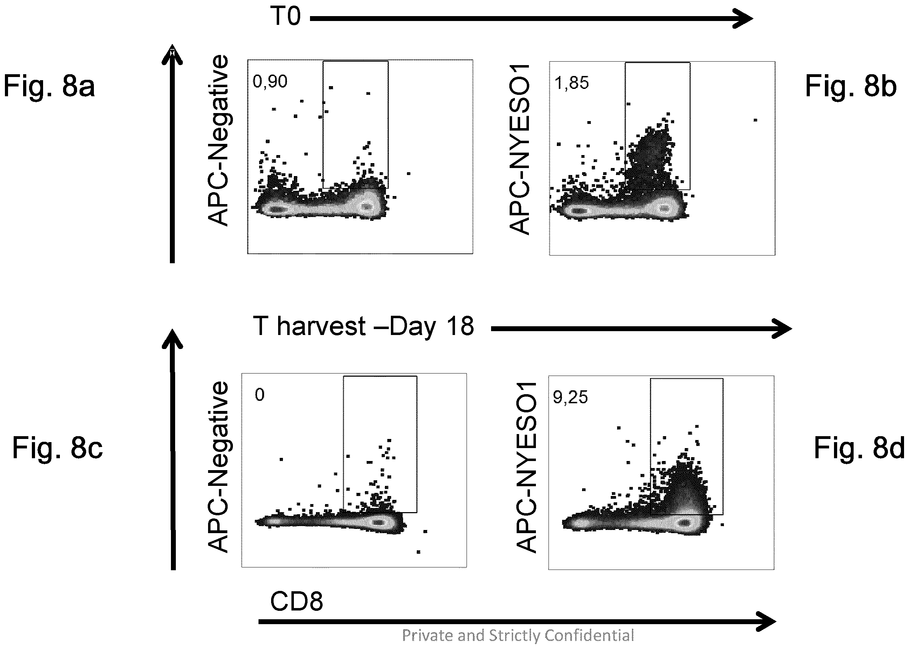

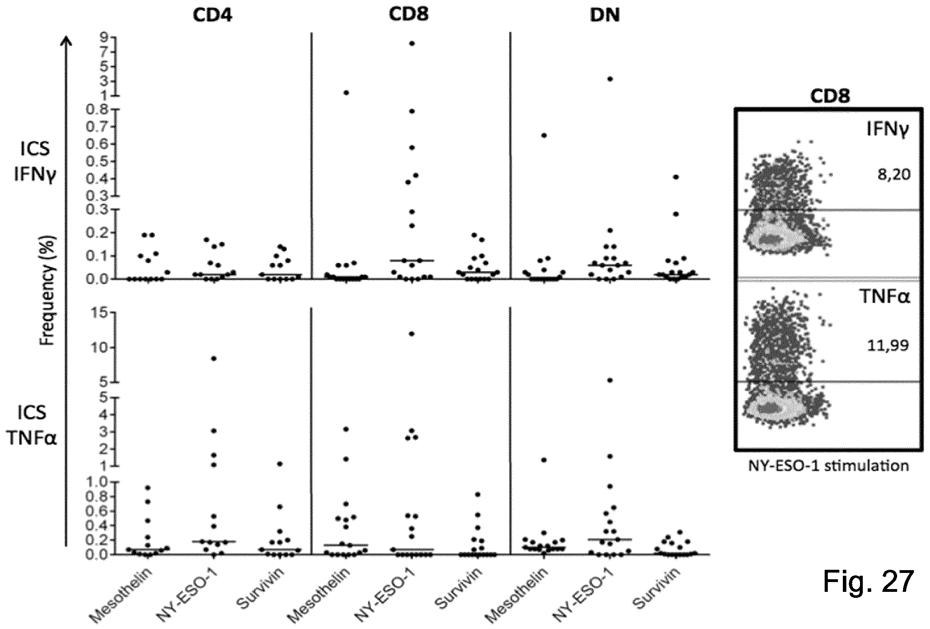

Fig. 27    shows the results of an intracellular cytokine production assay in CD4+, CD8 or DN T-cells in expanded lymphocytes from pancreatic cancer lesions. The graphs in Fig. 8 A show the percentage of T-cells producing the cytokines IFNy (upper panel) and TNFα (lower panel) after stimulation with tumor-associated antigens, i.e. Mesothelin, NY-ESO-1 or surviving in the CD4+ (left), CD8+ (middle) and DN subset (right). Fig. 8 B displays examples of a flow-cytometric analysis with NY-ESO-1 stimulation. T-cells gated on CD3+ and then on CD8+. are in the side scatter (SSC) versus IFNy (upper box) or TNFα (lower box) production. The show that IL-2, IL-15 and IL-21 expanded TIL from patients with pancreatic cancer show a strong reactivity to a commonly shared tumor antigens, i.e. NY-ESO-1.

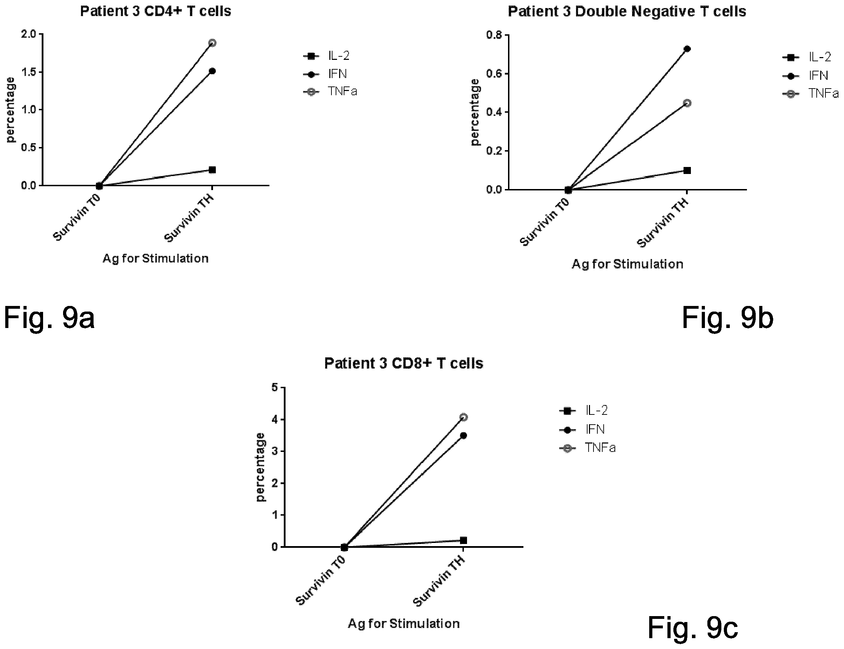

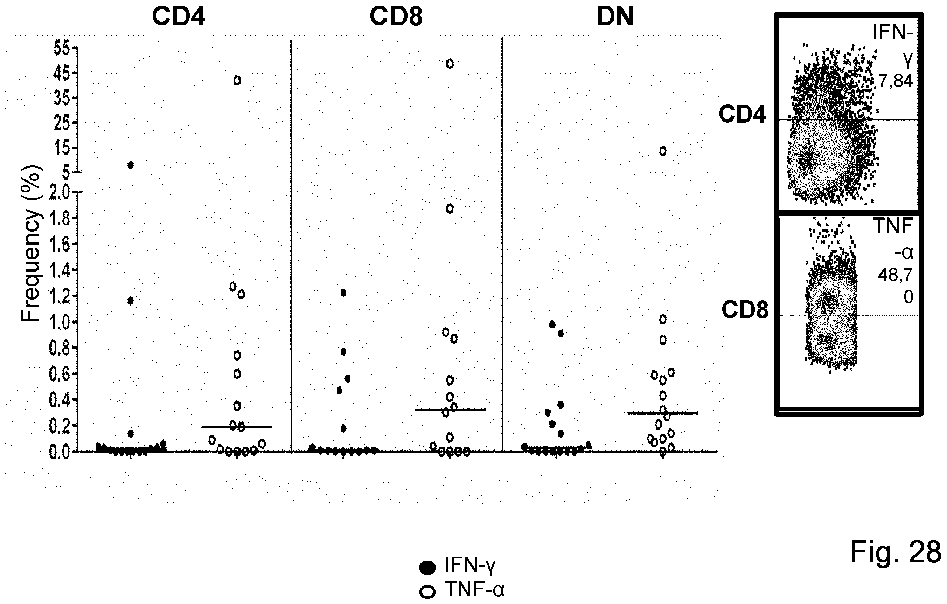

Fig. 28    shows the results of an intracellular cytokine production assay in CD4+, CD8 or DN T-cells in expanded lymphocytes from glioblastoma lesions after stimulation with autologous tumor cells. The graphs in Fig. 9 A show the percentage of T-cells producing the cytokines IFNy and TNFα for CD4+ left panel, CD8+ (middle panel) and DN T-cells (right panel) after stimulation with autologous tumor cells. Fig. 9 B displays examples of a flow-cytometric analysis of cells stimulated with autologous tumor cells. T-cells were gated on CD3+ and then on CD 4+ (upper box) or CD8+ (lower box) are in the side scatter (SSC) versus IFNy (upper box) or TNFα (lower box) production. The show that IL-2, IL-15 and IL-21 expanded TIL from patients with glioblastom show a strong reactivity to autologous tumor cells.

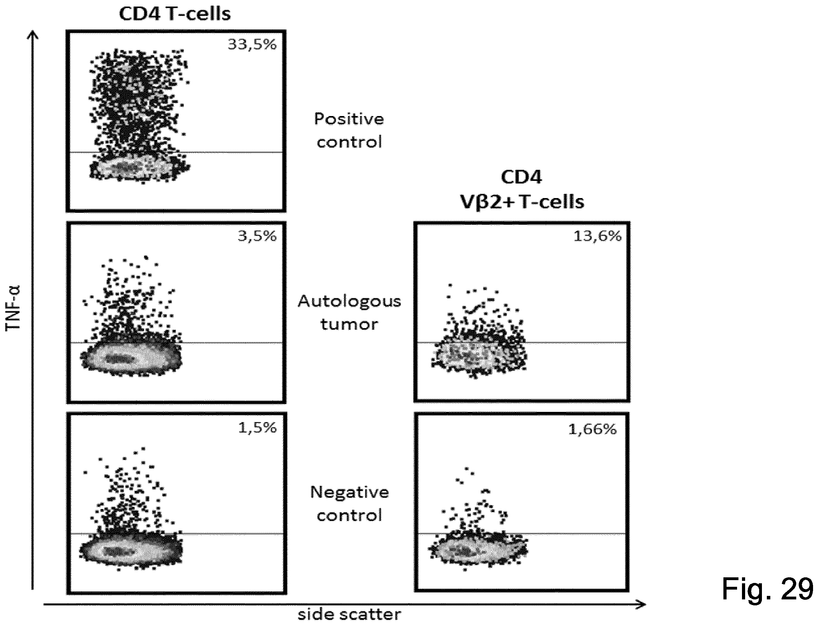

Fig. 29    shows the results of an intracellular cytokine production assay measuring TNFα production of lymphocytes expanded with the cytokine cocktail of IL-2, IL-15 and IL-21. The top panel shows the positive control (maximal stimulation). The middle panel shows results CD4+ gated expanded T-cells cytokine production in response to autologous tumor cells (left: all TIL, right: TIL gated on VB2+ T-cells). Lower panel: background production in the entire TIL population (left) and in the VB2+ TIL (right). The data show that preferentially expanded TCR VB families in IL-2, IL-15, IL-21 TIL (here: TCR VB2) are directed against autologous tumor cells.

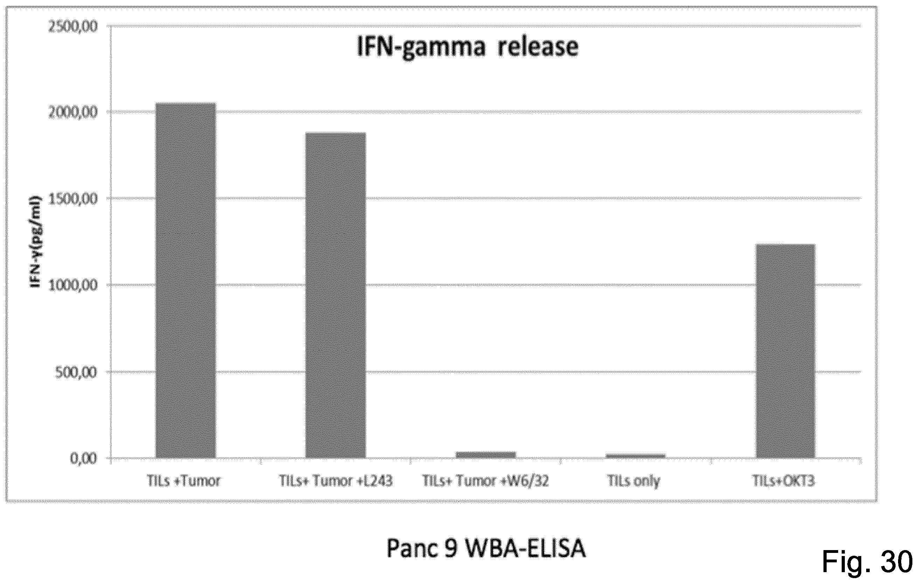

Fig. 30    shows the level of INFy in lymphocytes expanded from pancreatic tumor tissues after after stimulation. TIL+tumor stands for a stimulation of the expanded lymphocytes with autologous tumor cells. TIL+OKT3 stands for a stimulation of the lymphocytes with a CD3 antibody. W6/32 is an antibody blocking CD8+ TIL. Antibody L243 blocks CD4+ TIL. The data show that IL-2, IL-15 and IL-21 expanded TIL are specific against the patients autologous tumor.

Fig. 31     shows the result of an analysis of the cytolytic response of expanded TIL from patients with glioblastoma against autologous tumor cells. The numbers on the X-axis represent the ratio of TIL to tumor cells. The percentage on the Y-axis represents the number of killed tumor cells after 4 h of treatment with expanded TIL measured by radioactivity release.

Fig. 32     shows the result of an analysis of the cytolytic response of expanded monoclonal T-cells and/or preferentially expanded TIL from patients with glioblastoma against autologous tumor cells. The numbers on the X-axis represent the ratio of TIL to tumor cells. The percentage on the Y-axis represents the number of killed tumor cells after 4 h of treatment with expanded TIL measured by radioactivity release.

Fig. 33     shows the result of an analysis of the cytolytic response of expanded TIL from patients with pancreatic cancer against autologous tumor cells. The numbers on the X-axis represent the ratio of TIL to tumor cells. The percentage on the Y-axis represents the number of killed tumor cells after 4 h of treatment with expanded TIL measured by radioactivity release. These IL-2, IL-15 and IL-21 expanded TIL showed a very focused TCR repertoire and show a strong cytotoxic response against the autologous tumor cells.

DETAILED DESCRIPTION OF THE INVENTION

The inventors have found that a combination of the interleukins IL-2, IL-15 and IL-21 provides significant improvements for immunotherapy with lymphocytes. One major advantage is that the expansion and stimulation of lymphocytes derived from a patient with the composition of a combination of at least two types of cytokines selected from IL-2, IL-15 and IL-21 specifically favors the generation of lymphocytes, in particular T-cells, which are clinically relevant.

According to the invention "clinically relevant lymphocytes" are specific for and interact with clinically relevant antigens. There are three groups of clinically relevant lymphocytes, namely tumor-reactive lymphocytes, infectious disease reactive lymphocytes and autoimmune disease reactive lymphocytes.

"Clinically relevant lymphocytes" are also referred to as antigen-edited lymphocytes. The term clinically relevant is also used for subgroups of lymphocytes. Particularly preferred clinically relevant lymphocytes are clinically relevant T-cells or antigen-edited T-cells.

"Clinically relevant antigens" according to the invention are antigens involved in a disease. Accordingly, clinically relevant antigens can be tumor-associated antigens TAA, pathogen associated antigens (PAA) or autoantigens. Tumor-reactive lymphocytes are specific for and interact with TAAs. Infectious disease reactive lymphocytes are specific for and interact with PAAs and autoimmune disease reactive lymphocytes are specific for and interact with autoantigens.

According to the invention an "antigen" (Ag) is any structural substance which serves as a target for the receptors of an adaptive immune response, TCR or antibody, respectively. Antigens are in particular proteins, polysaccharides, lipids and substructures thereof such as peptides. Lipids and nucleic acids are in particular antigenic when combined with proteins or polysaccharides.

"Pathogen associated antigens" (PAA) refer to parts, such as capsules, cell walls, flagella and toxins of pathogens such as bacteria, viruses and other microorganisms.

"Autoantigens" are usually peptides, oligopeptides, polypeptides or complexes of proteins from an individual that are recognized by the immune system of the same individual. This effect usually leads to an autoimmune disease.

"Tumor associated antigens" or "TAA" according to the invention are antigens that are presented by MHC I or MHC II molecules or non-classical MHC molecules on the surface of tumor cells. As used herein TAA includes "tumor-specific antigens" which are found only on the surface of tumor cells, but not on the surface of normal cells.

With a combination of IL-2, IL-15 and IL-21 it is possible to specifically induce the proliferation of the clinically relevant lymphocytes in a body sample obtained from a patient as shown in the examples. The method according to the invention provides an easy protocol for the expansion of clinically relevant lymphocytes. It is in particular advantageous in comparison to the protocols in the state of the art as no dendritic cells are required. Moreover, the inventors could show that the resulting lymphocyte population after expansion with the cytokine cocktail of a combination of at least two types of cytokines selected from IL-2, IL-15 and IL-21 contains a composition of lymphocytes that is advantageous for clinical application. For example, the composition has a high percentage of $T_{H1}$ helper T-cells and almost no $T_{H2}$ helper T-cells. A further advantage is that there is no significant expansion of regulatory T-cells which might cause a suppression of the therapeutic action of the expanded lymphocyte population.

Thus, according to a first aspect the invention provides a composition for expanding lymphocytes comprising at least two types of cytokines selected from interleukin 2 (IL-2), interleukin 15 (IL-15) and interleukin 21 (IL-21).

IL-2, IL-15 and IL-21 are members of the cytokine family each of which has a four alpha helix bundle. IL-2 has key roles in key functions of the immune system, tolerance and immunity, primarily via its direct effects on T-cells. IL-2 induces T-cells proliferation and differentiation into effector and memory T-cells.

IL-15 is a cytokine that is structurally similar to IL-2. Like IL-2, IL-15 binds to and signals through a complex composed of the IL-2/IL-15 receptor beta chain. IL-15 induces a T-cell activation and proliferation in particular of CD8+ T-cells (30) and also provides survival signals to maintain memory cells in the absence of antigens, favored CD8+ T-cells and activates monocytes. IL-15 appears to drive rather proliferation of immune effector T-cells, along with the protection from inhibition of tumor-associated immunosuppression (31).

IL-21 is a cytokine that has potent regulatory effects on cells of the immune system, including natural killer (NK) cells and cytotoxic T-cells. IL-21 enriches central memory type T-cells with a CD28+ CD127hi CD45RO+ phenotype and enhances the cytotoxity of cytotoxic T-cells. IL-21 may keep T-cells in their early phase of differentiation and maturation (35).

According to the invention the composition of a combination of at least two types of cytokines selected from IL-2, IL-15 and IL-21 is also referred to as "the cytokine cocktail".

As used herein, "interleukin 2" or "IL-2" refers to human IL-2 as defined by SEQ ID NO: 1 and functional equivalents thereof. Functional equivalents of IL-2 include relevant substructures or fusion proteins of IL-2 that remain the functions of IL-2. Accordingly, the definition IL-2 comprises any protein with a sequence identity to SEQ ID NO: 1 of at least 80 %, preferably at least 90 %, more preferably at least 95 %, most preferably at least 98 %. Recombinant human IL-2 produced in *E. coli* as a single, non-glycosylated polypeptide chain with 134 amino acids and having a molecular mass of 15 kDa is commercially available in lyophilized form from Prospec as CYT-209.

As used herein, "interleukin 15" or "IL-15" refer to human IL-15 and functional equivalents thereof. Functional equivalents of IL-15 include relevant substructures or fusion proteins of IL-15 that remain the functions of IL-15. Accordingly the definition IL-15 comprises any protein with a sequence identity to SEQ ID NO: 2 of at least 80 %, preferably at least 90 %, more preferably at least 95 %, most preferably at least 98 %. Recombinant human IL-15 produced in E. coli as a single, non-glycosylated polypeptide chain with 114 amino acids (and an N-terminal Methionine) and having a molecular mass of 12.8 kDa is commercially available in lyophilized form from Prospec as CYT-230.

As used herein, "interleukin 21" or "IL-21" refer to human IL-21 and functional equivalents thereof. Functional equivalents of IL-21 included relevant substructures or fusion proteins of IL-21 that remain the functions of IL-21. Accordingly the definition IL-21 comprises any protein with a sequence identity to SEQ ID NO: 3 of at least 80 %, preferably at least 90 %, more preferably at least 95 %, most preferably at least 98 %. Recombinant human IL-21 produced in E. coli as a single, non-glycosylated polypeptide chain with 132 amino acids and having a molecular mass of 15 kDa is commercially available in lyophilized form from Prospec as CYT-408.

A "peptide" as used herein may be composed of any number of amino acids of any type, preferably naturally occurring amino acids, which, preferably, are linked by peptide bonds. In particular, a peptide comprises at least 3 amino acids, preferably at least 5, at least 7, at least 9, at least 12, or at least 15 amino acids. Furthermore, there is no upper limit for the length of a peptide. However, preferably, a peptide according to the invention does not exceed a length of 500 amino acids, more preferably it does not exceed a length of 300 amino acids; even more preferably it is not longer than 250 amino acids.

Thus, the term "peptide" includes "oligopeptides", which usually refer to peptides with a length of 2 to 10 amino acids, and "polypeptides" which usually refer to peptides with a length of more than 10 amino acids.

The term "protein" refers to a peptide with at least 60, at least 80, preferably at least 100 amino acids.

The term "fusion protein" according to the invention relates to proteins created through the joining of two or more genes, cDNAs or sequences that originally coded for separate proteins/peptides. The genes may be naturally occurring in the same organism or different organisms or may synthetic polynucleotides.

The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et a/., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using thenobrief option) is used as the percent identity and is calculated as follows:

(Identical Residues x 100)/(Length of Alignment - Total Number of Gaps in Alignment)

The transitional term "comprising", which is synonymous with "including," "containing," or "characterized by," is inclusive or open-ended and does not exclude additional, unrecited elements or method steps. The transitional phrase "consisting of" excludes any element, step, or ingredient not specified in the claim, except for impurities ordinarily associated therewith. When the phrase "consists of" appears in a clause of the body of a claim, rather than immediately following the preamble, it limits only the element set forth in that clause; other elements are not excluded from the claim as a whole. The transitional phrase "consisting essentially of" limits the scope of a claim to the specified materials or steps "and those that do not materially affect the basic and novel characteristic(s)" of the claimed invention. "A 'consisting essentially of' claim occupies a middle ground between closed claims that are written in a 'consisting of' format and fully open claims that are drafted in a 'comprising' format."

"Expansion" or "clonal expansion" as used herein means production of daughter cells all arising originally from a single cell. In a clonal expansion of lymphocytes, all progeny share the same antigen specificity.

According to one embodiment of the invention the composition according to the first aspect comprises two or three types of cytokines. Further cytokines may interfere with the expansion results provided by the composition according to the invention.

Alternatively, other cytokines used in addition to a combination of IL-2, IL-15 and IL-21 may positively influence the lymphocyte population. Thus, the composition of the first aspect of the invention may contain more cytokines in addition to IL-2, IL-15 and IL-21. Examples are IL-1beta, IL-4, GM-CSF, IL-12, IL-8, IL-17, TNF$\alpha$, IL-32. IL-1$\beta$ is involved in priming, the differentiation into effector B-cells or T-cells upon first contact with the specific antigen. IL-4 and GM-CSF are involved in dendritic cell stimulation and/or priming. IL-12 is involved in $T_{H1}$ responses. IL-18 stimulates $\gamma\delta$-T-cells. IL-17 and TNF$\alpha$ act pro-inflammatory. IL-32 also acts pro-inflammatory favoring long-term protective immune responses.

According to one embodiment of the first aspect the composition comprises IL-2 and IL-15. The composition may also comprise IL-2 and IL-21. Alternatively, the composition may comprise IL-15 and IL-21. Although, already two of the cytokines IL-2, IL-15 and IL-21 may be enough to obtain a population of clinically relevant lymphocytes it is preferred to use all three cytokines in the composition.

According to a further embodiment, the composition of first aspect is in liquid form. In particular the composition is a cell culture medium. According to the invention any known cell culture medium is possible. Non-limiting examples of cell culture media are a synthetic medium, a medium derived from serum, plasma or whole blood or any combination thereof.

According to a further embodiment, the concentration of IL-2 in the liquid composition is in the range of from 10 to 6000 U/ml. The International Unit (U) is the standard measure for an amount or IL-2. It is determined by its ability to induce the proliferation of CTLL-2 cells. A concentration below 10 U/ml is too low to achieve any significant effect. A concentration above 6000 U/ml might have a cytotoxic effect. The concentration of IL-2 is preferably in the range from 500 to 2000 U/ml. More preferably the concentration of IL-2 is in the range from 800 to 1100 U/ml. As shown in the examples optimal results were achieved with a concentration of about 1000 U/ml.

According to a further embodiment of the first aspect, the concentration of IL-15 is in the range of 0.1 to 100 ng/ml. The concentration range follows the same rational as for IL-2. A concentration below 0.1 ng/ml is believed not to have any significant effect on the cells. A concentration above 100 ng/ml might have a cytotoxic effect. Preferably, the concentration of IL-15 is in the range from 2 to 50 ng/ml, more preferably in the range from 5 to 20 ng/ml. The most preferred concentration is about 10 ng/ml.

In a further embodiment the concentration of IL-21 is in the range from 0.1 ng/ml, preferably in the range from 2 to 50 ng/ml, more preferably in the range from 5 to 20 ng/ml.

It is to be understood that according to the invention any of these concentration ranges of one of the cytokines can be combined with any of concentration ranges of the other cytokines.

According to one embodiment the combination comprises a mixture of IL-15 and IL-21. The mixture comprises preferably each of IL-15 and IL-21 in the range form 10 to 100 ng/ml. IL-15 and IL-21 may provides synergistic effects, particularly on lymphocyte subsets in precursor, memory and effector populations.

According to one embodiment of the first aspect the combination comprises IL-2 in a concentration of 800 to 1000 U/ml and IL-15 and IL-21 in a concentration of 5 to 20 ng/ml. According to another embodiment the composition comprises IL-2 in a concentration of about 1000 U/ml and IL-15 and IL-21 in a concentration of about 10 ng/ml.

The composition of IL-2, IL-15 and IL-21 is in particular beneficial for promoting the expansion of clinically relevant lymphocytes in a composition of lymphocytes, in particular a patient's sample. As shown in the examples the inventors have developed a method for preparing specifically clinically relevant lymphocytes from a patient's sample.

The composition of IL-2, IL-15 and IL-21 is in particular beneficial for promoting the expansion of clinically relevant lymphocytes in a composition of lymphocytes, in particular a patient's sample. As shown in the examples the inventors have developed a method decreasing the frequency of PD1 and LAG3 + T-cells, where PD1 and/or LAG3 expression serves as a marker for T-cell exhaustion and not as marker for antigen-experienced T-cells.

The composition of IL-2, IL-15 and IL-21 is in particular beneficial for promoting the expansion of clinically relevant lymphocytes in a composition of lymphocytes, in particular a patient's sample. As shown in the examples the inventors have developed a method increasing the frequency of 4-1BB expression as a marker for antigen-experienced T-cells.

Thus, according to a second aspect the invention provides a method of preparing a population of clinically relevant lymphocytes comprising the steps of

- obtaining a body sample from a mammal in particular a tissue sample or body liquid sample, comprising at least one lymphocyte and optionally separating the cells in the body sample,
- culturing the body sample *in-vitro* to expand and/or stimulate lymphocytes in the sample wherein the culturing comprises using at least two types of cytokines selected from IL-2, IL-15 and IL-21,
- and optionally determining the presence of clinically relevant lymphocyte in the cultured sample.

The at least two types of cytokines selected from IL-2, IL-15 and IL-21 are preferably used in the concentrations defined above. Preferably all three cytokines IL-2, IL-15 and IL-21 are used together.

As shown in the examples the method can be used for generating a population of tumor reactive lymphocytes, autoimmune disease reactive lymphocytes or infectious disease reactive lymphocytes. Preferably, the lymphocyte population generated by the method of the second aspect is a population of tumor reactive lymphocytes.

The lymphocytes in general will comprise a variety of different lymphocytes. Among these lymphocytes may be lymphocytes present that have the right receptor to interact with a clinically relevant antigen, in particular a tumor associated antigen, an infectious disease associated antigen or an autoimmune disease associated antigen. With the method according to the invention this clinically relevant lymphocyte in particular is strongly expanded. However, other lymphocytes that do not have the specificity for the clinically relevant antigen also are expanded in the method according to the invention.

Thus, the outcome of culturing the cells from the body sample with the composition comprising IL-2, IL-15 and/or IL-21 leads to the formation of a population of lymphocytes which includes the population of clinically relevant lymphocytes. The determination of the presence of clinically relevant lymphocytes in the cultured sample is a possible but not necessary step of the method according to the second aspect of the invention. The steps useful to verify the expanded lymphocytes population indeed can be used as a therapeutic.

The body sample can be taken from any part of the body that contains lymphocytes. Examples of body samples are peripheral blood, cord blood, bone marrow, lymph nodes, liver pleural effusion, thorax, abdominal cavity, synvial fluid, peritoneum, retroperitoneal space, thymus, and tumor.

A lymphocyte sample derived from tumor is also referred as tumor infiltrating lymphocytes (TIL). "TIL" as used herein is short for "tumor infiltrating lymphocytes". TIL is any kind of lymphocyte that is located in, on or around a tumor. TIL is any kind of lymphocyte that is located in and around a tumor.

Due to their localization in the tumor, the TIL may have experienced tumor-associated antigens. Accordingly, clinically relevant lymphocytes, in particular tumor reactive lymphocytes can be expanded with the method according to the invention without expansion antigen.

A sample from peripheral blood is also referred to peripheral blood mononuclear cells (PBMCs). Depending on the type of disease different body samples may be preferred.

As used herein, the term "mammal" refers to any mammal, including, but not limited to, mammals of the order Rodentia, such as mice and hamsters, and mammals of the order Logomorpha, such as rabbits. It is preferred that the mammals are from the order Carnivora, including Felines (cats) and Canines (dogs). It is more preferred that the mammals are from the order Artiodactyla, including Bovines (cows) and Swines (pigs) or of the order Perssodactyla, including Equines (horses). It is most preferred that the mammals are of the order Primates, Ceboids, or Simoids (monkeys) or of the order Anthropoids (humans and apes). An especially preferred mammal is the human.

According to one embodiment of the second aspect the mammal from which the body sample is obtained is a human. The mammal may have a tumor disease, or be at risk of developing a tumor disease. The risk of developing a tumor disease includes a high risk, a moderated risk and a low risk. A mammal in such a pre-malignant condition for example has a premalignant lesion which is morphologically atypical tissue which appears abnormal under microscopic examination and in which cancer is more likely to occur as in its apparently normal counterpart.

Moreover, the mammal may have an infectious disease or be at risk of developing an infectious disease. The risk of developing an infectious disease includes a high risk, a moderated risk and a low risk. The mammal may also have an autoimmune disease or be at risk of developing and autoimmune disease. The risk of developing an autoimmune disease includes a high risk, a moderated risk and a low risk. For instance, a high risk to develop intracellular infections (CMV, EBV, TB, HPV) upon certain genetic mutations of the host (IFNγ receptor defects, or acquired antibodies directed against cytokines, e.g. IL-12 or IFNγ). An intermediate risk would be immune-suppression using corticosteroids or treatment of patients with anti-TNFα or TNFα-receptor directed reagents. A low risk could be the co-infection with other pathogens or the temporary decrease of immune-competence in the course of major surgery. Similar examples are different clinical presentations - in association with genetic markers - of Multiple Sclerosis, rare neurological diseases, e.g. narcolepsy, Rheumatoid Arthritis, as well as chronic autoimmune-diseases associated with the gastrointestinal system.

If the mammal has a tumor disease or is at high risk of developing a tumor disease preferred body sample are peripheral blood or the tumor itself. As shown in the examples lymphocytes from peripheral and tumor can be treated with a method according to the invention to develop strong antitumor properties. If the disease is an autoimmune disease the preferred body sample is peripheral blood. Moreover, when the disease is an infectious disease the preferred body sample is also peripheral blood. As shown in the examples clinically relevant lymphocytes can be expanded from peripheral blood in these cases. Without being bound to theory it is assumed that the peripheral blood contains lymphocytes that have been in contact with the clinically relevant antigen, e.g. on the tumor or the infection.

Culturing of the body sample in vitro to expand and/or stimulate lymphocytes may comprise one or more substeps. Accordingly, in one embodiment the in vitro culturing comprises a first expansion step comprising a incubation in culture medium comprising IL-2, IL-15 and IL-21 until lymphocytes become detectable.

"Detectable" according to the invention means that the lymphocytes, for example, become visible, in particular by microscopy. Lymphocytes usually become detectable using standard light microscopy upon reaching a concentration of $5 \times 10^3$ cells/ml.

The detection of the lymphocytes may include any method known in the art that is eligible to detect the presence of lymphocytes above a certain threshold. The first expansion step has the purpose of gently inducing cell proliferation together with a stimulation of the cells by the cytokine cocktail.

The time of incubation of the first expansion step is in the range from 6 hours to 180 days. The large range of incubation time is first of all due to the fact that samples from different donors may behave very differently. Also it was shown that the lymphocytes from different body samples have very different growth rates. For example lymphocytes derived directly from the tumor of a glioblastoma or a pancreas cancer grow very differently. From pancreas cancer derived lymphocytes are already detectable within two to five days. Lymphocytes derived from glioblastoma are only detectable after one to two weeks. Accordingly lymphocytes from other body samples may take even longer to become detectable.

Preferably, the incubation time of the first expansion step is in the range from 4 days to 10 days. It was shown that with peripheral blood cells, incubation times about 7 days are particularly beneficial for the outcome of other expansion. However, as mentioned above, depending on the sample an expansion of only 4 days may be enough or on the other hand about 10 days or more may be necessary. Due to the good results with PBMCs shown in the examples an incubation time in the range from 6 to 8 days, in particular about seven days, is preferred.

According to one embodiment of the invention the culture medium of the first expansion step comprises at least one expansion antigen. The expansion antigen is a known clinically relevant antigen or a fragment, a mutant or a variant thereof. As used herein a "mutant" is defined as an amino acid sequence that differs from the reference sequence by an insertion, deletion or replacement of at least one amino acid. The expansion antigen is preferably selected from TAA, PAA and autoantigen.

Preferably the method according to the invention comprises multiple copies of an expansion antigen. An increased number of copies of an expansion antigen leads to increased expansion rate of a clinically relevant lymphocyte, in particular T-cells.

In one embodiment of the invention the culture medium of the first expansion step comprises multiple expansion antigens. Preferably, the multiple expansion antigens include a known clinically relevant antigen and a one or more mutants of the clinically relevant antigen. Instead of the antigen itself also the MHC class I/peptide presenting the antigen, in particular peptide can be mutated. The use of one or more wild type, variant or mutants of the clinically relevant antigen or MHC class I molecules as expansion antigens leads to a diverse set of lymphocytes, in particular T-cells reactive against the nominal clinically relevant antigen.

According to a preferred embodiment of the method according to the second aspect, the body sample is tumor and no expansion antigen is used in culturing.

The tumor tissue is preferably washed twice before separating the cells in the tumor sample.

Another preferred embodiment of the invention, the culture medium of the first expansion step comprises multiple expansion antigens. By having multiple antigens the T-Cell product responds in a more diverse T-Cell receptor repertoire as demonstrated by Vβ-usage associated with the use of the stimulating antigen(s).

Another preferred embodiment of the invention the culture medium of the first expansion step comprises multiple expansion antigens. The stimulation of T-Cells from peripheral blood results in T-Cell recognizing diverse epitopes, defined by cytoxicity. When naïve T-Cells are selected from the blood and stimulated with such antigens, they result in diverse epitopes of the antigen being recognized.

This is in contrast to pre-stimulated T-Cells or T-Cells from the tumor, which tend to recognize a more limited number or standard number of epitopes.

The choice of expansion antigen is dependent on the disease to be treated. In particular, if the expanded clinically relevant lymphocyte population is to be used against the tumor diseases, the expansion antigen added to the first expansion step is a preferably TAA. Alternatively, if the disease to be treated is an infectious disease, the expansion antigen added in the first expansion step is a PAA. Moreover, if the population of clinically relevant lymphocytes is to be used for the treatment of an autoimmune disease, the expansion antigen is preferably an autoantigen.

It is believed that the expansion antigen in the first expansion step leads to a stimulation of clinically relevant lymphocytes within the cell mixture already at an early stage and thus together with the cytokine cocktail enhances the expansion of clinically relevant lymphocytes. This stimulation is also referred to as antigen activation or antigen editing.

The first expansion step may be followed by a second expansion step wherein the cells are incubated with feeder cells and/or an antibody against CD3 in addition to the at least two types of cytokines selected from IL-2, IL-15 and IL-21. An expansion with feeder cells and the antibody against CD3 has been described in the state of the art. It is believed that feeder cells lead to an improvement of cell growth. Feeder cells are irradiated cells that do not proliferate or proliferate only to a small extent. The feeder cells increase the number of cell contacts in the culture and additionally feed the proliferating and expanding cell culture. Feeder cells are preferably in irradiated PBMCs. Allogeneic feeder. are from a different organism as the mammal to be treated with the expanded clinically relevant lymphocytes. Autologous feeder cells are from the mammal to be treated.

The antibody against CD3 is preferably the antibody defined as OKT3. OKT3 is a murine monoclonal antibody of the immunoglobulin IgG2a isotype. The target of OKT3, CD3, is a multi-molecular complex found only on mature T-cells. An interaction between T-cells, OKT3 and monocytes causes T-cell activation in vitro.

Preferably, feeder cells are used in combination with CD3 and the cytokines IL-2, IL-15 and IL-21. According to one embodiment of the method according to the second aspect, the ratio of feeder cells to lymphocytes is in the range from 1:1 to 1:100. Preferably, the ration of feeder cells to lymphocytes is in the range from 1:2 to 1:50. As shown in the examples very low ratios of feeder cells are sufficient for a strong expansion of clinically relevant lymphocytes, in particular clinically relevant T-cells.

In the examples, a ratio of 1:10 is sufficient to support growth and expansion of lymphocytes. Accordingly it is believed that a value in the range from 1:5 to 1:20 would not lead to a different result. The low number of feeder cells has at least two advantages. First, there are less distracting cellular signals allowing more homogeneous and reliable expansion results. Secondly, fewer feeder cells lead to less exogenous material in the immunotherapy product obtained by the method, i.e. the population of the clinically relevant lymphocytes.

The second expansion step optionally also includes an expansion antigen in the culture medium. Preferably no clinically relevant antigen or fragment is added to the medium of the second expansion step.

According to a preferred embodiment of the second aspect of the invention the method comprises a refocusing step. The refocusing step comprises a culturing in culture medium comprising refocusing cells. Refocusing cells are cells from the mammal, in particular a human, from which the body sample is obtained. Accordingly, the refocusing cells are autologous cells that have been treated with at least one refocusing antigen. Any expansion antigen as defined herein can also be used as refocusing antigen. Preferably, in the method the one or more refocusing antigens are identical to the one or more expansion antigens.

Refocusing cells are cells that have been incubated with the refocusing antigen for at least 30 minutes, or more, for example at least one hour, at least two hours, at least five hours, or at least ten hours. After incubation with the refocusing the refocusing cells are irradiated with at least 40 Gy. Preferably the cells are irradiated with at least 45 Gy, or more, for example at least 50 Gy and particularly preferred with an intensity of 55 Gy. Due to this treatment, antigen-specific T-cells are more effectively expanded, recognize tumor, pathogens or auto-immune cells and may confer protection against cancer cells or pre-malignant lesions.

The time of the refocusing step is in the range from 1 to 6 days, preferably 1 to 3 days. Although the refocusing step can be rather short, it leads to significant improvement in the yield of expanded clinically relevant lymphocytes, in particular for clinically relevant lymphocytes expanded from peripheral blood.

Also the number of refocusing cells in comparison to the number of lymphocytes is rather low. In particular the ratio of refocusing cells to lymphocytes is in the range from 1:1 to 1:100. It is found that the best results are achieved if the refocusing step is performed right after the first expansion step and followed by the second expansion step. The sequence of culturing steps is in particular useful for generating a population of clinically relevant lymphocytes, in particular T-cells.

According to one embodiment of the method, the first expansion step comprises adding IL-2, IL-15 and IL-21 simultaneously to the cell culture. For this, it is possible to prepare a mixture of IL-2, IL-15 and IL-21 and add the same to the cell culture medium or IL-2, IL-15 and IL-21 are added separately but simultaneously to the cell culture medium. As shown in the examples, the simultaneous application of IL-2, IL-15 and IL-21 leads to a preferred lymphocyte composition of the expanded lymphocyte population.

In order to shift the composition of the expanded lymphocyte population it is possible in the method of the invention to first add only IL-21 to the cell culture medium in the first expansion step. After the addition of IL-21, IL-15 and IL-2 may be added simultaneously or sequentially. Preferably IL-15 is added secondly and IL-2 added last. In an alternative embodiment IL-15 is added as the first cytokine, followed by simultaneous addition of IL-2 and IL-21 or a sequential addition of IL-2 and IL-21. For example, IL-21 is added secondly and IL-2 is added last.

The culture medium of the first and/or second expansion step may comprise least one expansion antigen. The expansion antigen may for example be fragment of a known TAA. Possible TAAs for as expansion antigen are for example NY-ESO-1, tyrosinase tumor antigen, tyrosinase related protein (TRP)-1, TRP-2, VEGFR-2, and a member of the MAGE family of proteins, telomerase, p53, HER2/neu, mesothelin, carcinoembryonic, survivin, EGFRvIII, VEGF, CAMPATH 1-antigen, CD22, CA-125, Mucin-1, Alpha-1-getoprotein, PSMA.

The fragment of the TAA is in particular a peptide. Such peptide can be for example a peptide comprising at least eight continuous amino acids of an amino acid sequence that is at least 80 % identical to the amino acid sequence of SEQ ID NO: 4, SEQ ID NO: 5, SEQ ID NO: 6, SEQ ID NO: 7.

SEQ ID NO: 4 is the amino acid sequence of the known tumor associated antigen NY-ESO-1. SEQ ID NO: 5 is the amino acid sequence of the known tumor associated antigen survivin. SEQ ID NO: 6 is the amino acid sequence of the known tumor associated antigen mesothelin. SEQ ID NO: 7 is the amino acid sequence of the tumor associated antigen EGFRvIII.

The expansion antigen may for example be fragment of a known PAA. Possible PAAs as expansion antigens are for example CMVpp65, or EBV (EBNA-3, EBNA-1), HPV-16/33 E6, E7 or L1. The fragment of the PAA is in particular a peptide. Such peptide can be for example a peptide comprising at least eight continuous amino acids of an amino acid sequence that is at least 80 % identical to the amino acid sequence of SEQ ID NO: 8, SEQ ID NO: 9, SEQ ID NO: 10, or SEQ ID NO: 11, which are the amino acid sequence of the known pathogen associated antigens CMVpp65, EBNA-3, EBNA-1, and HPV-L1, respectively.

The expansion antigen may for example be fragment of a known PAA. Possible autoantigens as expansion antigens are for example PRDM2, UCHL3, INO80E, SLC12A6, and Reelin. The fragment of the PAA is in particular a peptide. Such peptide can be for example a peptide comprising at least eight continuous amino acids of an amino acid sequence that is at least 80 % identical to the amino acid sequence of SEQ ID NO: 12, SEQ ID NO: 13, SEQ ID NO: 14, or SEQ ID NO: 15, which are the amino acid sequences of the autoantigens PRDM2, INO80E, UCHL3, and DNaseB, respectively.

Additional components such as further cytokines may be added during the culturing step in particular during the first or second expansion step or the refocusing step.

Moreover, a promoter compound may be added in the culturing step, in particular in the first or second expansion step or in the refocusing step. Preferably the promoter compound is added in the first expansion step. A promoter compound as used herein is a compound that in the expansion process causes an increase a specific subset of lymphocytes. A preferred promoter compound is Zoledronic acid. Zoledronic acid promotes the expansion of gamma delta T-cells. A further preferred promoter compound is promotes the expansion of B-cells

According to one embodiment in the culturing step, in particular in the first or second expansion step or in the refocusing step a co-stimulatory compound is added. A co-stimulatory compound is for example a ligand for CD28 mediating T-cell signals. Examples of ligands for CD28 mediating T-cell signals are members of the B7 superfamily, in particular B7-1 (CD80) and B7-2 (CD86).

According to one embodiment of the method according to the second aspect testing for the presence of clinically relevant lymphocytes in the expanded lymphocyte sample comprises using evaluation antigens.

For the testing the lymphocyte population is incubated with the evaluation antigens. An evaluation antigen may be an antigen under the definition of the expansion antigen. For example, a known clinically relevant antigen or fragment thereof is added as evaluation antigen to the culture medium of the lymphocyte population to stimulate the lymphocytes. After an incubation time a parameter indicative for the activation of clinically relevant lymphocytes is measured.

Preferably the evaluation antigen is added to the lymphocytes in a form bound to a MHC I complex. For example, evaluation antigens may be presented to the lymphocytes bound to MHC dextramers. MHC dextramers are fluorescently labeled MHC multimers bound to a dextrose backbone.

The use of multimeric MHC structures has the advantage that multiple copies of the antigen can be presented to a single lymphocyte thereby increasing the simulation by the evaluation antigen. Alternatively known clinically relevant antigens may be presented to the cultured sample as evaluation antigens in the form of cells expressing the clinically relevant antigen as a transgene. Additionally, it is possible to add at least partially genetically matched allogeneic cells that present clinically relevant antigens on the cell surface to the expanded lymphocytes.

The parameter indicative for the presence of clinically relevant lymphocytes can be for example the production of one or more cytokines, in particular IFN-y or TNFα production. Further parameters indicative of the presence of clinically relevant lymphocytes are an increased cell proliferation, an increased cytotoxicity, increased cell signaling and/or intracellular phosphorylation. The determination of these parameters is known in the art and exemplified in the examples.

Preferably in addition to using evaluation antigens derived from known clinically relevant antigens it is also possible to test for clinically relevant lymphocytes specific for the mammal to be treated. For this, cells, in particular tumor cells, that are derived from the same mammal as the expanded lymphocytes are used for presenting the evaluation antigens. Using these autologous cells as evaluation presenting antigens the presence of clinically relevant lymphocytes specific for clinically relevant antigens that are not necessarily known clinically relevant antigens can be verified.

In one embodiment according to the second aspect of the invention the evaluation antigens presented to the cultured sample are in a form selected from cells, in particular tumor cells, derived from the same mammal as the cultured sample (autologous cells) at least partially genetically matched allogeneic cells, in particular tumor cells or cell expressing the clinically relevant antigens as a transgene.

According to a further embodiment the method of the testing for the presence of clinically relevant lymphocytes comprises contacting the lymphocytes with at least one evaluation antigen and determining a change in either one of cytokine production, in particular IFN-$\gamma$ or TNF$\alpha$ production, cell proliferation, cytotoxicity, signaling and/or intracellular phosphorylation.

The testing of these parameters can be combined with flow cytometry and cell sorting. Accordingly, it is also possible to isolate the clinically relevant lymphocyte population, in particular the tumor reactive lymphocyte population from the expanded lymphocyte population. The isolated population of clinically relevant lymphocytes may further be cultured or directly used for immunotherapy.

The method of the second aspect of the invention leads to the formation of population of lymphocytes, which includes a population of clinically relevant lymphocytes. Clinically relevant lymphocytes included in the expanded lymphocyte population can be any type of lymphocyte.

Lymphocytes include B-cells, NK-cells and T-cells. According to one embodiment the clinically relevant lymphocyte is a B-cell. According to one embodiment the clinically relevant lymphocyte is an NK-cell.

According a third aspect, the invention provides a clinically relevant lymphocyte obtained by the method according to the second aspect wherein the clinically relevant lymphocyte is a T-cell, an NK-cell or a B-cell..

The T-cell is preferably selected from a helper T-cell ($T_H$-cell or CD4$^+$-T-cell), in particular a $T_{H1}$-cell, a cytotoxic T-cell (Tc-cell or CD8$^+$-T-cell), in particular CD8$^+$CXCR3$^+$ T-cell, a memory T-cell, in particular a central memory T-cell ($T_{CM}$-cell), a stem cell memory T-cell ($T_{SCM}$-Cell) or peripheral memory cell ($T_{PM}$-cell), a gamma-delta T-cell ($\gamma\delta$-T-cell), a NK-T-cell, a Mucosal-associated invariant T-cell (MAIT), a double-negative T-cell (CD3$^+$CD4-CD8- T-cell).

According to one embodiment the clinically relevant lymphocyte is a lymphocyte that expresses molecules that facilitate entry into tissues, in particular tumor or infected or inflamed tissue (e.g. CXCR3). According to a further embodiment the clinically relevant lymphocyte is a lymphocyte that is enriched for markers of any long-term memory, in particular CD117 and c-kit and cytolytic immune cell responses, in particular CD107a. As explained the expanded lymphocyte population not only contains clinically relevant lymphocytes but also other lymphocytes that do not recognize clinically relevant antigens.

It is believed that clinically relevant and other lymphocytes in the culture obtained by the method according to the second aspect participate in the therapeutic effect. According to one embodiment of the third aspect, the invention relates to a lymphocyte obtained by a method according to the second aspect that expresses molecules and cytokines promoting the formation of combination of lymphocytes useful for medical application, including immunotherapy. The combination of lymphocytes useful for medical application preferably comprises T-cell precursors, $T_{CM}$, $T_{SCM}$ and/or $T_{PM}$ cells.

According to one embodiment the lymphocyte obtained by the method according to the second aspect expresses molecules and cytokines that promote the expansion of clinically relevant lymphocytes. According to a further embodiment the lymphocyte obtained by the method according to the second aspect produces cytokines selected from IFN-$\gamma$, TNF-$\alpha$, IL-2, IL-17 and any combination thereof. According to one embodiment the lymphocyte is a CD3+CD4-CD8-T-cell.

According to a fourth aspect the present invention provides a population of lymphocytes obtained by the second aspect of the invention comprising a population of clinically relevant lymphocytes.

The population of lymphocytes may consist of a population of clinically relevant lymphocytes. The population of clinically relevant lymphocytes may be monoclonal, oligoclonal or polyclonal.

In one embodiment the population of clinically relevant lymphocytes is polyclonal and responds to multiple antigens or to different epitopes of the same antigen. Preferably, the population of clinically relevant lymphocytes responds to different antigens.

Lymphocytes, in particular T-cells responding to multiple antigens may prevent the risk of recurrence of pre-malignant cells, tumor cells and pathogens, thereby decreasing the risk for immune escape.

The population of lymphocytes according to the fourth aspect has a composition of different lymphocyte phenotypes, in particular T-cell phenotypes that is beneficial for immunotherapy.

The population of lymphocytes has a low percentage of regulatory T-cells. Regulatory T-cells are known to suppress the therapeutic function of the population of lymphocytes. According to one embodiment of the fourth aspect in the population of lymphocytes the percentage of $T_{reg}$ based on the total number of T-cells is below 5 %, preferably below 3 %.

Moreover, the majority of $T_H$ cells in the population of lymphocytes comprise the $T_{H1}$ phenotype. According to one embodiment of the invention the percentage of $T_{H1}$ cells based on the total number of $T_H$ cells is at least 10 %, preferably at least 50 %, more preferably at least 70 %.

In the population of lymphocytes the percentage of the cytotoxic CD8+ T-cells is increased as could be shown that increased percentage of CXCR3+ cells. According to one embodiment of the invention the percentage of CXCR3+ cells based on the total number of CD8+ T-cells is at least 10 %, at least 50 %, more preferably at least 70 %.

In addition the population of lymphocytes may comprise an increased number of T-cells that have recently been in contact with its antigen as for example identified by the 4-1BB+ phenotype. According to one embodiment of the fourth aspect the percentage of 4-1BB+ T-cells based on the total number of T-cells is at least 1 %, preferably at least 2 %, more preferably at least 2.5 %. According to one embodiment the percentage of CD107+ cells based on the total number of T-cells is at least 1 %, preferably at least 2 %, more preferably at least 2.5 %. CD117+ T-cells have been associated with long-term memory population.

The CXCR3+ phenotype CD8+ T-cells is further associated with invasiveness into tissues and thus maybe in particular beneficial for immunotherapy against cancer. Further the population of lymphocytes may comprise sufficient percentage of CD3+CD4-CD8- cells. These double negative T-cells are highly specific for the antigen target as they are dependent of the co-receptor CD4+ or CD8+ and produce inflammatory cytokines. Thus, they are cytotoxic.

According to one embodiment a population of lymphocytes comprises a percentage of CD3+CD4-CD8- cells based on the total number of T-cells of at least 1 %, preferably at least 3 %, more preferably at least 5 %. According to a further embodiment the percentage of gamma delta T-cells based on the total number of T-cells in a population of lymphocytes is at least 1 %, preferably at least 3 %, more preferably at least 5 %. Gamma delta T-cells have the effect that they recognize stressed cells, transformed cells, infected cells, e.g. CMV+ target cells. Gamma delta T-cells cross - recognize as well virally and transformed cells.

According to one embodiment the population of lymphocytes comprises the following features:

- the percentage of $T_{reg}$ based on the total number of T cells is below 5 %, preferably below 3 %;-
- the percentage of $T_{H1}$-cells based on the total number of $T_H$-cells is at least 50 %, preferably at least 70 %, more preferably at least 80 %,
- the percentage of CXCR3+ T-cells based on the total number of CD8$^+$ T-cells is at least 50 %, preferably at least 70 %, more preferably at least 80 %,
- the percentage of 4-1BB+ T-cells based on the total number of T-cells is at least 1 %, preferably at least 2 %, more preferably at least 2.5 %,
- the percentage of CD117+ T-cells based on the total number of T-cells is at least 1 %, preferably at least 2 %, more preferably at least 2.5 %,
- the percentage of CD3+CD4-CD8- cells based on the total number of T-cells is at least 1 %, preferably at least 3 %, more preferably at least 5 %; and
- the percentage of γδT-cells based on the total number of T-cells is at least 1 %, preferably at least 3 %, more preferably at least 5 %.

According to one embodiment in the population of lymphocytes, the percentage of precursor T-cells (CD45RA+ CCR7+) based on the number of total T-cells is at least 1 %, preferably at least 2 %, more preferably at least 3 %. Central memory T-cells were already shown to relate to success of a T-cell therapy.

The central memory cells produce several cytokines beneficial for therapy, provide a good memory response. However, the central memory cells do not infiltrate into tissue well. In one embodiment the percentage of peripheral memory cells (CD45RA- CCR7-) based on the total number of T-cells at least 2 %, preferably at least 5 %, more preferably at least 10 %. Peripheral memory cells show high cytokine production and infiltrate well into tissue. Thus, these cells are in particular useful for immunotherapy against cancer.

Also terminally differentiated T-cells (CD45RA+ CCR7-) are able to enter into a tissue. Moreover, these cells show a production of therapeutically useful cytokines. According to one embodiment the percentage of effector T-cells (CD45RA+ CCR7-) based on the total number of T-cells is at least 1 %, preferably at least 3 %, more preferably at least 5 %. The percentage of precursor T-cells (CD45RA+ CCR7+) based on the total number of T-cells may be at least 1 %. Preferably the percentage is at least 2 %, more preferably at least 3 %. Precursor T-cells show only cytokine production and barely enter in tissue. However, these cells may be reverted T-cells with a lifelong memory effect.

The population of lymphocytes according to the fifth aspect may be further characterized by an intracellular cytokine production after stimulation with a evaluation antigen having a value that is at least twofold of the standard deviation without evaluation antigen stimulation. Moreover, the stimulation with the evaluation antigen leads to a value of CD107a induction that is at least twofold of the standard deviation without evaluation antigen stimulation.

Due to these parameters the population of lymphocytes and/or the population of clinically relevant lymphocytes represent useful immunotherapy products for the treatment of cancers, infectious diseases and autoimmune diseases.

According to a fifth aspect the invention provides an immunotherapy for treating or preventing a tumor disease, an infectious disease or an autoimmune disease in a mammal comprising the steps generating a clinically relevant lymphocyte population according to the second aspect of the invention wherein the body sample is obtained from said mammal and administering the clinically relevant lymphocyte population to said mammal. The infectious disease may be any known infectious disease relating to any known pathogen.

The tumor disease according to the invention can be any cancer, including any of acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the vulva, chronic lymphocytic leukemia, chronic myeloid cancer, cervical cancer, glioma, Hodgkin lymphoma, hypopharynx cancer, kidney cancer, larynx cancer, liver cancer, lung cancer, malignant mesothelioma, melanoma, multiple myeloma, nasopharynx cancer, non-Hodgkin lymphoma, ovarian cancer, peritoneum, omentum, mesentery cancer, pancreatic cancer, pharynx cancer, prostate cancer, rectal cancer, renal cancer, skin cancer, soft tissue cancer, testicular cancer, thyroid cancer, ureter cancer, urinary bladder cancer, and digestive tract cancer such as, e.g., esophageal cancer, gastric cancer, pancreatic cancer, stomach cancer, small intestine cancer, gastrointestinal carcinoid tumor, cancer of the oral cavity, colon cancer, and hepatobiliary cancer. Preferred cancers are glioblastoma and pancreatic cancer..

In order to avoid immune effects against the clinically relevant lymphocytes, the cancer patients may "conditioned" before administering the T-cells. The conditioning has three purposes. First is to provide more space for the infused lymphocyte population, second to remove adverse effectors and third to increase production of growth factors that may favor the rapid expansion and survival of T-cells, i.e. autologous IL-7 and IL-15 production.

The clinically relevant lymphocytes can be mixed with a pharmaceutically acceptable carrier to form a pharmaceutical composition, which is also within the scope of the present disclosure. In one embodiment, the clinically relevant lymphocytes may be autologous to the subject, i.e., the clinically relevant lymphocytes are obtained from the subject in need of the treatment, and then administered to the same subject.

Administration of autologous cells to a subject may result in reduced rejection of the host cells as compared to administration of non-autologous cells. Alternatively, the host cells are allogeneic cells, i.e., the cells are obtained from a first subject and administered to a second subject that is different from the first subject but of the same species. For example, allogeneic clinically relevant lymphocytes may be derived from a human donor and administered to a human recipient who is different from the donor.

To practice the methods disclosed herein, an effective amount of the clinically relevant lymphocytes described herein, or compositions thereof, can be administered to a subject (e.g., a human cancer patient, a patient with an infectious disease, a patient with an autoimmune disease) in need of the treatment via a suitable route, such as, for example, intravenous administration. The cells may be introduced by injection, catheter, or the like. If desired, additional drugs (e.g., cytokines) may also be co-introduced or introduced sequentially. Any of the cells or compositions thereof may be administered to a subject in an effective amount. As used herein, an effective amount refers to the amount of the respective agent (e.g., the cells or compositions thereof) that upon administration confers a desirable therapeutic effect on the subject. Determination of whether an amount of the cells or compositions described herein achieved the desired therapeutic effect would be evident to one of skill in the art. For example, see references 2-5. Effective amounts vary, as recognized by those skilled in the art, depending on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. In some embodiments, the effective amount alleviates, relieves, ameliorates, improves, reduces the symptoms, or delays the progression of the desired disease or disorder in the subject.

"Administering" refers to the physical introduction of a composition comprising a cell or pharmaceutical composition described herein to a subject, using any of the various methods and delivery systems known to those skilled in the art.

The administering of the population of tumor reactive lymphocytes preferably is an introduction into the patient either locally in proximity of a tumor or into the tumor. Alternatively the population of tumor reactive lymphocytes is administered into the blood circuit. Other clinically relevant lymphocytes are preferably added into the blood circuit.

According to an embodiment of the present invention, the subject is administered with a dose of clinically relevant lymphocytes comprising at least about, $4 \times 10^6$, $4.5 \times 10^6$, $5 \times 10^6$, $5.5 \times 10^6$, $6 \times 10^6$, $6.5 \times 10^6$, $7 \times 10^6$, $7.5 \times 10^6$, $8 \times 10^6$, $8.5 \times 10^6$, $9 \times 10^6$, $9.5 \times 10^6$, $10 \times 10^6$, $12.5 \times 10^6$, $15 \times 10^6$, $20 \times 10^6$, $25 \times 10^6$, $30 \times 10^6$, $35 \times 10^6$, $40 \times 10^6$, $45 \times 10^6$, $50 \times 10^6$, $60 \times 10^6$, $70 \times 10^6$, $80 \times 10^6$, $90 \times 10^6$ of cells per kilogram body weight. {please expand to other ranges as appropriate}

The immunotherapy according to the fifth aspect population of clinically relevant lymphocytes

- brings about regression of cancer cells in the mammal;
- interferes with the move from pre-malignant to malignant lesions;
- brings about fast senescence of tumor cells or pre-malignant cells;
- brings about removal of autoantigen-positive cells;
- brings about killing, growth arrest or containment of pathogens;

- interferes with cancer stem cells; and/or
- induce growth arrest of cancer cells, or cells expressing auto-antigens.

According to a sixth aspect the invention provides a composition according to the first aspect of the invention for use in the medical treatment, in particular for treating and preventing an infectious disease, an autoimmune disease or a tumor disease.

According to one embodiment of the sixth aspect the use comprises a generation of a population of clinically relevant lymphocytes with a method according to the second aspect of the invention.

According to one embodiment of the composition according to the sixth aspect the use comprises an immunotherapy according to the fifth aspect of the invention. As dicussed above, the particular cytokines of the cytokine cocktail not necessarily have to be used simultaneously but may be added at a different time point.

According to a seventh aspect the invention provides the kit for use in medical treatment, in particular for treating or preventing an infectious disease, an autoimmune disease or a tumor disease, wherein the kit comprises IL-2, IL-15 and IL-21. The kit further optionally comprises at least one of a component that stimulates the TCR, in particular OKT3, costimulatory molecules, feeder cells and a peptide comprising the amino acid sequence of clinically relevant antigen. Preferably the kit comprises all of the mentioned components.

The invention is further defined by the following examples.

## EXAMPLES

## Example 1 - Lymphocytes expansion protocol using peripheral blood mononuclear cells

A) Materials and equipment

i) Equipment

    24 well plate, (Becton, Dickinson (BD), REF 353504)
    Sterile scalpel
    Sterile tweezers
    Medimachine (tissue homogenate machine), (BD, Cat:340588)
    Medicon, 50$\mu$m, (BD, Cat:340591)
    Filcons, 200 $\mu$m (BD, Cat:340613)
    Laminar flow hood (Class 2 biosafety hood)
    Low temperature freezer, -80 °C
    Refrigerator
    Centrifuge

ii) Supplies

    Gloves (latex)
    Lab coat
    Sterile centrifuge tubes, 15mL.
    Pipet tips
    Pipette aid
    Waste container

iii) Reagents

    RPMI 1640, (Gibco, REF 61870-044)
    Cellgro, (CellGenix, Cat:20801-0100)
    Pooled human AB serum (Innoative Research, IPLA-SerAB-13458).
    Fetal Bovine Serum (Gibco, REF: 26140-079).

Anti-CD3 antibody (OKT3), (Biolegend, Cat:317304).
Human IL-2 (Prospec, Cat: CYT-209-b).
Human IL-15 (Prospec, Cat:cyt-230-b).
Human IL-21 (Prospec, Cat:cyt-408-b)
PEST (antibiotics)
Amphotericin

B) Procedure

Aphaeresis was performed on a healthy donor primed with NY-ESO-1. After separation of the blood components the product containing the leucocytes is separated from the rest. Cells are suspended in Cellgro comprising 5 % pooled human AB serum, 1000 U/ml IL-2, 10 ng/ml IL-15 and 10 ng/ml IL-21. The medium in addition contained 10 $\mu$mol NY-ESO-1 peptide as expansion antigen. The concentration of cells from. The expansion of lymphocytes from peripheral blood contained the step of stimulation by irradiated autologous PBMCs pulsed with the antigen of interest. For this, autologous PBMCs were pulsed for two hours with 10 mM NY-ESO-1 peptide at RT, and then irradiated at 55 Gy. The pulsed irradiated autologous PBMCs were added to the lymphocyte culture on day 7 and re-suspended in fresh culture medium supplemented with 5% human pooled AB serum with cytokines in concentrations as defined above. On day 10 cells were expanded with Cellgro supplemented with 5 % pooled human AB serum and cytokines. OKT3 antibody was added in a concentration of 30 ng/ml in the same culture medium described as above plus irradiated feeder cells (55Gy) at a 1:10 (feeder cell: T-cell ratio) the peptide and cytokines. On day 17 to 20 of culturing the cells are harvested for analysis or transferred to the patient.

**Example 2 Lymphocytes expansion protocol using peripheral blood mononuclear cells**

The procedure of Example 2 is identical to the procedure of Example 1 only that the donor was a patient who received vaccination with NY-ESO-1.

**Example 3 - Lymphocytes expansion protocol using peripheral blood mononuclear cells**

Example 3 is identical to Example 1 only that the donor is a patient whose tumor already expressed the TAA NY-ESO-1.

**Example 4 - gamma delta T-cells can enriched in expanded lymphocytes with the cytokines IL-2, IL-15 and IL-21**

The expansion is carried out according to the protocol described in Example 1 with the difference that zoledronic acid was added to the cell culture in a concentration of 5 $\mu$Mol. Zoledronic acid is known to promote the expansion of TCR gamma delta T-cells. As shown in Fig. 1 this experimental setup leads to a strong increase of TCR gamma delta T-cells in both frequency and absolute numbers. In Fig. 1 flow cytometry images of the expanded culture at different times are shown. Accordingly on the first day of expansion 3.68 % of he cells carry the TCR gamma delta. There is already a slight increase on the second day. Strikingly, after 7 days of expansion almost 50 % of T-cell are the $\gamma\delta$ subset.

**Example 5 - Expansion of the lymphocytes with the cytokine cocktail induces double negative T-cells (CD3+CD4-CD8-).**

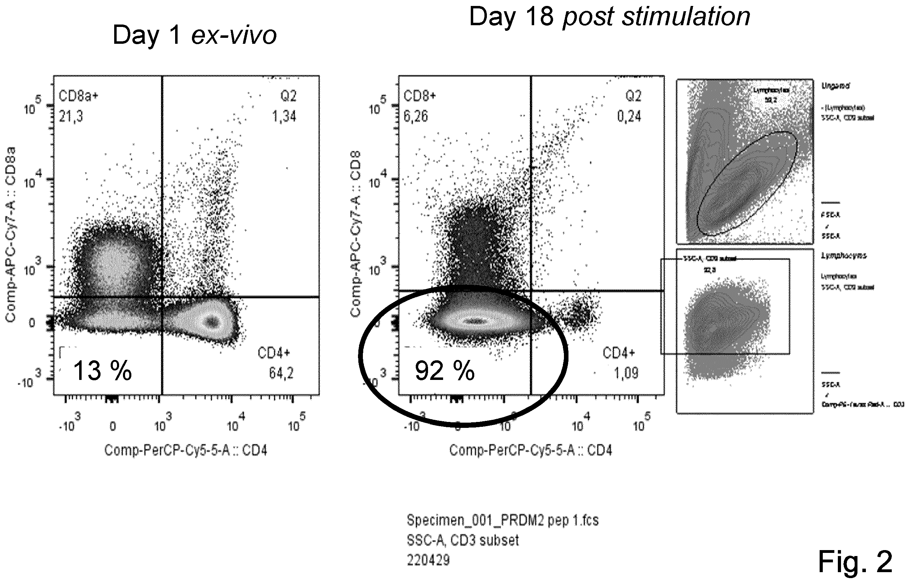

Lymphocytes were obtained from a patient with narcolepsy. The expansion was carried out as described in Example 1 but with the PRDM2 peptide as expansion antigen and stimulating.. The cultured cells were tested for the T-cell phenotype on day 1 and day 18. Fig. 2 shows the flow cytometry results of these samples. In the results first the lymphocytes are filtered and then the CD3+ lymphocytes are filtered. These cells are then further analyzed for the presence of CD4 and CD8. From the panels it can be seen that after day 1 only 13 % of the cells are in the double negative state. The majority of the cells are CD4+. After 18 days of expansion with the cytokine cocktail 92 % of the cells are double negative cells (CD3+CD4-CD8-) T-cells.

Furthermore, the induction of IFN-y production of the expanded lymphocytes upon PRDM2 peptide stimulation is tested. As shown in Fig. 3 on day 1 only 0.89 % of the cells produce IFN-y upon PRDM2 stimulation. In contrast, the sample from day 18 shows a significantly increased population of cytokine producing PRDM2 specific T-cells. As most of the T-cells are in the CD3+CD4-CD8- state, this shows that the expansion protocol leads to the formation of clinically relevant double negative T-cells.

**Example 6 - Sorted Dextramer cells produce IFN-y in response to autologous EBV-LCLs loaded with peptide and protein antigens**

The expansion protocol according to Example 1 was carried out with blood from two different patients with narcolepsy again with PRDM2 as expansion peptide. In two different setups per patient either DNAseB or PRDM2 peptides were added to the cytokine cocktail in the culture medium. CD3+CD4-CD8- and conventional CD8+ T-cells that were MHC restricted and directed against PRDM2 or DNAseB were sorted by flow cytometry and tested whether they recognize naturally processed and presented epitopes. T-cells from the same patients were pulsed with peptides or with recombinant proteins and IFN-y production was measured. The results of the experiment are summarized in Table 1:

| | Stimulus | Sorted Dextramers – % Frequency | DNAseB WT | DNAseB MUT | PRDM2 WT | DNAseB Protein | PRDM2 Protein | T Cells + Autologous B Cells |
|---|---|---|---|---|---|---|---|---|
| Patient 1 | DNAseB | PRMD2 CD3+ DN- 0.27% | 10.7 | 1.2 | 6.6 | 1.0 | 23.0 | 0.05 |
| | | DNAseB CD3+ DN- 0.29% | 0.7 | 7.7 | 1.5 | 3.1 | 3.1 | 0.07 |
| | PRDM2 | PRMD2 CD3+ DN- 0.25% | 0.0 | 0.0 | 2.3 | 0.0 | 0.0 | 0.10 |
| | | DNAseB CD3+ DN- 0.45% | 5.3 | 0.6 | 0.4 | 1.8 | 2.7 | 0.0 |
| Patient 2 | DNAseB | PRMD2 CD8+- 0.70% | 0.4 | 0.2 | 0.4 | 2.3 | 1.6 | 0.01 |
| | | DNAseB CD8+- 0.26% | 1.1 | 0.9 | 0.4 | 2.5 | 1.1 | 0.04 |
| | PRDM2 | PRMD2 CD8+- 0.28% | 0.9 | 25.8 | 10.7 | 1.3 | 6.5 | 0.0 |
| | | DNAseB CD8+- 0.32% | 22.6 | 7.0 | 14.8 | 11.0 | 7.9 | 0.0 |

Table 1

The measured values are the IFN-y concentration in pg/ml. This experiment shows that the cytokine cocktail of IL-2, IL-15 and IL-21 in combination with a specific antigen is able to expand clinically relevant T-cells, in particular T-cells specific for the antigen in peripheral blood. The T-cells can preferentially reside in the so-called DN CD3+CD4-CD8- T-cell population and these T-cells are highly functionally active, they produce IFN-y and recognize biologically and medically relevant targets.

**Example 7 - Analysis of lymphocytes expanded with the cytokine cocktail and tumor associated antigens**

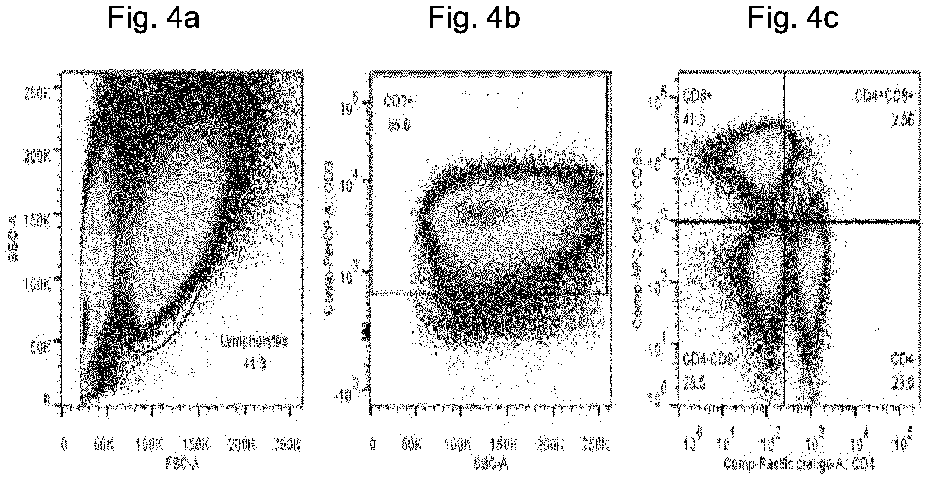

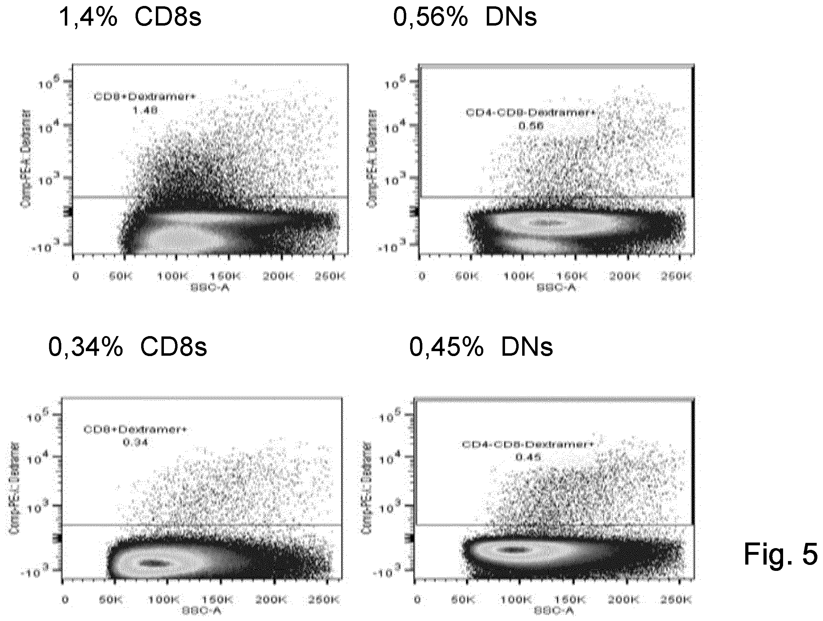

The expansion procedure was carried out as described in Example 1 with the exception that INO80E and UCHL3 were used instead of NY-ESO-1 as expansion antigen. After 18 days of expansion the cells were stimulated with either INO80E or UCHL3 analyzed by flow cytometry. The results of the flow cytometry are shown in Figs. 4 and 5. Fig. 4A shows the signal of the cells separated by sideward and forward scattering. Lymphocytes are gated as shown by the black elliptic circle. The filtered lymphocytes are then again filtered for the presence of CD3. The CD3+ cells were further analyzed by a separation according to the presence or non-presence of CD8 and CD4. In this experiment 41 % of the cells are CD8+, 29,6 % CD4+ and 26,5 % double negative. The double negative and CD8+ were then tested for IFN-γ production upon stimulation with the target antigens INO80E and UCHL3. Note that the T-cells are objectively measured using MHC class 1/peptide complexes. According to the results shown in Fig. 5 A 1.4 % of the CD8+ cells produce IFN-y upon INO80E stimulation but also 0.56 % of the double negative T-cells produce IFN-y upon stimulation (see Fig. 5 B.). Similar results are obtained for UCHL3 activation as shown in Fig. 5 0.34 % of the CD8+ cells produce IFN-y. Also 0.45 % of the double negative cells produce IFN-y (see Fig. 5D).

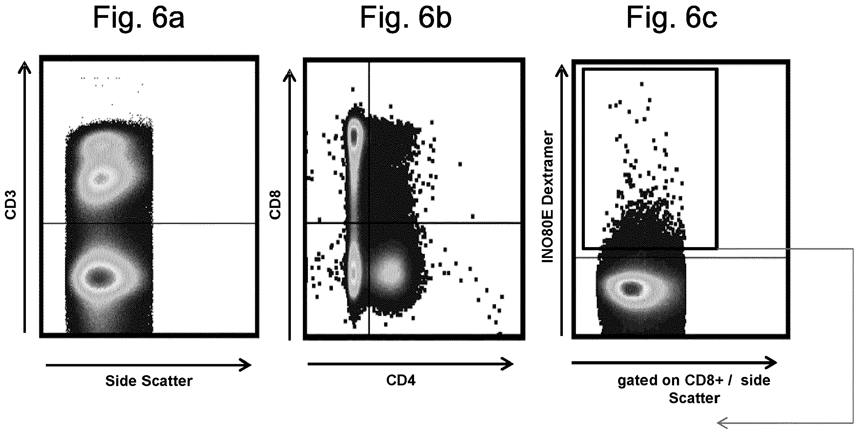

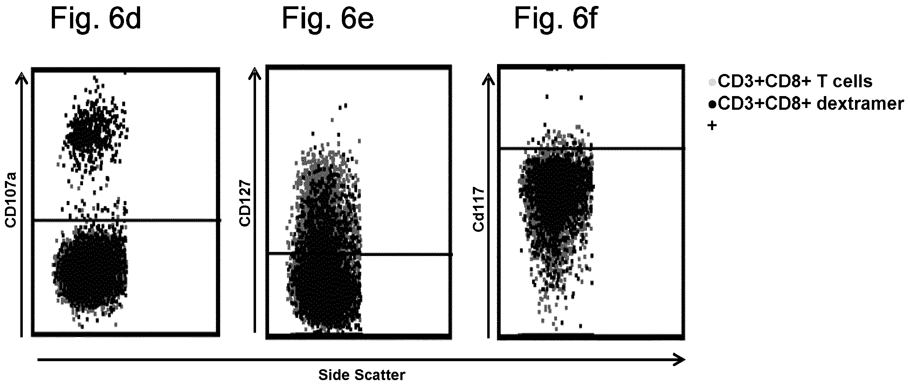

The cells stimulated with INO80E were further analyzed for the production of cytokines. Fig. 6A to 6C again shows the gating of CD8+ IFN-y producing T-cells. The analysis of cells producing CD107a, CD127 (IL-7R) and CD117 are shown in Fig. 6D to 6F. Grey signals stand for the entire CD3+CD8+ T-cell population (irrespective of the antigen-specificity), , black signals for CD3+CD8+ T-cells that are antigen-specific. The analysis shows that the TAA reactive CD8+ T-cells express CD107a, a marker associated with ccytotoxicity and CD127 (the Il-7R receptor, mediating survival signal signals), but not CD117, a marker for T-cells with stem-cell like properties..

**Example 8 - Expansion of PBMCs with the cytokine cocktail and the CMVpp65 peptide**

PBMCs were obtained from a patient with glioblastoma. and were expanded with the cytokine cocktail of IL-2, IL-15 and IL-21 according to Example 1 but in combination with CMVpp65 peptide. This experiment shows that the CMVpp65 peptide stimulation in combination with the cytokine cocktail of IL-2, IL-15 and IL-21 leads to a strong expansion of high affinity antigen specific T-cells (tetramer analysis). Results are shown in Fig. 7A to 7D. APC-CMV bzw. PE-CMV und FITC-CMV stand for i) identical MHC class I-HLA-0201 molecules, ii) identical peptides. But the MHC molecule is mutated. The only difference is the mutation in the MHC molecule - which detects T-cells with different affinity.. The CMV tetramers are differently mutated. APC-CMV (medium affinity, mutation in the position 245; between PE-CMV (high affinity) und FITC-CMV (wild type, comparably low affinity). The data show that mutant tetramers that only allow high affinity T-cell receptors for binding detect high affinity T-cells (as well as T-cells with low and intermediate affinity) High affinity T-cells are believed to mediate stronger immune effector functions and to be superior in removing tumor cells and/or pathogens.

**Example 9 - Analysis of the frequency of tumor reactive T-cells after expansion of lymphocytes from peripheral blood with the cytokine cocktail and NY-ESO-1**

PBMCs were obtained from a patient with pancreatic cancer. The experiment was carried out as described in Example 1. Fig. 8 shows a flow cytometry analysis of samples of the expansion on day 0 and day 18. While on day 1 the IFN-y production upon NY-ESO-1 stimulation is only 1.85, this concentration increases drastically to 9.25 on day 18 (see Fig. 8B and 8D). Thus the number of NY-ESO-1 specific T-cells is drastically increased during expansion.

**Example 10 - Expansion of survivin reactive T-cells from peripheral blood from patients with cancer**

PBMCs were obtained from a patient with glioblastoma and were expanded with the cytokine cocktail of IL-2, IL-15 and IL-21 according to Example 1 but together with the known TAA survivin. Again cells were analyzed on day 1 and after 18 days of culture using flow cytometry. Before analysis the expanded cells were stimulated with survivin. Cells were grouped into CD4+, CD8+ and double negative T-cells. In these groups the concentration of IL-2, IFN-$\gamma$ and TNF-$\alpha$ was determined. Fig. 9A shows the results for CD4+ cells, Fig. 9B the results for the double negative and Fig. 9C the results for the CD8+ cells. The results can be summarized as follows: there are no detectable T-cell responses, defined by cytokine production at time point 0 (T0). In contrast, after 18 days of expansion strong cytokine production is found in response to stimulation by survivin proving the presence of survivin specific T-cells. In this regard it has to be noted that T-cell responses are generally very difficult to induce against survivin.

**Example 11 - Analysis of the phenotype of expanded lymphocytes**

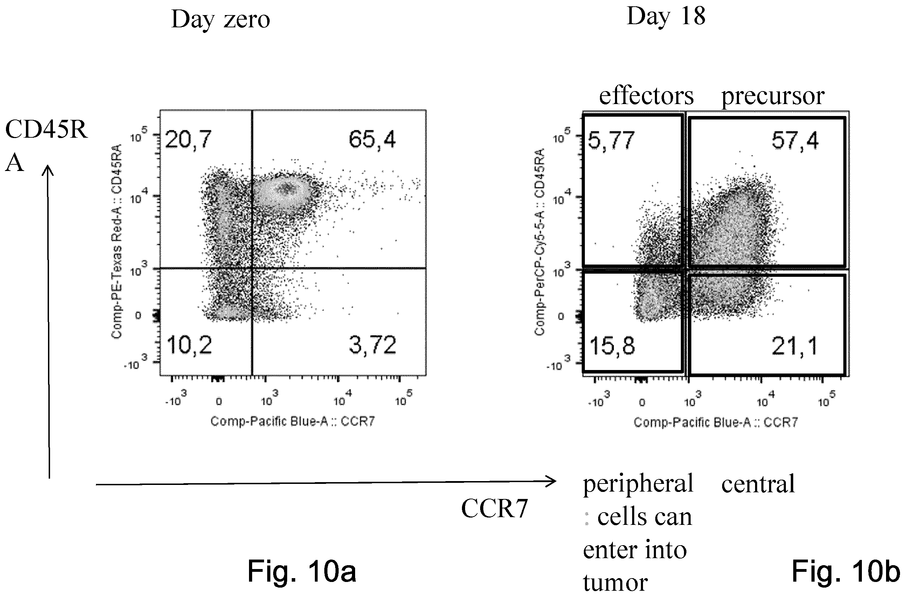

PBMCs were obtained from a patient with glioblastoma. The expansion was carried out as described in Example 1. Samples from day 0 and day 18 were analyzed according to the presence of CD45RA and CCR7 using flow cytometry. The results are shown in Fig. 10A and Fig. 10B. For this experiment, cells were grouped into the following groups. Positive signals for CD45RA and CCR7 upper right section of the graph stands for precursor cells. CCR7+ CD45RA-lower right is central memory cells. CD45RA positive signal with CCR7- upper left area are the effector cells and the negative signal in both regions lower left area are the peripheral memory cells. As shown in comparison of Fig. 10A and Fig. 10B it is obvious that the number of central memory cells due to expansion with the cytokine cocktail strongly increases, i.e. form 3.72 to 21.1. The number of peripheral memory cells slightly increases while the number of effector cells strongly decreases. The number of precursor cells only slightly decreases from 65.4 to 57.4. Accordingly, expansion with the cytokine combination according to the invention enriches central memory T-cells.

**Example 12 - Analysis of the differentiation of different T-cell subsets upon expansion with the cytokine cocktail of IL-2, IL-15 and IL-21 together with different TAA**

PBMCs from patients with pancreas cancer were treated according to Example 1 with different antigens: GPI which is the cell surface bound part of mesothelin, survivin and NY-ESO-1. The results are summarized in the following Table 2:

Table 2

| | T-cell population | Antigen for stimulation | Central memory CD45RA-CCR7+ | | Precursor CD45RA+CCR7+ | | Effector T-cells CD45RA+CCR7- | | Peripheral memory CD45RA-CCR7- | |
|---|---|---|---|---|---|---|---|---|---|---|
| | | | Timepoint 0 | After stimulation | Timepoint 0 | After stimulation | Timepoint 0 | After stimulation | Timepoint 0 | After stimulation |
| **Patient X** | **CD4+** | **GPI** | **12,80** | **24,30** | **44,50** | **24,00** 12,20 | **7,93** | **2,85** | **34,80** | **48,70** |
| | | NY-ESO-1 | | 29,10 | | | | 3,71 | | 54,90 |
| | | Survivin | | 15,50 | | 4,24 | | 1,89 | | 78,40 |
| | CDB+ | GPI | 0,61 | 4,80 | 26,70 | 50,90 | 57,60 | 25,50 | 15,10 | 18,80 |
| | | NY-ESO-1 | | 10,90 7,20 | | 11,30 | | 11,50 | | 66,30 |
| | | Survivin | | | | 8,98 | | 14,50 | | 69,30 |
| | CD4-CD8- | GPI | 1,81 | 4,31 | 29,20 | 19,90 | 58,80 | 40,40 | 10,20 | 35,40 |
| | | NY-ESO-1 | | 6,90 | | 7,44 | | 66,80 | | 18,90 |
| | | Survivin | | 5,49 | | 7,10 | | 57,60 | | 29,80 |

In the first column the T-cell subset to be analyzed is shown: CD4+, CD8+, CD4- and CD8-. In the second column the antigen stimulus is defined. In the following columns the percentage of central memory, precursor, differentiated effector cells and peripheral memory cells is shown at day 0 and day 18 of the different experiments. Accordingly, some antigens such as mesothelin particularly drive the expansion of precursor cells and defined by CD45RA+ CCR7+ and central memory cells defined by CD45RA- CCR7+.

**Example 13 - Enrichment of precursor T-cells and c-kit expression**

PBMCs from patient with glioblastoma were expanded according to the protocol of Example 1 with peptides of the antigens (EGRvlll, NY-ESO-1 or survivin). The cells were stimulated with the same peptides analyzed by flow cytometry and the results are summarized in Table 3:

Table 3

| | Patient 1 | | | | Patient 2 | | | | Patient 3 | | | |
|---|---|---|---|---|---|---|---|---|---|---|---|---|
| | EGFRV | NY-ESO-1 | Survivin | TIMEPOINT 0 | EGFRVIII | NY-ESO-1 | Survivin | TIMEPOINT 0 | EGFRV | NY-ESO-1 | Survivin | TIMEPOINT 0 |
| Lymphocites | 71.2 | 60,3 | 72,7 | 83,2 | 74 | 48,8 | 51,9 | 68,2 | 71,4 | 67.1 | 62,8 | 85,1 |
| CD3+ | 76 | 54.6 | 75,9 | 46,4 | 65,4 | 60,8 | 60,9 | 48,9 | 57,1 | 63,8 | 66,5 | 53,0 |
| CD4+ | 11 | 27,4 | 11,1 | 42,5 | 35 | 63,4 | 61,2 | 47,8 | 9,05 | 24,9 | 23,6 | 35,8 |
| | 22,4 | 55,2 | 21,9 | 0,6 | 33,5 | 75,8 | 71,3 | 2,17 | 6,44 | 27,6 | 30,3 | 0,29 |
| CD107a+ | 5,62 | 17,5 | 5,32 | 0,98 | 2,69 | 14,8 | 13 | 1,47 | 2,56 | 8,5 | 12,2 | 0,69 |
| | | | | | | | | | | | | |
| Q1: CCR7-CD45RA+ | 5,58 | 10,5 | 5,8 | 23 | 81 | 56,5 | 53,3 | 52,8 | 1,49 | 7,67 | 6,67 | 4,46 |
| Q2:CCR7+CD45RA+ | 59,1 | 13 | 56,5 | 44,2 | 13 | 35,9 | 40,1 | 32,8 | 58,6 | 36,7 | 43,1 | 41,3 |
| Q3: CCR7+CD45RA- | 15,9 | 27,6 | 16,8 | 11,3 | 1,01 | 3,18 | 3,22 | 7,4 | 20,7 | 20,5 | 21,1 | 14,7 |
| Q4: CCR7-CD45RA- | 19,4 | 48,9 | 20,9 | 21,5 | 5 | 4,48 | 3,44 | 7,02 | 18,2 | 35,1 | 29,2 | 39,5 |
| CD8+ | 33,5 | 28,5 | 33,7 | 20,9 | 27.6 | 11,3 | 8,82 | 13,3 | 24,3 | 30 | 34,8 | 36 |
| c-kil+ | 40.6 | 33,4 | 40,7 | 0.66 | 23 | 90,8 | 91,9 | 1,08 | 11.4 | 49,3 | 32,7 | 0,078 |
| CD8aa | 29,9 | 48,3 | 33,7 | 49 | 42,1 | 49 | 50,2 | 40,4 | 23,9 | 16 | 5,61 | 32,7 |
| CD8ab | 66,5 | 48,4 | 62,3 | 47 | 53,9 | 46,6 | 45,7 | 55,9 | 73,8 | 82,2 | 93,5 | 64,4 |
| CD107a+ | 1,32 | 5,12 | 1,18 | 1.45 | 0.77 | 15 | 15,1 | 4 | 2,12 | 4,55 | 5,6 | 2,44 |
| | | | | | | | | | | | | |
| Q1: CCR7-.CD45RA+ | 15,8 | 42,2 | 14,1 | 51,6 | 92,4 | 75,8 | 69,7 | 68,9 | 14,5 | 15 | 30,2 | 37,1 |
| Q2: CCR7+.CD45RA+ | 9,11 | 9,98 | 7,66 | 25,3 | 3,8 | 15,1 | 24,4 | 24,6 | 23,9 | 41,1 | 50,1 | 37.3 |

| | Patient 1 | | | | Patient 2 | | | | Patient 3 | | | |
|---|---|---|---|---|---|---|---|---|---|---|---|---|
| | EGFRV | NY-ESO-1 | Survivin | TIMEPOINT 0 | EGFRVIII | NY-ESO-1 | Survivin | TIMEPOINT 0 | EGFRV | NY-ESO-1 | Survivin | TIMEPOINT 0 |
| Q3: CCR7+. CD45RA- | 5,02 | 3,51 | 5,8 | 2,47 | 0,11 | 0,84 | 1,19 | 2,34 | 2,34 | 6,41 | 2,53 | 1,96 |
| Q4: CCR7-. CD45RA- | 70,1 | 44,3 | 72,5 | 20,6 | 3,73 | 8,26 | 4,76 | 4,13 | 59,2 | 37,5 | 17,1 | 23,6 |
| DN | 54,7 | 38 | 54,6 | 36,2 | 36,3 | 15,2 | 22,4 | 38,6 | 66,4 | 37,2 | 31,5 | 27,9 |
| c-kll+ | 11,1 | 9,35 | 11,4 | 2,1 | 5,88 | 24,9 | 46,9 | 1,72 | 5,05 | 15,4 | 5,05 | 0,23 |
| CD10Ta+ | 5,36 | 14,2 | 4,39 | 2,5 | 3,12 | 19 | 13,7 | 3,93 | 3,37 | 15,5 | 17,6 | 3,76 |
| | | | | | | | | | | | | |
| Q1: CCR7- .CD45RA+ | 22,2 | 39.6 | 20,6 | 46,4 | 88,8 | 54,8 | 67 | 54,5 | 30,4 | 20,6 | 13.3 | 51.3 |
| Q2: CCR7+. CD45RA+ | 3,9 | 12,4 | 3,34 | 32,1 | 4,43 | 21,8 | 19,8 | 37,4 | 1,77 | 19,9 | 36,7 | 10,6 |
| Q3: CCR7+.CD45RA- | 4,02 | 7,93 | 4,05 | 5,41 | 0,76 | 8,17 | 3,13 | 4,11 | 1,52 | 10,5 | 15,3 | 3,13 |
| Q4: CCR7-CD45RA- | 69,9 | 40,1 | 72 | 16,2 | 5,99 | 15,2 | 10,1 | 4,02 | 66,3 | 49 | 34,7 | 35 |

EP 3 838 288 A1

The numbers are percentages of the T-cell populations in the parental CD4+, CD8+, or double negative phenotype for each of the respective antigens at time point 18 days with comparison to time point 0 (start of the expansion). An enrichment of precursor T-cell subsets, defined by CD45RA+ CCR7+ associated with antigen and strong expression of c-kit (CD117) is detected. C-kit is a marker for T-cells with stem-cell like features. CD117+ T-cells have been associated to provide long-term memory population. Strong induction of the cytotoxicity/degranulation marker CD107a in stimulated T-cells is also observed.

Example 14 - The expansion protocol with the cytokine cocktail and TAA peptide leads to 4-1BB and TIM-3 positive T-cells

PBMCs were derived from patients with pancreatic cancer and expanded according to Example 1. Again TAA peptides were derived from GPI, NY-ESO-1 and survivin. Using flow cytometry the presence of different markers was tested. The markers are: 4-1BB, CD25, CD127, CTLA-4, LAG3, PD1 and TIM3. The results are summarized in Table 4:

Table 4

| Patient | Cell | Antigen | 4-1 BB T0 | 4-1 BB Ag | CD 25+ T0 | CD 25+ Ag | CD 127+ T0 | CD 127+ Ag | CTL A-4+ T0 | CTL A-4+ Ag | LAG 3+ T0 | LAG 3+ Ag | PD 1+ T0 | PD 1+ Ag | TIM 3+ T0 | TIM 3+ Ag |
|---|---|---|---|---|---|---|---|---|---|---|---|---|---|---|---|---|
| Patient A | CD4+ | GPI | 0,03 | 0,33 | 6,47 | 37,00 | 66,70 | 36,80 | 0,01 | 0,01 | 0,16 | 0,18 | 8,22 | 26,10 | 0,00 | 0,02 |
| | | NY-ESO-1 | | 5,96 | | 25,80 | | 45,30 | | 0,00 | | 0,28 | | 25,70 | | 0,17 |
| | | Survivin | | 2,17 | | 32,10 | | 37,50 | | 0,01 | | 0,24 | | 25,80 | | 0,61 |
| | CD8+ | GPI | 0,04 | 0,03 | 0,03 1,46 | 23,80 | 23,40 | 1,77 | 0,10 | 0,06 | 0,85 | 0,29 | 7,43 | 39,20 | 0,00 | 0,01 |
| | | NY-ESO-1 | | 0,15 | | 39,50 | | 2,14 | | 0,10 | | 0,41 | | 44,80 | | 0,03 |
| | | Survivin | | 0,11 | | 37,10 | | 2,47 | | 0,15 | | 0,56 | | 39,70 | | 0,03 |
| | CD4- CD8- | GP1 | 0,04 | 0,14 | 0,89 | 28,10 | 12,90 | 1,05 | 0,07 | 0,02 | 0,32 | 0,17 | 12,00 | 23,70 | 0,00 | 0,06 |
| | | NY-ESO-1 | | 0,89 | | 40,70 | | 2,06 | | 0,04 | | 0,17 | | 11,60 | | 0,29 |
| | | Survivin | | 0,41 | | 50,10 | | 1,84 | | 0,03 | | 0,23 | | 12,50 | | 0,43 |
| Patient B | CD4+ | GPI | 0,06 | 1,47 | 4,26 | 78,30 | 54,70 | 27,30 | 0,03 | 0,20 | 0,45 | 0,35 | 17,10 | 62,70 | 0,00 | 0,43 |
| | | NY-ESO-1 | | 1,80 | | 56,30 | | 19,20 | | 0,06 | | 0,42 | | 28,90 | | 0,25 |
| | | Survivin | | 3,78 | | 75,10 | | 27,50 | | 0,21 | | 0,43 | | 61,90 | | 0,51 |
| | CD8+ | GPI | 0,05 | 1,51 | 2,15 | 55,30 | 18,00 | 7,74 | 0,10 | 0,21 | 0,82 | 3,97 | 11,80 | 47,60 | 0,00 | 0,34 |
| | | NY-ESO-1 | | 1.52 | | 40,40 | | 6,07 | | 0,13 | | 1,16 | | 30,80 | | 0,16 |
| | | Survivin | | 2,11 | | 56,40 | | 7,45 | | 0,33 | | 1,78 | | 44,10 | | 0,39 |
| | CD4- CD8- | GP1 | 0,00 | 3,19 | 0,67 | 41,60 | 7,80 | 4,94 | 0,29 | 0,52 | 0,87 | 0,75 | 10,70 | 31,60 | 1,07 | 3,12 |
| | | NY-ESO-1 | | 3,40 | | 25,90 | | 6,49 | | 0,72 | | 0,48 | | 23,40 | | 2,01 |
| | | Survivin | | 2,50 | | 32,10 | | 1,01 | | 2,50 | | 0,44 | | 29,50 | | 0,00 |

In the Table 4 T0 stands for the concentration of the individual subsets defined in column 2, prior to expansion (T0). Ag stands for 18 days of antigenic stimulation and cytokine-driven expansion. The 4-1BB values and TIM3 values are indicative for antigen experienced T-cells. It is found that in particular 4-1BB+ T-cells are strongly increased in some of the experiments and cellular subsets. For example in the CD4+ subset stimulation with NY-ESO-1 leads to an 200-fold increase in 4-1BB+ T-cells (from 0.03 to 5,96).

**Example 15 - Detailed analysis of cellular markers in lymphocytes expanded from peripheral blood of patients with glioblastoma**

The experiment was carried out according to the protocol of Example 1 with EGVRVIII, NY-ESO-1 or survivin, respectively. The results are summarized in Table 5.

Table 5

| | Stim Ag | | Patient 1 | | Patient 2 | | Patient 3 | | Patient 4 | | Patient 5 | |
|---|---|---|---|---|---|---|---|---|---|---|---|---|
| | | | T0 | Ag | T0 | Ag | T0 | Ag | T0 | Ag | T0 | Ag |
| **CD3+/ CD4+** | CD4+ | EGFRVIII | 40,4 | 12,2 | 70 | 39 | 36,2 | 10,9 | 48,8 | 44,3 | 65,6 | 74,4 |
| | | NY-ESO-1 | | 30,5 | | 62,5 | | 30,6 | | 63,1 | | 74,8 |
| | | Survivin | | 24,8 | | 70,5 | | 33,5 | | 63,7 | | 72,2 |
| | 4-1 BB+ | EGFRVIII | 0,13 | 1,39 | 0,16 | 1,85 | 0,032 | 0,39 | 0,28 | 0,91 | 0,083 | 1,47 |
| | | NY-ESO-1 | | 26,5 | | 50,2 | | 16,9 | | 3,47 | | 2,78 |
| | | Survivin | | 34,2 | | 43,4 | | 14,6 | | 11,9 | | 4,02 |
| | CD25+ | EGFRVIII | 2,76 | 31,8 | 6,43 | 50,6 | 4,01 | 12 | 10,1 | 84,3 | 4,73 | 86,9 |
| | | NY-ESO-1 | | 66 | | 85,7 | | 29,6 | | 60,5 | | 97,5 |
| | | Survivin | | 56,4 | | 88,2 | | 30,6 | | 76 | | 94,2 |
| | CD127+ | EGFRVIII | 77,6 | 77,5 | 69,5 | 34,7 | 88,5 | 84,9 | 35,7 | 16,4 | 42 | 8,08 |
| | | NY-ESO-1 | | 60,4 | | 49 | | 64,3 | | 34,3 | | 2,08 |
| | | Survivin | | 55,8 | | 54 | | 71,5 | | 35,8 | | 6,77 |
| | CTLA-4+ | EGFRVIII | 0,27 | 0,88 | 1,04 | 1,79 | 0,19 | 0,44 | 1,64 | 1,19 | 1,28 | 3,82 |
| | | NY-ESO-1 | | 6,87 | | 7,59 | | 0,93 | | 3,03 | | 3,66 |
| | | Survivin | | 4,91 | | 7,43 | | 1,46 | | <u>7,28</u> | | 7,04 |
| | LAG3+ | EGFRVIII | 10,7 | 7,71 | 12,5 | 10,1 | 4,05 | 4,68 | 3,66 | 2,73 | 2,21 | 3,06 |
| | | NY-ESO-1 | | 20,8 | | 26,3 | | 10,8 | | 4,65 | | 2,58 |
| | | Survivin | | 20,5 | | 17 | | 13,9 | | 5,98 | | 3,79 |
| | PD-1 | EGFRVIII | 7,1 | 21,9 | 37,8 | 34,2 | 14,8 | 9,13 | 3,99 | 29,3 | 4,64 | 84,5 |
| | | NY-ESO-1 | | 55,8 | | 50,2 | | 15,1 | | 39,4 | | 87,2 |
| | | Survivin | | 47,1 | | 55,4 | | 17,2 | | | | 90,5 |
| | TIM3+ | EGFRVIII | 5E-03 | 0,066 | 5E-03 | 0,056 | 0 | 0,016 | 0,13 | 0,35 | 0,044 | 0,75 |
| | | NY-ESO-1 | | 0,34 | | 0,36 | | 0,063 | | 0,52 | | 0,066 |
| | | Survivin | | 0,28 | | 0,27 | | 0,5 | | 1,71 | | 0,21 |

(continued)

| | | Stim Ag | Patient 1 | | Patient 2 | | Patient 3 | | Patient 4 | | Patient 5 | |
|---|---|---|---|---|---|---|---|---|---|---|---|---|
| | | | T0 | Ag | T0 | Ag | T0 | Ag | T0 | Ag | T0 | Ag |
| CD3+/ CD8+ | CD8+ | EGFRVIII | 18,2 | 34,7 | 19,6 | 30,8 | 39,6 | 25,3 | 20,1 | 34,9 25,5 22,2 | 9,55 | 16 |
| | | NY-ESO-1 | | 33,9 | | 9,63 | | 33,2 | | | | 13,2 |
| | | Survivin | | 36,2 | | 11,1 | | 45 | | | | 15,1 |
| | 4-1 BB+ | EGFRVIII | 1,64 | 0,78 | 1,16 | 0,49 | 0,66 | 0,47 | 0,067 | 0,96 7,57 16,2 | 0,17 | 1,93 |
| | | NY-ESO-1 | | 13,3 | | 24,8 | | 12,5 | | | | 10,6 |
| | | Survivin | | 12,4 | | 25,2 | | 11 | | | | 11,6 |
| | CD25+ | EGFRVIII | 5,58 | 59,3 | 5,98 | 44,1 | 1,59 | 25,8 | 3,47 | 90,1 50,9 65 | 1,4 | 86,9 |
| | | NY-ESO-1 | | 43 | | 97,6 | | 65,2 | | | | 96 |
| | | Survivin | | 45,4 | | 94,6 | | 44,4 | | | | 94,4 |
| | CD127+ | EGFRVIII | 68,8 | 32,6 | 62,7 | 30 | 67,4 | 43,6 | 42,5 | 15,6 37 37,1 | 52,6 | 21,6 |
| | | NY-ESO-1 | | 47,6 | | 57,1 | | 49,4 | | | | 12,1 |
| | | Survivin | | 42,9 | | 53,1 | | 57,1 | | | | 19,6 |
| | CTLA-4+ | EGFRVIII | 1,32 | 1,17 | 2,16 | 1,48 | 1,37 | 1,62 | 12 | 8,46 19,4 18,3 | 14,3 | 24,2 |
| | | NY-ESO-1 | | 3,58 | | 8,89 | | 2,08 | | | | 15,6 |
| | | Survivin | | 2,91 | | 7,09 | | 2,91 | | | | 28 |
| | LAG3+ | EGFRVIII | 79 | 92,3 | 88,3 | 94,5 | 91,9 | 96,1 | 15,9 | 12,6 25,6 26,5 | 18,9 | 26,2 |
| | | NY-ESO-1 | | 94,5 | | 93,1 | | 98,3 | | | | 14,3 |
| | | Survivin | | 95,5 | | 94,6 | | 98,1 | | | | 24,6 |
| | PD-1 | EGFRVIII | 7,53 | 18,2 | 26,3 | 39,5 | 4,63 | 14,4 | 6,04 | 21,4 26,4 37 | 5,34 | 56,5 |
| | | NY-ESO-1 | | 28,3 | | 44 | | 18,2 | | | | 80,4 |
| | | Survivin | | 29,4 | | 41,9 | | 12,8 | | | | 82,7 |
| | TIM3+ | EGFRVIII | 0 | 0,21 | 0,016 | 0,11 | 7E-03 | 0,15 | 0,12 | 0,69 1,72 4,13 | 0,13 | 2,93 |
| | | NY-ESO-1 | | 0,061 | | 0,11 | | 0,27 | | | | 1,2 |
| | | Survivin | | 0,19 | | 0,2 | | 1,75 | | | | 3,96 |

Again the lymphocytes are separated according to the presence of CD4 or CD8+. The numbers in the table refer to percentages of cells positive for the individual markers listed in the second column in the table at T0 or after 18 days of expansion (Ag). Again for most patients a strong induction of 4-1BB+ T-cells is found.

Example 16 - Analysis of lymphocytes expanded from a patient with pancreatic cancer and from a patient with glioblastoma

PBMCs were obtained from a patient with pancreatic cancer and from a patient with glioblastoma. The lymphocytes were expanded with the protocol according to Example 1. The lymphocyte culture is analyzed by flow cytometry against a variety of markers. The results are represented in Table 6:

Table 6

| | | Pane | | GBM | |
|---|---|---|---|---|---|
| | | T0 | TH | T0 | TH |
| **CD3** | | 64 | 69,9 | 70 | 88,6 |
| **CD4** | | 59,1 | 31,2 | 64 | 11 |
| | CCR6 Neg | 88,9 | 97,4 | 95,9 | 86,6 |
| | TH1 | 9,69 | 97,1 | 4,82 | 38,8 |
| | TH2 | 0,23 | 0 | 0,64 | 0,2 |
| | CCR6 Pos | 10,1 | 2,03 | 3,25 | 12 |
| | **TH1*** | **22,2** | **92,1** | **9,64** | **68,1** |
| | TH17 | 1,05 | 0 | 1,02 | 0,52 |
| | CCR7-CD45RA+ | 14,5 | 13,8 | 2,69 | 2,06 |
| | CCR7+ CD45RA+ | 12,1 | 0,25 | 49 | 61,9 |
| | CCR7+ CD45RA- | 10,9 | 0,2 | 21,2 | 23,3 |
| | CCR7- CD45RA- | 62,5 | 85,7 | 27,1 | 12,7 |
| | C-KIT | 0,52 | 0,051 | 0,77 | 3,12 |
| | CD107a | 0,4 | 0,14 | 0,8 | 4,39 |
| **CD8** | | 33,5 | 64,5 | 30,3 | 71,2 |
| | CXCR3+ | **6,67** | **96,8** | **17** | **85,5** |
| | CCR7- CD45RA+ | 61,1 | 30,8 | 20,1 | 7,39 |
| | CCR7+ CD45RA+ | 9,91 | 0,87 | 66,8 | 66,2 |
| | CCR7+ CD45RA- | 2,29 | 0,082 | 2,52 | 10,9 |
| | CCR7- CD45RA- | 26,6 | 68,2 | 10,5 | 15,5 |
| | C-KIT | 2,17 | 0,22 | 2,85 | 4,34 |
| | CD107a | 3,45 | 0,34 | 4,23 | 5,66 |
| **DN** | | 6,15 | 3,47 | 4,56 | 13,7 |
| | CXCR3+ | 8,67 | 87,3 | 24 | 58,3 |
| | CCR7- CD45RA+ | 73,1 | 37,2 | 41,7 | 15,7 |
| | CCR7+ CD45RA+ | 5,63 | 0,093 | 20,2 | 7,12 |
| | CCR7+ CD45RA- | 3,44 | 0,46 | 5,95 | 5,6 |
| | CCR7- CD45RA- | 17,8 | 62,3 | 32,1 | 71,6 |
| | C-KIT | 4,33 | 0,51 | 1,67 | 2,24 |
| | CD107a | 8,91 | 1,2 | 1,67 | 2,9 |

In the first column it is indicated what subgroup of lymphocytes was filtered. In the second column the respective tested markers are listed. If no marker is listed this line represents the percentage of total number of cells with the filter marker CD3+ based on the total number of cells and the CD4+, CD8+ or double negative. Again T0 stands for the culture prior to expansion and TH for the time of harvest at day 18. The numbers represent the percentage of cells carrying the selected markers in column 2 or column 1 respectively. It has to be noted that majority of CD4+ cells after expansion is TH1 positive. In pancreatic cancer the numbers increase from 22.2 to 92.1 % of the CD4 cells. In glioblastoma the results are only little less pronounced from 9.64 to 68.1 % of the CD4 cells. Likewise, moreover there is a strong expression of CXCR3+ in CD8+ T-cells after expansion 96.8 % in pancreas and 85.5 % in glioblastoma. Accordingly almost all of the CD8+ cells are CXCR3+.

Moreover it is noted a shift away from terminally differentiated T-cells into precursor or central memory T-cell subsets.

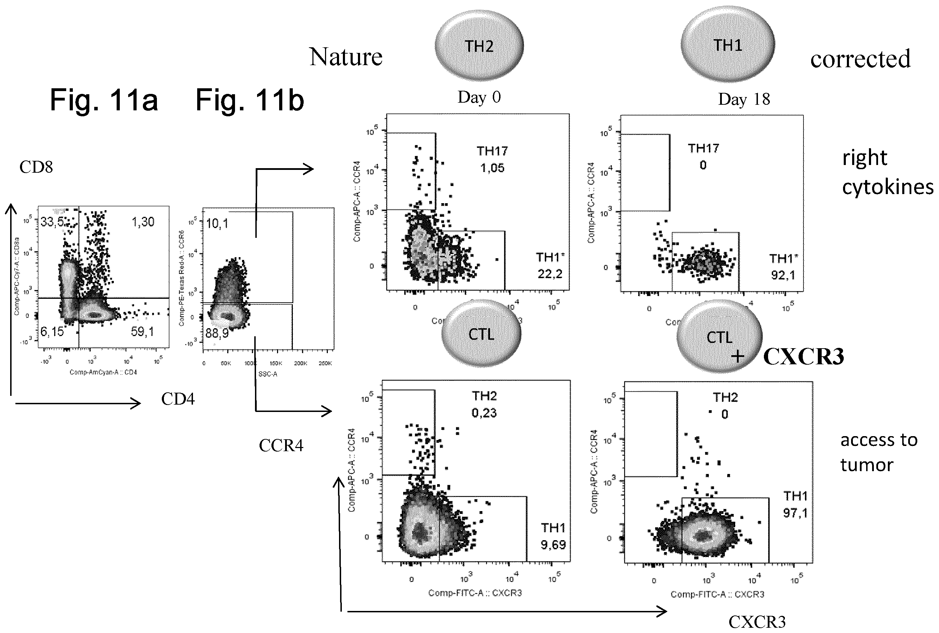

Fig. 11 shows flow cytometry graphs of the experiment for the determination of TH17, TH1, TH1* and TH2 in the sample from the pancreas cancer patient First the cells are gated on CCR6 and then on CCR3 and CCR4 respectively.

**Example 17 - Expansion of memory T-cells specific for certain antigens in the absence of stimulation with these antigens**

PBMCs from patients with pancreas cancer were expanded according to Example 1 with the tumor associated antigen NY-ESO-1. The resulting cells were stimulated for four hours with the following tumor associated antigens: CMV, EBNA-3a, INO80E, mesothelin, NY-ESO-1, survivin und UCHL3. Using flow cytometry the percentage of cytokine producing T-cells was determined. The numbers are represented in Table 7 for different T-cell subsets: CD8+, CD4+ and double negative. The following cytokines were tested: IFN-$\gamma$, IL-2 and TNF. Interestingly, not only stimulation with NY-ESO-1 led to cytokine producing T-cells and therefore tumor reactive T-cells but also stimulation with other antigens that were not used in the expansion process. Also stimulation with antigens of viral targets (CMV and EBNA-3a) led to T-cells producing cytokines showing the presence of T-cells specific for these targets.

**Table 7**

| AFTER EXPANSION | | AFTER MEDIUM SUBSTRACTION | | | | | | | |
|---|---|---|---|---|---|---|---|---|---|
| | | PMA | CMV | EBNA-3a | INO80E | Mesothelin | NY-ESO-1 | Survivin | UCHL3 |
| **CD8+** | IFN+ | 20.03 | 0.97 | 1.56 | 0.00 | 0.10 | 0.14 | 0.07 | 0.13 |
| | IL-2+ | 3.84 | 0.06 | 0.02 | 0.00 | 0.04 | 0.03 | 0.04 | 0.06 |
| | TNF+ | 21.61 | 1.49 | 1.46 | 0.07 | 0.41 | 0.27 | 0.25 | 1.01 |
| **CD4+** | IFN+ | 10.08 | 0.00 | 0.00 | 0.00 | 0.00 | 0.00 | 0.00 | 0.00 |
| | IL-2+ | 14.39 | 0.22 | 0.06 | 0.09 | 0.16 | 0.15 | 0.10 | 0.33 |
| | TNF+ | 10.29 | 0.00 | 0.00 | 0.00 | 0.02 | 0.00 | 0.00 | 0.65 |
| **DN** | IFN+ | 5.53 | 0.05 | 0.14 | 0.00 | 0.00 | 0.00 | 0.00 | 0.00 |
| | IL-2+ | 5.89 | 0.20 | 0.14 | 0.02 | 0.09 | 0.22 | 0.19 | 0.23 |
| | TNF+ | 4.82 | 1.15 | 0.36 | 0.34 | 1.22 | 0.59 | 0.56 | 2.18 |

**Example 18 - Cytotoxicity after HPV L1 specific expansion**

In this example peripheral blood was obtained from a patient who suffered from severe HPV disease (HPV33 and HPV56). The stimulation was performed with the HPV L1 peptide and the cytokine cocktail comprising IL-2, IL-15 and IL-21. After 18 days of expansion the cells were stimulated with L1 peptide and measured by flow cytometry. The results of this are shown in Fig. 12. Fig. 12D to 12F show the gating for lymphocytes CD3+ and CD8+ respectively. In Figs. 12A, 12B and 12C the rectangle marks the area showing cells that express cytokine CD107a, a marker for degranulation/cytotoxicity that is antigen-specific. Fig. 12A the results obtained with the L1 peptide, Fig. 12B a positive control and Fig. 12C just medium as negative control. Accordingly there is an increase from 0.77 to 2.23 in the percentage of cells producing CD107a.

**Example 19 - T-cells expanded with the cytokine cocktail and tumor antigens recognize autologous tumor cells**

PBMCs from a patient with glioblastoma were stimulated with NY-ESO-1 peptides and CMVpp65 peptides. Peripheral blood from a patient with glioblastoma was treated according to the protocol of Example 4 wherein in two setups the stimulating peptide was either NY-ESO-1 or CMVpp65. Again after 18 days a cytokine production assay was performed using different antigens for stimulation or the autologous tumor cells. It was found that antigen-specific expansion of T-cells using peptides and the cytokine cocktail leads to expansion of T-cells directed against the stimulating target, yet also against the patient's own autologous tumor cells. This reactivity was not present prior to expansion of T-cells with NY-ESO-1 and cytokines. The data is summarized in Table 8. Numbers represent percentages of T-cells present in the parental T-cell population.

**Table 8**

| GBM | | PMA | | NY-ESO-1 | | Survivin | | CMV | | Tumor | | Medium | |
|---|---|---|---|---|---|---|---|---|---|---|---|---|---|
| | | T0 | TH | T0 | TH | T0 | TH | T0 | TH | T0 | TH | T0 | TH |
| CD4 + | IFN-γ | 16,3 5 | 45,2 0 | 0,00 | 0,33 | 0,00 | 0,30 | 0,00 | 0,66 | 0,00 | 0,23 | 0,00 | 0,11 |
| | IL-2 | 50,5 8 | 64,1 5 | 0,00 | 0,15 | 0,00 | 0,05 | 0,00 | 0,08 | 0,05 | 0,21 | 0,02 | 0,05 |
| | IL-17 | 0,69 | 2,09 | 0,02 | 0,08 | 0,01 | 0,11 | 0,00 | 0,01 | 0,04 | 0,21 | 0,03 | 0,09 |
| | TNF -α | 45,3 8 | 68,8 4 | 0,00 | 0,42 | 0,00 | 0,20 | 0,00 | 0,45 | 0,10 | 0,28 | 1,68 | 0,42 |
| CD8 + | IFN-Y | 13,0 5 | 87,1 6 | 0,01 | 0,91 | 0,01 | 1,20 | 0,04 | 2,53 | 0,00 | 0,82 | 0,00 | 0,64 |
| | IL-2 | 28,6 9 | 67,2 1 | 0,03 | 0,07 | 0,01 | 0,10 | 0,02 | 0,10 | 0,02 | 0,08 | 0,01 | 0,04 |
| | IL-17 | 0,08 | 0,46 | 0,04 | 0,07 | 0,04 | 0,18 | 0,04 | 0,13 | 0,00 | 0,12 | 0,01 | 0,08 |
| | TNF -α | 14,2 8 | 63,9 9 | 0,00 | 0,36 | 0,00 | 0,45 | 0,00 | 1,13 | 0,00 | 0,15 | 2,02 | 0,41 |
| CD4- CD8- | IFN-γ | 8,11 | 63,7 7 | 0,00 | 2,89 | 0,00 | 3,20 | 0,25 | 7,73 | 0,07 | 2,38 | 1,89 | 0,00 |
| | IL-2 | 56,0 2 | 41,4 2 | 0,03 | 0,32 | 0,00 | 0,39 | 0,25 | 0,10 | 0,30 | 0,40 | 0,13 | 0,14 |
| | IL-17 | 0,22 | 0,11 | 0,16 | 0,02 | 0,00 | 0,12 | 0,15 | 0,04 | 0,32 | 0,15 | 0,06 | 0,04 |
| | TNF -α | 28,7 8 | 48,8 0 | 0,00 | 1,88 | 0,17 | 2,15 | 0,16 | 5,14 | 0,00 | 1,11 | 1,95 | 1,03 |

**Example 20 - Comparison of lymphocyte expansion with two different cytokine combinations and TAA stimulation**

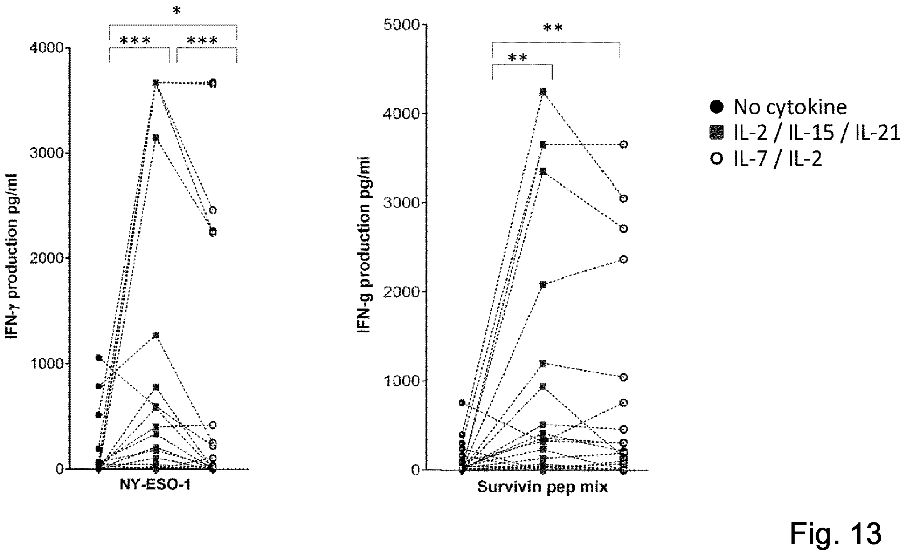

Peripheral blood from patients with pancreatic cancer was obtained and treated according to the protocol of Example 4. In different experimental setups either NY-ESO-1 or survivin was used together with the cytokine cocktail. In addition the same experiments were carried out without the addition of cytokines or with a combination of IL-7 and IL-2. After 18 days of expansion the cells were tested for their IFN-γ production upon stimulation with either NY-ESO-1 or survivin peptide mix. The results are presented in Fig. 13. Fig. 13 shows strongly increased IFN-γ production and thus concentration of antigen-specific lymphocytes after expansion with IL-2, IL15 and IL-21 in comparison to the other cytokine composition or no cytokines.

**Example 21 - Comparison of lymphocyte expansion with two different cytokine combinations and TAA stimulation**

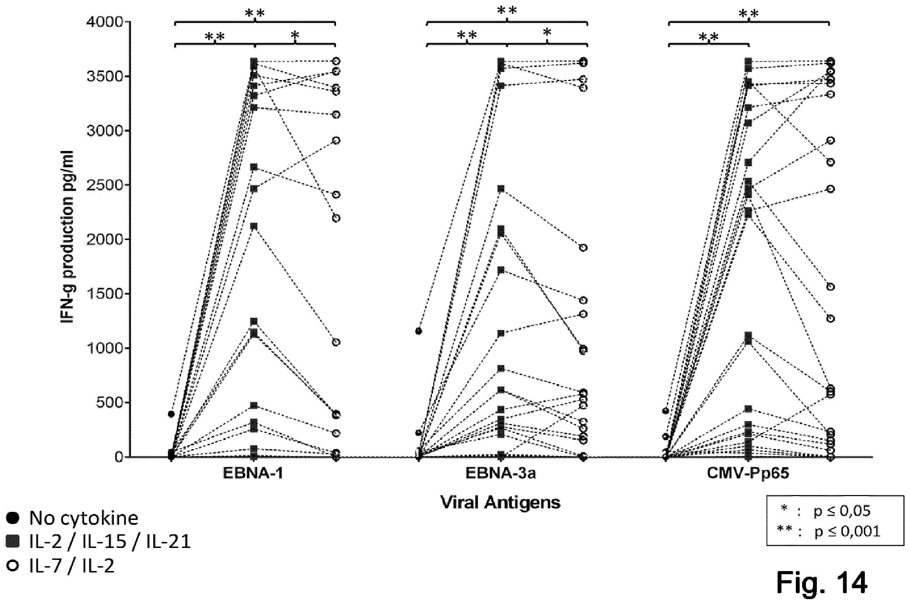

The experiment according to Example 20 was repeated with the viral antigens EBNA-1, EBNA-3a and CMVpp65. The results are summarized in Fig. 14. Fig. 14 shows strongly increased IFN-γ production and thus concentration of antigen-specific lymphocytes after expansion with IL-2, IL15 and IL-21 in comparison to the other cytokine composition or no cytokines.

**Example 22 - Analysis of Treg expansion with the cytokine cocktail**

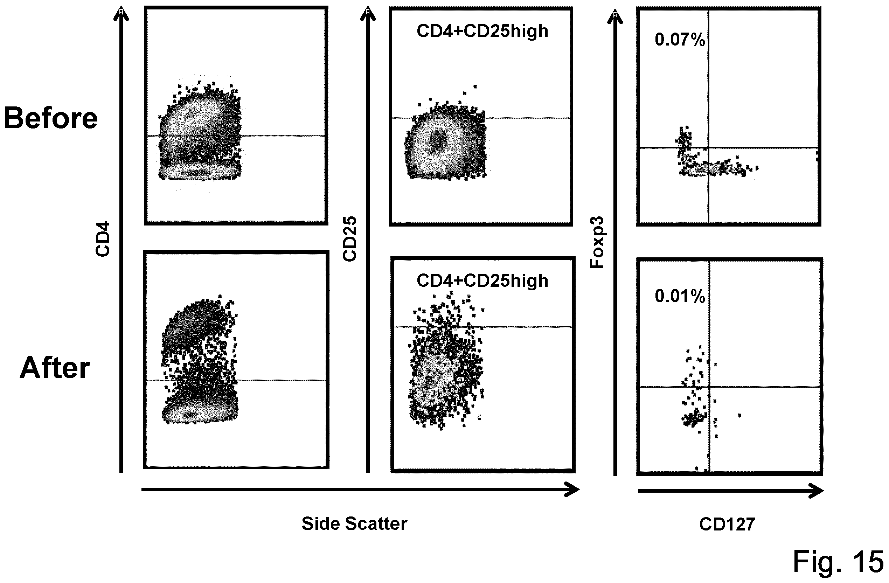

T-cells derived from PBMCs were cultured in the presence of the cytokine cocktail IL-2, IL-15 and IL-21 and Treg (regulatory T-cells) were identified prior to and after expansion of T-cells using flow cytometry. The results are shown in Fig. 15. In Fig. 15 from left to right: the gating of the T-cells on CD4+ T-cells and then on CD25high designating the high expression of the IL-2 receptor on activated T-cells is shown. Then the cells were gated on II-2R (high CD125) cells and tested for expression of the IL-7Receptor (CD127) and Foxp3 (intracellularly). A Treg cell is defined as CD4+CD25high, Foxp3+ and CD127-negative. The number of Treg was initially low (0.07% in CD4+ T-cells) and was even lower after T-cell expansion (0.01%). The data imply that the cytokine cocktail is superior in T-cell expansion since IL-2 is known to drive strong Treg expansion, it also underlines the synergistic effects of the IL-2/IL-15/IL-21 combination that blocks Treg expansion, while allowing anti-tumor directed T-cells to expand.

**Example 23 - Analysis of VB family distribution after expansion**

T-cells were expanded using the cytokine mix IL-2/IL-15/IL-21 along with the NY-ESO-1 antigens for 21 days. A flow cytometric analysis has been performed before (T0 = time point 0) and after expansion (TH = Time of harvest). T-cells stimulated with cytokines alone served as the control. The results are summarized in Table 9. Note the strong preferential expansion of the VB7.2 family in the CD8+ T-cells expanded in the GRex flask, as well as the VB13.2 family in the CD8+ T-cells (also expanded in the GRex flask). The data show that the presence of the antigen (NY-ESO-1) drives preferential expansion of individual TCR VB families - and shows also the diversity of the response driven by the antigen (not only by the cytokines). Focused, yet diverse TCR VB families suggest a diverse anti-target directed T-cell response.

| | | CD4+ | | CD8+ | |
|---|---|---|---|---|---|
| | | T 0 | TH | T 0 | TH |
| Vβ1 | G-Rex | | 0,23 | | 0,54 |
| | Cyto | 1,14 | 1,71 | 0,96 | 1,68 |
| | GE | | 1,18 | | 1,01 |
| Vβ2 | G-Rex | | 0,57 | | 1,55 |
| | Cyto | 6,74 | 1,68 | 3,54 | 5,06 |
| | GE | | 7,35 | | 0,10 |
| Vβ3 | G-Rex | | 0,13 | | 10,70 |
| | Cyto | 3,84 | 1,35 | 19,00 | 10,70 |
| | GE | | 1,15 | | 6,05 |
| Vβ4 | G-Rex | | 0,20 | | 1,30 |
| | Cyto | 1,68 | 0,61 | 4,53 | 3,92 |
| | GE | | 5,89 | | 4,78 |
| Vβ5.1 | G-Rex | | 0,94 | | 0,22 |
| | Cyto | 2,51 | 3,91 | 1,10 | 0,62 |
| | GE | | 0,98 | | 2,78 |
| Vβ5.2 | G-Rex | | 0,13 | | 0,10 |
| | Cyto | 0,37 | 0,43 | 0,18 | 0,17 |
| | GE | | 2,06 | | 0,30 |
| Vβ5.3 | G-Rex | | 0,14 | | 3,30 |
| | Cyto | 0,48 | 0,83 | 0,50 | 1,77 |
| | GE | | 2,58 | | 3,82 |
| Vβ7.1 | G-Rex | | 79,30 | | 0,53 |
| | Cyto | 5,47 | 31,60 | 0,66 | 0,96 |
| | GE | | 37,20 | | 1,19 |
| Vβ7.2 | G-Rex | | 0,90 | | 17,50 |
| | Cyto | 2,00 | 3,46 | 4,39 | 4,62 |
| | GE | | 5,84 | | 8,46 |
| Vβ8 | G-Rex | | 0,38 | | 0,63 |
| | Cyto | 2,83 | 2,58 | 1,39 | 2,92 |
| | GE | | 1,64 | | 5,38 |

| | | CD4+ | | CD8+ | |
|---|---|---|---|---|---|
| | | T 0 | TH | T 0 | TH |
| Vβ12 | G-Rex | | 0,12 | | 0,33 |
| | Cyto | 0,68 | 0,43 | 3,09 | 1,00 |
| | GE | | 2,31 | | 0,44 |
| Vβ13.1 | G-Rex | | 4,75 | | 1,16 |
| | Cyto | 7,42 | 2,65 | 1,96 | 0,93 |
| | GE | | 16,90 | | 1,90 |
| Vβ13.2 | G-Rex | | 2,65 | | 28,70 |
| | Cyto | 4,56 | 2,10 | 14,70 | 18,50 |
| | GE | | 16,70 | | 12,40 |
| Vβ13.6 | G-Rex | | 0,07 | | 6,30 |
| | Cyto | 0,85 | 0,79 | 0,71 | 0,76 |
| | GE | | 8,34 | | 10,00 |
| Vβ14 | G-Rex | | 1,50 | | 4,83 |
| | Cyto | 15,30 | 8,17 | 11,30 | 8,45 |
| | GE | | 15,50 | | 0,04 |
| Vβ16 | G-Rex | | 0,08 | | 0,09 |
| | Cyto | 0,53 | 0,20 | 0,33 | 0,05 |
| | GE | | 3,38 | | 0,72 |
| Vβ17 | G-Rex | | 0,27 | | 0,54 |
| | Cyto | 2,89 | 1,15 | 1,06 | 1,33 |
| | GE | | 7,07 | | 0,14 |
| Vβ18 | G-Rex | | 0,75 | | 0,03 |
| | Cyto | 0,81 | 2,05 | 0,29 | 0,12 |
| | GE | | 1,61 | | 1,77 |
| Vβ20 | G-Rex | | 0,42 | | 1,78 |
| | Cyto | 0,95 | 1,37 | 0,74 | 1,33 |
| | GE | | 1,17 | | 1,24 |
| Vβ21.3 | G-Rex | | 2,94 | | 0,31 |
| | Cyto | 3,70 | 3,07 | 0,32 | 0,66 |
| | GE | | 3,86 | | 0,55 |

| | | G-Rex | | 0,20 | | 2,83 | | | G-Rex | | 0,22 | | 0,12 |
|---|---|---|---|---|---|---|---|---|---|---|---|---|---|
| Vβ9 | | Cyto | 1,64 | 1,14 | 1,84 | 1,59 | Vβ22 | | Cyto | 2,97 | 1,38 | 1,03 | 0,58 |
| | | GE | | 3,45 | | 0,57 | | | GE | | 0,51 | | 0,02 |
| | | G-Rex | | 0,11 | | 0,25 | | | G-Rex | | 0,93 | | 0,58 |
| Vβ11 | | Cyto | 0,45 | 0,31 | 1,09 | 4,10 | Vβ23 | | Cyto | 0,20 | 3,06 | 0,34 | 2,17 |
| | | GE | | 0,13 | | 0,33 | | | GE | | 7,42 | | 12,30 |

Table 9. TH: Harvest timepoint, 21days; G-Rex: G-Rex flask, NY-ESO-1 stimulation; Cyto: Cytokine only, no stimulation; GE: GE wave system, NY-ESO-1 stimulation

## Example 24 - PD1 marker in CD8+ cells

T-lymphocytes have been expanded using the IL-2/IL-15/IL-21 and the NY-ESO-1 peptide mix. Top panel: prior, bottom panel: after cytokine/TAA expansion = T-cells have been cultured in the presence of IL-2/IL-15/IL-21. A flow cytometic analysis has been performed before and after T-cell stimulation. From Left to right: i) gating on lymphocytes, ii) gating on CD3+ T-cells. iii) gating on CD4/CD8. Top right: PD1 expression on CD8+ activated T-cells (37% T-cells). Cytokine / TAA-expanded T-cells show decreased PD1 expression on the CD4CD8+ T-cells. The data show that the cytokine/peptide expanded T-cells exhibit decreased frequency of PD1 + T-cells on CD8+ T-cells - that implies that these expanded T-cells may show longer life-time and therefore superior efficacy against tumor cells.

## Example 25 - Multiple Antigens

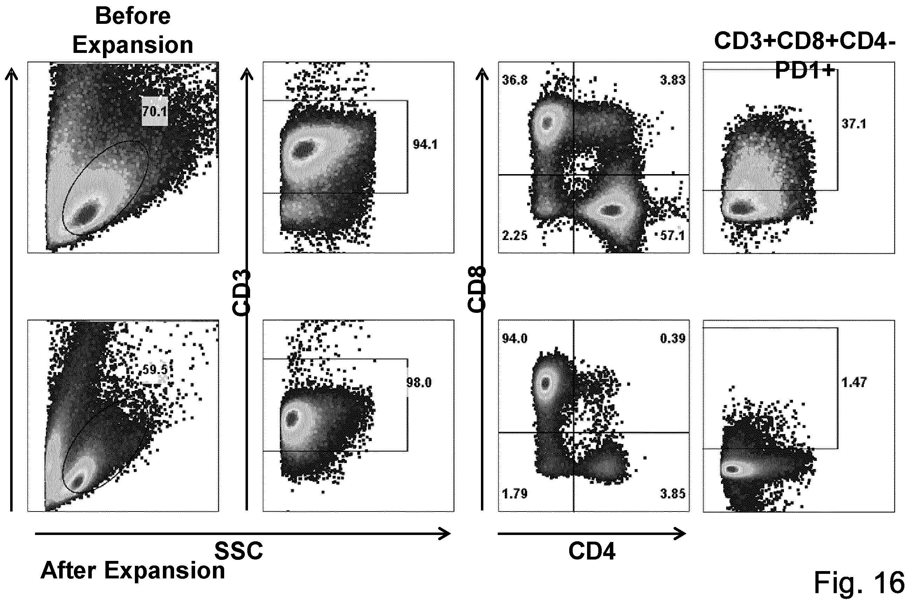

A peptide cocktail consisting of 12 individual peptides from the L1 protein from HPV 33, along with the cytokine cocktail, was used to stimulate T-cells from patients with HPV+ lesions. At day 21, the T-cells were tested for cytotoxicity directed against each individual peptide. Here, autologous EBV-transformed B-cells were taken and pulsed with peptides 1-12, followed by a standard Cr51 assay. This assays measure the capacity of T-cells to kill specific targets. The results are shown Fig 16.

T-cells from donor A recognize strongly peptides 11 and 12. T-cells from d B reccognized strongly peptides 7-12 with a strong reactivity against peptide 11. These data show that expansion of T-cells with the IL-2/IL-15/IL-21 cytokine cocktail and peptides leads to i) generation of cytotoxic T-cells and ii) that the T-cell response is diverse and focused to a variant set of individual peptide species. This implies that T-cells stimulated with the peptide/cytokine cocktail can be preferentially cytotoxic and they can also target several epitopes - which makes them more diverse in recognizing tumor cells, or virally infected cells, that display on their surface different sets of peptide species.

## Example 26 - CD117 expression before and after cytokine- and NY-ESO-1 driven expansion of PBMCs.

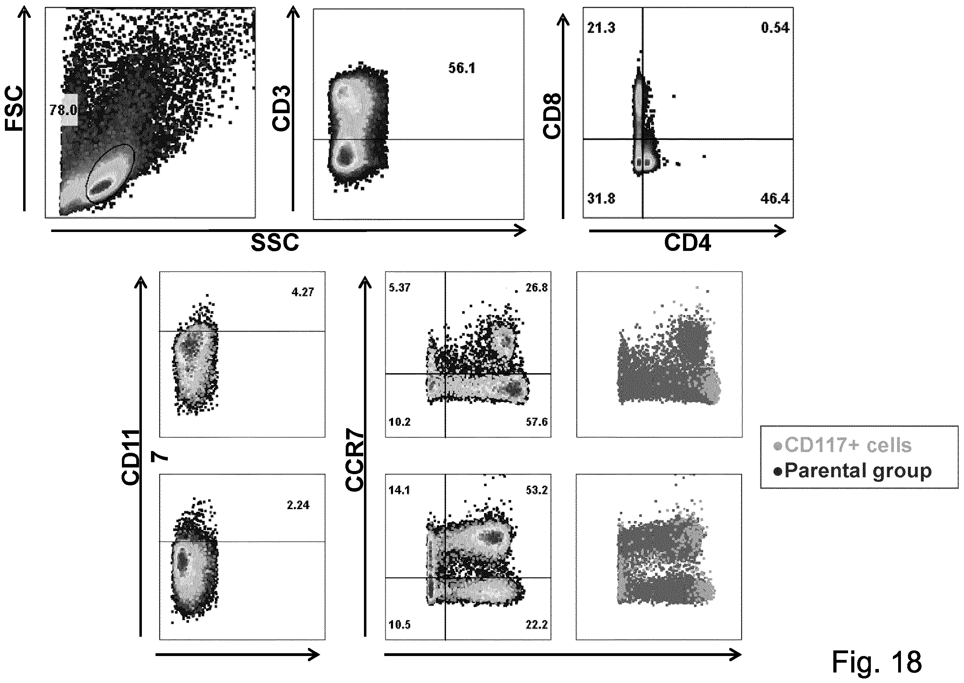

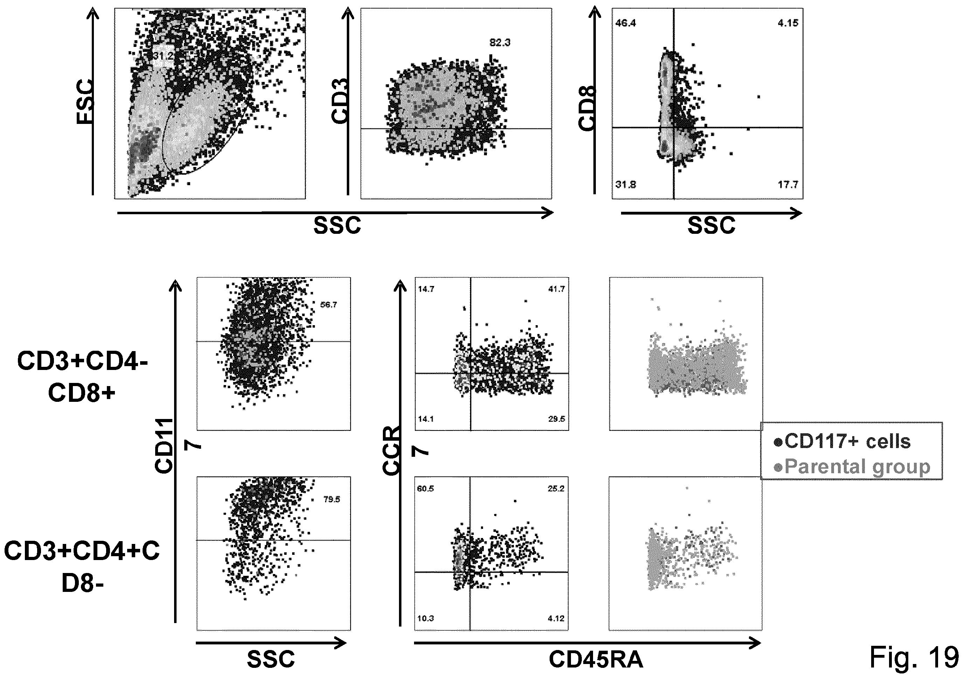

PBMCs were expanded with IL-2/IL-15/IL-21-mix in the presence of the tumor - associated antigen NY-ESO-1. The cells were analyzed by flow cytometry before (Fig. 18) and after expansion (Fig. 19). First, CD3+ T-cells are gated, then the CD3+ T-cells are gated on CD4+ and CD8+ T-cells. CD117 + cells were then detected on CD8+ T-cells (top) and on CD4+ T-cells (bottom panel, left). Middle panel: CD45RA/CCR7 expression in CD8+ and CD4+ T-cells with a high population on CD45RA+ CCR7+ T-cells, representing precursor Tcells (middle panel). Right: CD117+ T-cells (blue labeled) reside in effector T-cells in CD8+ T-cells; CD117+ T-cells can be found in different T-cell populations in the CD4+ T-cell subsets. The data implies that CD117 expression, a marker of T-cell 'stem-ness' present is present on different T-cell subsets. CD117+ T-cells are advantageous to provide a source for long-term T-cell memory.

First, CD3+ T-cells are gated, then the CD3+ T-cells are gated on CD4+ and CD8+ T-cells. Very strong expression and a high frequency of CD117 + cells on CD8+ T-cells (top) and on CD4+ T-cells (bottom panel, left). Middle panel: CD45RA/CCR7 expression in CD8+ and CD4+ T-cells with a high population on CD45RA+ CCR7+ T-cells, representing precursor T cells (middle panel). Right: CD117+ T-cells (blue labeled) reside in periphery effector T-cells in CD45RA+ CCR7- T-cells (effectors). CD84 T-cells; CD117+ T-cells can be found in the T-cell precursor CD45RA+CCR7+ population. The data implies that CD117 expression, a marker of T-cell 'stem-ness' present is present on different T-cell subsets. CD117+ T-cells are advantageous to provide a source for long-term T-cell memory. It also shows that the cytokine cocktail strongly expands CD117+ T-cells that bring about long-term immune cell memory, also that CD117+ T-cells reside in precursor T-cells - to ensure long-term tumor immune responses.

**Example 27 - Expansion of TIL culture from Glioblastoma**

This example describes the procedure for culturing TILs from pancreatic cancer for a function test and immunotherapy.

For material, equipment and supplies see Example 1.

Tumor tissue obtained from a patient was put into a sterile container. The tissue was cut into pieces of 1 to 2 mm$^3$ with a sterile scalpel. The tissue pieces were put into the wells of 24 well plate with 1 piece per well. Cell culture medium is prepared using Cellgro with 10 % human AB serum. Into this medium IL-2, IL-15 and IL-21 are added to a final concentration of 1000 u/ml for IL-2, 10 ng/ml for each of IL-15 and IL-21. Furthermore, PEST and amphotericin were added to the medium. 1 ml medium was added into each well and the cell culture was incubated for 7 days at 37° Celsius. In parallel PBMCs from a healthy donor were cultured in Cellgro containing 10 % human AB serum. The concentration of the PBMCs was determined and adjusted to a concentration of 10$^6$/ml. The PBMCs were then radiated for 18 minutes at 55 Gy. After Irradiation OKT3 was added to a final concentration of 10 ng/ml. This culture is referred to as feeder cells with OKT3. On day 10 of the TIL culture, 100 μl of the feeder cell culture with OKT3 is added to each of the wells of the 24 well plate. Accordingly, the culture in each of the well to the final concentration of 10 ng/ml OKT3 and a total amount of 10$^6$ feeder cells. The ratio of TILs and feeder cells is about 1:10.

**Example 28 - Expansion of TIL culture from Pancreatic Cancer.**

Tumor tissue obtained from a patient was put into a sterile container. The tissue was cut into pieces of 1 to 2 mm$^3$ with a sterile scalpel. The tissue pieces were put into the wells of 24 well plate with 1 piece per well. Cell culture medium is prepared using Cellgro with 10 % human AB serum. Into this medium IL-2, IL-15 and IL-21 are added to a final concentration of 1000 u/ml for IL-2, 10 ng/ml for each of IL-15 and IL-21. Furthermore, PEST and Amphotericin were added to the medium. 1 ml medium was added into each well and the cell culture was incubated for 4 days at 37° Celsius. In parallel PBMCs from a healthy donor were cultured in Cellgro containing 10 % human AB serum. The concentration of the PBMCs was determined and adjusted to a concentration of 10$^6$/ml. The PBMCs were then radiated for 18 minutes at 55 Gy. After Irradiation OKT3 was added to a final concentration of 10 ng/ml. This culture is referred to as feeder cells with OKT3. On day 10 of the TIL culture 100 μl of the feeder cell culture with OKT3 is added to each of the wells of the 24 well plate. Accordingly, the culture in each of the well contains the final concentration of 10 ng/ml OKT3 and a total amount of 10$^6$ feeder cells. The ratio of TILs and feeder cells is about 1:10.

**Example 29 - Procedure for generating a tumor cell line from tissue**

Pancreas tumor tissue is obtained directly after operation and put into a sterile container. The tumor tissue is cut into pieces of 1 to 2 mm$^3$ with a sterile scalpel. Each of the tissue pieces are to be transferred into a well of a 24 well plate. 1 ml of the culture medium RPMI 1640 with 10 % fetal bovine serum was added into each of the wells. The medium was changed on day 7 and day 14 which means that the culture medium was removed and replaced by fresh culture medium of the same kind. When the tumor cells reached a very high density the culture was transferred to a 6 well plate.

**Example 30 - Determination of the correct time point for feeder cell addition**

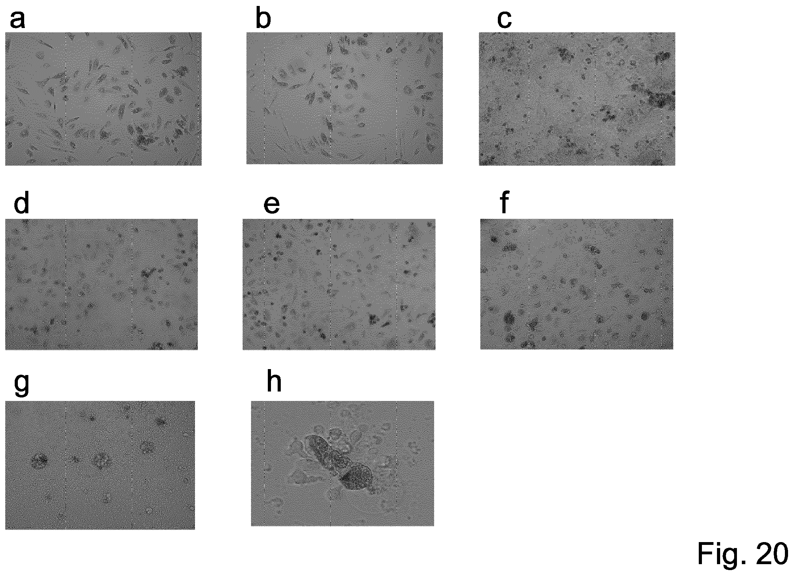

It was found that the addition of feeder cells does not lead to a good expansion of tumor infiltrating lymphocytes if the feeder cells are added when the concentration of lymphocytes is very low. Fig. 20 A shows the lymphocyte culture of the one week of incubation. Some lymphocytes are detectable, however the concentration of lymphocytes is below the basic line of expansion. Fig. 20 B and Fig. 20 C show the same lymphocyte culture after two weeks of incubation. Now the number of lymphocytes that can be detected has increased in this above the basic line of expansion. Feeder cells may be added at this stage. Figs. 1 D, E and E show the culture four days, one week and two weeks subsequently after adding the feeder cells. In the figures can be seen that the number of lymphocytes drastically increases from the image in Fig. D to the image in Fig. F. In Fig. F it can be seen that the lymphocytes are gathering and attacking tumor cells in culture.

**Example 31 - Analysis of phenotype and activation/exhaustion marker expression of lymphocytes expanded from TIL obtained from Glioblastoma.**

Tumor tissue was obtained from 16 Glioblastoma patients. TILs were expanded from the tissue with the cytokine cocktail of IL-2, IL-15 and IL-21 according to the protocol of Example 1.

A)

The expanded cells were analyzed by flow cytometric analysis with regard to their CD4/CD8 phenotype using antibodies directed against CD3, CD4 and CD8.

The results are summarized in Table 10.

Table 10

| | Age | M7F | Diagnosis | Tumor | Cell line CD3+ | CD8+ | CD4+ | CD4- CD8- | CD4+ CD8+ |
|---|---|---|---|---|---|---|---|---|---|
| GBM-A | 69 | M | GBM (grade IV) | Y | 75,50 | 11,40 | 85,20 | 2,92 | 0,47 |
| GBM-B | 66 | M | PXA (grade II-III) | Y | 73,00 | 90,10 | 8,31 | 1,07 | 0,50 |
| GBM-C | 64 | M | GBM (grade IV) | Y | 94,60 | 12,40 | 85,40 | 2,13 | 0,25 |
| GBM-D | 65 | F | GBM (grade IV) | Y | 73,50 | 24,20 | 71,20 | 3,71 | 0,99 |
| GBM-E | 54 | M | GBM (grade IV) | Y | 98,20 | 4,00 | 89,90 | 2,63 | 3,53 |
| GBM-F | 70 | M | GBM (grade IV) | Y | 99,60 | 3,58 | 89,90 | 6,15 | 0,38 |
| GBM-G | 49 | M | GBM (grade IV) | Y | 94,30 | 0,52 | 77,80 | 21,60 | 0,08 |
| GBM-H | 76 | M | GBM (grade IV) | Y | 92,70 | 38,50 | 52,60 | 5,80 | 3,10 |
| GBM-I | 45 | M | GBM (grade IV) | Y | 98,50 | 44,20 | 2,81 | 52,90 | 0,08 |
| GBM-J | 73 | F | GBM (grade IV) | Y | 98,00 | 1,16 | 91,70 | 6,34 | 0,84 |
| GBM-K | 67 | M | GBM (grade IV) | Y | 85,20 | 33,30 | 49,40 | 10,80 | 6,50 |
| GBM-L | 40 | F | GBM (grade III) | Y | 94,20 | 81,30 | 12,20 | 6,02 | 0,46 |
| GBM-M | 79 | M | GBM (grade IV) | Y | 99,90 | 0,02 | 92,50 | 7,39 | 0,10 |
| GBM-N | 50 | F | O (grade II) | Y | 99,70 | 95,40 | 0,24 | 4,33 | 0,07 |
| GBM-O | 17 | M | AE (grade III) | Y | 82,50 | 0,59 | 84,70 | 14,60 | 0,12 |
| GBM-P | 42 | F | Relapse Necrosis | Y | 99,40 | 1,37 | 80,70 | 17,80 | 0,10 |
| N=16 | 58 (17-79) | 11M/4F | | Y 100% | | | | | |

The data show that TIL could be expanded from each individual tumor mainly contain CD3+ T-cells. Depending on the patient either one of CD4+, CD8+ and DN T-cell are in the majority. However, CD4+ was found most frequently in majority. The table also shows that the cytokine cocktail is able to expand T-cells from different CNS tumor histologies. (see column 4 "Diagnosis")

B)

The expanded cells were analyzed by flow cytometric analysis with regard to their specific phenotype - precursor (CD45RA+CCR7+), central memory (CD45RA-CCR7+), peripheral memory (CD45RA-CCR7-), or differentiated effector (CD45RA+CCR7-) T-cells and the expression of activation/exhaustion markers in the base phenotypes CD8+, CD4+ and double negative T-cells.

The antibodies used for flow cytometric analysis of TIL populations are summarized in Table 11.

Table 11

| Immuno-phenocyte | Color | Clone | Intracellular Cytokine Staining | Color | Clone |
|---|---|---|---|---|---|
| CD3 | PerCp | SK7 | CD3 | ECD | UCHT1 |
| CD4 | krome Orange | 13B8.2 | CD4 | PerCp Cy5.5 | L200 |
| CD8a | APC Cy7 | SK1 | CD8a | APC Cy7 | SK1 |
| CD8b | FiTC | 2ST8.5H7 | TNF-$\alpha$ | APC | MAb11 |
| CD107a | Alexa Fluor 700 | H4A3 | IFN-$\gamma$ | PE Cy7 | B27 |
| CD45RA | ECD | 2H4 | IL-2 | PE | MQ1-17H12 |
| c-kit | PE Cy7 | 104D2 | | | |
| CD127 | APC | R34.34 | **V$\beta$ Repertoire** | **Color** | **Clone** |
| CCR7 | Brilliant violet 421 | **G043H7** | CD3 | PE Cy7 | SK7 |
| | | | CD4 | krome Orange | 13B8.2 |
| **Exhaustion and Activation** | **Color** | **Clone** | CD8a | APC Cy7 | SK1 |
| CD3 | Brilliant violet 570 | UCHT1 | TCR V$\beta$ Repertoire Kit | | |
| CD4 | Brilliant violet 510 | OKT4 | | | |
| CD8a | APC Cy7 | SK1 | **Treg** | **Color** | **Clone** |
| 4-1BB | FITC | 4B4-1 | CD3 | ECD | UCHT1 |
| CD127 | APC AF700 | R34.34 | CD4 | V450 | RPA-T4 |
| CD45RA | ECD | 2H4 | CD8a | APC Cy7 | SK1 |
| CCR7 | Brilliant violet 421 | G043H7 | CD25 | PE Cy7 | 2A3 |
| LAG-3 | APC | polyclonal | CD127 | APC | R34.34 |
| CD25 | PE Cy7 | 2A3 | CD73 | efluor 710 | AD2 |
| CTLA-4 | PE Cy5 | BNI3 | CD39 | PE | TU66 |
| TIM3 | PercP eFluor 710 | F38-2E2 | FoXP3 | Alexafluor 488 | 259D |
| PD-1 | PE | EH12.1 | | | |

The results are summarized in Fig. 22 A and B. The majority of CD8+, CD4+ or DN T-cells are in the central memory T-cell subset that has been shown to be crucial for effective anti-cancer responses and long-term immune memory. The results shows in average a strong expansion of DN-T-Cells. This subset is highly activated and bears affinity T-cell receptors. Also a strong expansion of CD45RA+CCR7+ precursor T-cell subsets is observed that provide long-term memory T-cell responses advantageous for long-term immune surveillance.

The reported expression of 4-1BB, LAG-3 or TIM-3 in the T-cells (see Fig 3 B) are indicative for antigen/tumor specific T-cells. The example shows that the cytokine cocktail expands TIL that reside primarily in the memory (the central memory) T-cell subset which has been associated with beneficial clinical responses, it also shows that TIL are expanded that bear markers associated with antigen-specificity.

**Example 32 - Analysis of phenotype and activation/exhaustion marker expression of lymphocytes expanded from TIL obtained from pancreas cancer**

Tumor tissue was obtained from 17 pancreas cancer patients. Table 12 summarizes the patient's age, sex, type of sample and histology.

Table 12 - The cytokine cocktail expands TIL from different pancreas cancer histologies.

| Patients ID | Age | Sex | Sample | Histology |
|---|---|---|---|---|
| Panc 1 | 72 | M | Biopsy | papillary adenocarcinoma |
| Panc 2 | 66 | M | Tumor | ductal adenocarcinoma |
| Panc 3 | 68 | M | Tumor | duodenal adenocarcinoma |
| Panc 4 | 67 | M | Tumor | ductal adenocarcinoma |
| Panc 5 | 81 | F | Tumor | ductal adenocarcinoma |
| Panc 6 | 50 | M | Biopsy | ductal adenocarcinoma |
| Panc 7 | 68 | M | Biopsy | ductal adenocarcinoma |
| Panc 8 | 74 | F | Tumor | ductal adenocarcinoma |
| Panc 9 | 71 | M | Tumor | ductal adenocarcinoma |
| Panc 10 | 60 | F | Tumor | adenocarcinoma |
| Panc 11 | 42 | F | Tumor | ductal adenocarcinoma |
| Panc 12 | 70 | M | Tumor | ductal adenocarcinoma |
| Panc 13 | 59 | F | Tumor | Pancreatic adenosquamous carcinoma |
| Panc 14 | 60 | F | Tumor | ductal adenocarcinoma |
| Panc 15 | 72 | M | Tumor | ductal adenocarcinoma |
| Panc 16 | 81 | F | Tumor | ductal adenocarcinoma |
| Panc 17 | 61 | M | Biopsy | ductal adenocarcinoma |

TILs were expanded from the tissue with the cytokine cocktail of IL-2, IL-15 and IL-21 according to the protocol of Example 28. CD4/CD8 phenotype using antibodies directed against CD3, CD4 and CD8.

A)

The expanded cells were analyzed by flow cytometric analysis with regard to their CD4/CD8 phenotype using antibodies directed against CD3, CD4 and CD8. The results are summarized in Table 13.

Table 13 T-cell phenotype analysis in pancreas cancer TIL. Dominant CD8+ TIL populations.

| Patients ID | CD3+ | CD4+ | CD8+ | CD4+ CD8+ | CD4- CD8- |
|---|---|---|---|---|---|
| Panc 1 | 99,9 | 4,33 | 89,3 | 2,33 | 4 |
| Panc 2 | 99,9 | 56,1 | 29,8 | 2,76 | 11,3 |
| Panc 3 | 99,8 | 17,2 | 71,4 | 1,21 | 10,1 |
| Panc 4 | 99,6 | 50,5 | 32,1 | 2,3 | 15,1 |
| Panc 5 | 99,5 | 99 | 0,073 | 0,51 | 0,39 |
| Panc 6 | 99,7 | 41,1 | 51,3 | 3,09 | 4,47 |
| Panc 7 | 99,7 | 6,57 | 89,9 | 2,62 | 0,93 |
| Panc 8 | 99,7 | 32,8 | 64,9 | 1,09 | 1,24 |
| Panc 9 | 94,8 | 2,07 | 97,2 | 0,3 | 0,47 |

(continued)

| Patients ID | CD3+ | CD4+ | CD8+ | CD4+ CD8+ | CD4- CD8- |
|---|---|---|---|---|---|
| Panc 10 | 98,6 | 92,2 | 5,75 | 0,6 | 1,47 |
| Panc 11 | 99,7 | 2,24 | 96,7 | 0,3 | 0,76 |
| Panc 12 | 99,2 | 71,8 | 22,9 | 4,12 | 1,17 |
| Panc 13 | 91,7 | 70,9 | 24 | 3,01 | 2,06 |
| Panc 14 | 99,9 | 0,66 | 95,7 | 0,13 | 3,55 |
| Panc 15 | 99,1 | 75,9 | 19,5 | 3,09 | 1,47 |
| Panc 16 | 99,3 | 34,1 | 54,4 | 8,6 | 2,86 |
| Panc 17 | 91,8 | 17,3 | 65,1 | 2,92 | 14,6 |
| Mean | 99,6 | 34,1 | 54,4 | 2,33 | 2,06 |

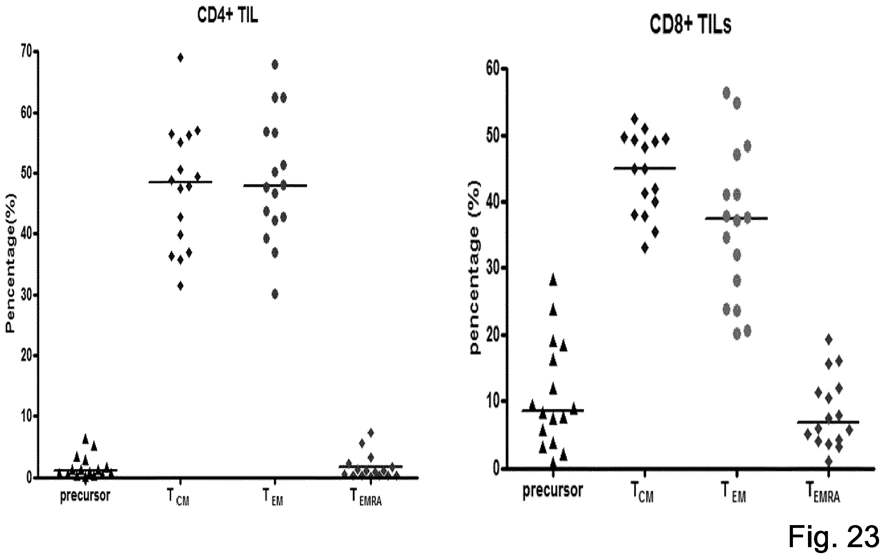

The expanded cells were analyzed by flow cytometric analysis with regard to their specific phenotype - precursor (CD45RA+CCR7+), central memory (CD45RA-CCR7+), peripheral memory (CD45RA-CCR7-), or differentiated effector (CD45RA+CCR7-) T-cells in the base phenotypes CD8+ and CD4+. The results are summarized in Fig. 23 and 24.

The majority of CD8+, CD4+ or DN T-cells are in the central memory T-cell subset that has been shown to be crucial for effective anti-cancer responses and long-term immune memory. The results shows in average a strong expansion of DN-T-Cells (data not shown). This subset is highly activated and bears affinity T-cell receptors. Also a strong expansion of CD45RA+CCR7+ precursor T-cell subsets is observed that provide long-term memory T-cell responses advantageous for long-term immune surveillance.

The reported expression of 4-1BB, LAG-3 or TIM-3 in the T-cells (see Fig. 24) are indicative for antigen/tumor specific T-cells. The example shows that the cytokine cocktail expands TIL that reside primarily in the memory (the central memory) T-cell subset which has been associated with beneficial clinical responses, it also shows that TIL are expanded that bear markers associated with antigen-specificity.

**Example 33 - Analysis of the TCR length of T-cells expanded from tumor tissue of patients with pancreatic cancer**

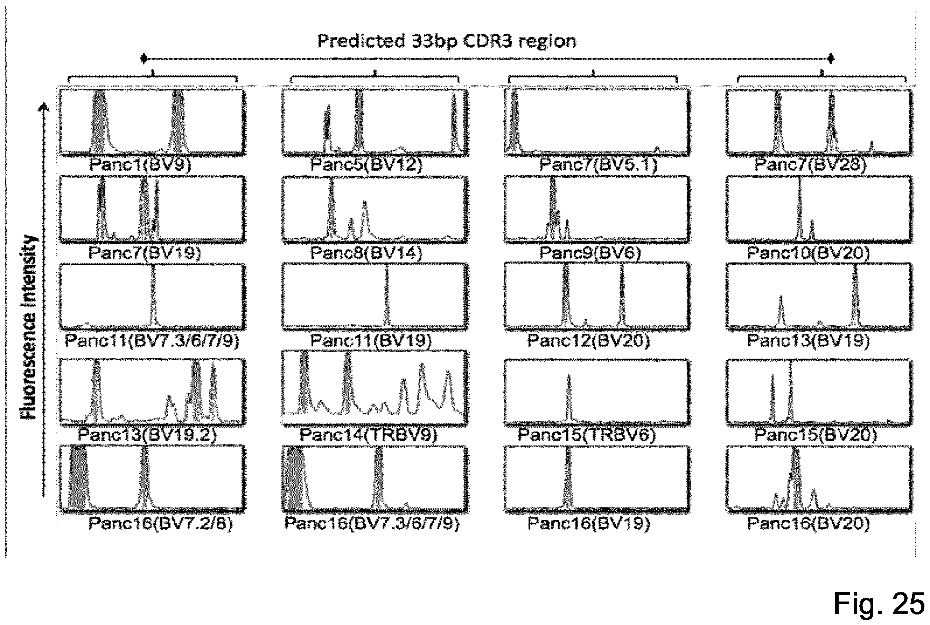

Tumor tissue was obtained from 17 pancreas cancer patients. TILs were expanded from the tissue with the cytokine cocktail of IL-2, IL-15 and IL-21 according to the protocol of Example 28. Using a PCR-based approach the TCR length was measured. The 'normal' image of a TCR family is a Gauss-distribution of the length of the T-cell receptor. Single peaks suggest monoclonality in individual TCR families. The data presented in Fig. 25 shows that the TIL composition is monoclonal or polyclonal, suggestive to be focused against autologous tumor targets, the cytokine cocktail aids to expand a focused TCR repertoire.

**Example 34 - Analysis of cytokine production in lymphocytes expanded from TIL obtained from patients with glioblastoma**

Tumor tissue was obtained from glioblastoma patients. TILs were expanded from the tumor tissue with the cytokine cocktail of IL-2, IL-15 and IL-21 according to the protocol of Example 1. The expanded lymphocytes were stimulated with peptides derived from tumor - associated antigens, i.e. the EGRvrIII, NY-ESO-1 or survivin and the percentage of cells producing either one of the cytokines IFN$\gamma$ and TNF$\alpha$ was measured.

The results are shown in Fig 26 B. For comparison maximal stimulation is tested with PMA/inonomycin as a positive control and the background signal is determined with media only (negative control). The results demonstrate that the expanded T-cells recognize these commonly shared tumor antigens. Accordingly, the cytokine cocktail expands T-cells from glioblastoma that are reactive to tumor-antigens that have been described to be clinically relevant and associated with clinical responses.

**Example 35 - Analysis of cytokine production in lymphocytes expanded from TIL obtained from patients with pancreas cancer**

Tumor tissue was obtained from pancreas cancer patients.

TILs were expanded from the tumor tissue with the cytokine cocktail of IL-2, IL-15 and IL-21 according to the protocol of Example 27. The expanded lymphocytes were stimulated with peptides derived from tumor-associated antigens, i.e. the EGRvrIII, NY-ESO-1 or survivin and the percentage of cells producing either one of the cytokines IFNγ and TNFα was measured. The results are shown in Fig 27 A. Fig. 27 B shows images of the flow cytometry analysis for NY-ESO-1. The results confirm that the expanded T-cells recognize these commonly shared tumor antigens. Accordingly, the cytokine cocktail expands T-cells from pancreas tumor that are reactive to tumor-antigens that have been described to be clinically relevant and associated with clinical responses.

**Example 36 - Analysis of cytokine production in lymphocytes expanded from TIL obtained from patients with glioblastoma and autologous stimulation**

Tumor tissue was obtained from glioblastoma patients. TILs were expanded from the tumor tissue with the cytokine cocktail of IL-2, IL-15 and IL-21 according to the protocol of Example 27. The expanded lymphocytes were stimulated with autologous tumor cells. The results are shown in Fig 28 A. Fig. 28 B shows images of the flow cytometry analysis. The results confirm that the expanded T-cells recognize autologous tumor cells. Accordingly, the cytokine cocktail expands T-cells from glioblastoma that are reactive to autologous tumor cells, accordingly the patient's own mutations.

**Example 37: Link of TCR usage in pancreatic cancer with recognition of autologous tumor cells**

Tumor tissue was obtained from glioblastoma patients. TILs were expanded from the tumor tissue with the cytokine cocktail of IL-2, IL-15 and IL-21 according to the protocol of Example 27. The expanded lymphocytes were stimulated with autologous tumor cells. TIL were first gated on CD3+ T-cells, then on CD4+ and CD8+ T-cells. The frequency of individual Vβ families were tested using a panel of TCR VB antibodies. This TCR panel covers about 75% of the human TCR repertoire, there is therefore a possibility that not all TCR Vβ families are captured sufficiently. The TCR Vβ family distribution is about 2 to 6 % in each family, except for TCR VB 2 that can reach over 10%. Table 14 shows the preferential expansion of the Vβ-2 family in TIL from patients with glioblastoma.

| Patient | | Vb1 | Vb2 | Vb3 | Vb4 | Vb5.1 | Vb5.2 | Vb5.3 | Vb7.1 | Vb7.2 | Vb8 | Vb9 | Vb11 | Vb12 | Vb13.1 | Vb13.2 | Vb13.6 | Vb14 | Vb16 | Vb17 | Vb18 | Vb20 | Vb21.3 | Vb22 | Vb23 | Total |
|---|---|---|---|---|---|---|---|---|---|---|---|---|---|---|---|---|---|---|---|---|---|---|---|---|---|---|
| GBM-A | CD4+ | 0.24 | 34.30 | 3.54 | 0.14 | 0.34 | 1.63 | 0.07 | 1.91 | 3.73 | 2.21 | 0.90 | 0.25 | 2.37 | 2.14 | 0.88 | 0.35 | 2.90 | 0.35 | 0.08 | 0.08 | 0.45 | 0.41 | 0.30 | 1.39 | 104.73 |
| GBM-A | CD8+ | 0.74 | 0.11 | 0.36 | 0.03 | 0.18 | 0.10 | 0.23 | 0.11 | 1.53 | 1.67 | 0.56 | 0.12 | 28.40 | 0.03 | 0.19 | 0.08 | 0.70 | 0.03 | 0.62 | 0.08 | 16.70 | 41.50 | 0.06 | 0.07 | 84.46 |
| GBM-B | CD4+ | 2.45 | 17.60 | 2.97 | 0.27 | 1.72 | 0.65 | 1.01 | 0.86 | 1.53 | 6.90 | 0.56 | 0.12 | 4.18 | 2.60 | 3.25 | 1.64 | 3.63 | 3.84 | 1.35 | 0.62 | 2.68 | 2.47 | 6.50 | 0.91 | 72.71 |
| GBM-B | CD8+ | 0.74 | 2.52 | 9.29 | 0.04 | 0.76 | 0.33 | 0.82 | 0.38 | 1.67 | 3.25 | 0.53 | 2.17 | 2.34 | 1.12 | 2.02 | 0.15 | 1.97 | 0.28 | 0.10 | 0.03 | 2.92 | 3.71 | 0.53 | 0.79 | 40.70 |
| GBM-C | CD4+ | 11.00 | 3.69 | 10.30 | 0.01 | 0.11 | 0.87 | 1.48 | 4.99 | 2.00 | 1.23 | 0.14 | 0.95 | 2.20 | 1.14 | 5.27 | 1.06 | 1.84 | 4.28 | 4.71 | 0.21 | 4.39 | 8.16 | 1.63 | 1.20 | 72.46 |
| GBM-C | CD8+ | 1.49 | 7.92 | 6.83 | 0.04 | 0.76 | 0.65 | 0.42 | 2.00 | 0.86 | 4.89 | 2.74 | 2.64 | 4.44 | 5.27 | 1.12 | 2.02 | 6.75 | 4.28 | 3.61 | 0.10 | 8.54 | 0.96 | 1.63 | 2.56 | 72.16 |
| GBM-D | CD4+ | 0.19 | 33.30 | 1.33 | 0.05 | 0.14 | 0.23 | 0.03 | 0.07 | 0.35 | 2.34 | 0.28 | 0.43 | 3.67 | 4.47 | 0.88 | 0.56 | 2.00 | 0.56 | 0.39 | 0.08 | 3.67 | 4.18 | 1.33 | 0.10 | 77.80 |
| GBM-D | CD8+ | 0.12 | 1.67 | 1.00 | 0.07 | 0.11 | 2.23 | 0.08 | 1.80 | 0.32 | 1.23 | 0.14 | 2.08 | 2.20 | 1.14 | 2.95 | 0.74 | 6.03 | 5.27 | 17.70 | 0.04 | 2.88 | 6.71 | 1.63 | 2.56 | 74.48 |
| GBM-E | CD4+ | 4.06 | 13.60 | 9.81 | 0.07 | 9.98 | 0.40 | 0.64 | 1.28 | 6.80 | 4.18 | 0.18 | 0.85 | 12.10 | 0.41 | 0.59 | 3.01 | 3.87 | 2.27 | 0.83 | 0.80 | 2.68 | 2.47 | 6.50 | 0.91 | 62.65 |
| GBM-E | CD8+ | 0.25 | 2.48 | 2.11 | 0.03 | 0.09 | 0.16 | 0.08 | 1.80 | 1.53 | 3.25 | 5.66 | 0.05 | 1.04 | 0.06 | 0.10 | 1.98 | 1.70 | 0.27 | 0.80 | 0.03 | 2.47 | 45.90 | 0.02 | 0.02 | 84.99 |
| GBM-F | CD4+ | 10.30 | 0.00 | 0.00 | 0.00 | 0.00 | 0.00 | 0.00 | 0.00 | 0.00 | 0.00 | 2.86 | 0.00 | 0.00 | 85.10 | 0.00 | 0.00 | 0.00 | 0.00 | 42.90 | 0.00 | 0.00 | 0.00 | 0.12 | 0.00 | 27.71 |
| GBM-F | CD8+ | 0.09 | 0.54 | 0.00 | 0.00 | 0.01 | 0.00 | 0.01 | 0.03 | 0.00 | 0.03 | 0.04 | 0.01 | 0.02 | 0.00 | 0.10 | 0.37 | 0.01 | 0.00 | 0.00 | 0.01 | 8.48 | 3.45 | 0.01 | 0.00 | 65.76 |
| GBM-G | CD4+ | 0.02 | 0.00 | 0.00 | 0.00 | 0.01 | 0.00 | 0.00 | 0.00 | 0.00 | 4.77 | 0.39 | 0.19 | 0.14 | 1.08 | 0.08 | 0.32 | 1.68 | 0.21 | 10.20 | 0.00 | 3.62 | 0.13 | 0.46 | 0.12 | 51.37 |
| GBM-G | CD8+ | 0.00 | 0.00 | 0.00 | 0.00 | 0.00 | 0.00 | 0.00 | 0.00 | 0.00 | 0.00 | 2.86 | 0.00 | 0.00 | 0.00 | 3.57 | 0.00 | 0.00 | 0.00 | 42.90 | 0.00 | 2.04 | 0.00 | 0.00 | 0.00 | 94.91 |
| GBM-H | CD4+ | 0.19 | 1.21 | 0.92 | 0.16 | 0.24 | 0.71 | 0.08 | 0.64 | 0.00 | 40.40 | 0.11 | 0.07 | 0.00 | 0.08 | 0.08 | 0.08 | 45.30 | 0.05 | 0.00 | 0.00 | 0.06 | 0.50 | 9.96 | 0.19 | 32.84 |
| GBM-H | CD8+ | 0.06 | 0.00 | 0.00 | 0.09 | 0.00 | 0.25 | 0.00 | 0.00 | 0.00 | 0.00 | 0.07 | 0.07 | 0.00 | 0.27 | 0.00 | 0.00 | 0.00 | 0.01 | 0.00 | 0.02 | 45.90 | 0.01 | 0.00 | 0.00 | 97.47 |
| GBM-I | CD4+ | 0.01 | 99.60 | 0.00 | 0.08 | 0.00 | 0.04 | 0.00 | 0.01 | 0.00 | 0.01 | 0.00 | 0.00 | 0.03 | 0.00 | 0.01 | 0.12 | 0.00 | 0.01 | 0.27 | 0.03 | 0.08 | 0.01 | 0.01 | 0.02 | 100.04 |
| GBM-I | CD8+ | 0.00 | 0.00 | 0.00 | 0.00 | 97.00 | 0.00 | 0.05 | 0.08 | 0.09 | 0.65 | 0.27 | 0.00 | 0.00 | 0.00 | 0.01 | 0.05 | 0.00 | 0.00 | 0.00 | 0.00 | 0.43 | 0.00 | 0.01 | 0.00 | 98.35 |
| GBM-J | CD4+ | 0.02 | 0.06 | 0.00 | 0.00 | 0.01 | 0.32 | 0.00 | 0.00 | 0.01 | 0.00 | 0.00 | 0.01 | 0.00 | 0.03 | 0.00 | 0.00 | 0.00 | 0.02 | 0.00 | 0.00 | 1.01 | 98.40 | 0.10 | 0.00 | 100.01 |
| GBM-J | CD8+ | 0.00 | 0.00 | 0.00 | 0.00 | 0.01 | 0.00 | 0.00 | 0.00 | 0.00 | 0.65 | 0.00 | 0.00 | 0.00 | 0.00 | 0.00 | 0.00 | 0.00 | 0.00 | 0.00 | 0.00 | 0.43 | 0.00 | 0.00 | 0.00 | 5.76 |
| GBM-K | CD8+* | 0.00 | 0.03 | 0.12 | 0.00 | 0.37 | 0.06 | 0.00 | 0.00 | 0.04 | 2.65 | 0.00 | 0.04 | 0.53 | 0.06 | 0.38 | 0.15 | 0.77 | 0.06 | 0.03 | 0.09 | 0.46 | 0.04 | 0.21 | 0.20 | 5.76 |
| GBM-K | CD4+ | 0.58 | 0.25 | 0.81 | 0.11 | 0.39 | 0.43 | 0.09 | 0.21 | 0.97 | 3.02 | 0.02 | 0.29 | 0.53 | 0.09 | 6.41 | 0.16 | 13.70 | 1.59 | 0.06 | 0.34 | 1.10 | 5.06 | 1.01 | 1.39 | 38.62 |
| GBM-L | CD4+ | 1.72 | 12.20 | 4.46 | 0.47 | 0.24 | 0.71 | 0.08 | 0.64 | 2.71 | 2.19 | 0.39 | 0.19 | 2.60 | 1.08 | 2.71 | 0.32 | 1.68 | 2.91 | 3.17 | 0.01 | 4.84 | 1.15 | 2.86 | 0.91 | 106.44 |
| GBM-L | CD8+ | 1.78 | 0.90 | 0.38 | 0.08 | 1.67 | 0.61 | 4.48 | 1.51 | 6.92 | 2.35 | 0.72 | 0.69 | 0.34 | 0.62 | 6.70 | 0.08 | 1.80 | 4.17 | 1.94 | 7.08 | 1.32 | 36.40 | 1.09 | 0.19 | 93.91 |
| GBM-M | CD4+ | 0.47 | 1.39 | 19.30 | 0.15 | 0.08 | 0.15 | 0.22 | 0.65 | 0.05 | 3.41 | 1.70 | 0.19 | 1.93 | 11.10 | 19.90 | 1.08 | 1.91 | 0.97 | 0.83 | 0.01 | 1.32 | 23.30 | 0.30 | 0.36 | 93.42 |
| GBM-M | CD8+ | 1.72 | 3.72 | 3.70 | 0.98 | 0.00 | 0.53 | 0.85 | 1.99 | 2.93 | 23.10 | 3.55 | 0.43 | 6.91 | 1.54 | 11.10 | 3.08 | 18.70 | 5.08 | 1.52 | 0.00 | 23.30 | 5.06 | 0.30 | 0.86 | 66.47 |
| GBM-N | CD4+ | 0.27 | 40.90 | 1.33 | 0.03 | 0.33 | 1.28 | 0.05 | 90.70 | 0.00 | 1.02 | 7.62 | 0.10 | 1.81 | 0.18 | 0.87 | 0.13 | 0.13 | 0.16 | 0.40 | 0.03 | 0.36 | 0.08 | 0.04 | 0.40 | 106.44 |
| GBM-N | CD8+ | 1.37 | 2.38 | 1.34 | 0.01 | 0.16 | 0.27 | 0.06 | 0.05 | 0.95 | 0.31 | 0.13 | 0.00 | 0.51 | 3.15 | 0.18 | 0.15 | 2.48 | 0.23 | 0.06 | 0.12 | 0.12 | 0.20 | 0.22 | 0.83 | 15.57 |
| GBM-O | CD4+ | 0.28 | 0.04 | 0.02 | 0.01 | 0.45 | 0.10 | 0.02 | 0.07 | 0.00 | 0.07 | 0.07 | 0.10 | 0.04 | 0.05 | 0.02 | 0.27 | 0.01 | 0.04 | 0.06 | 0.05 | 0.01 | 0.06 | 0.11 | 0.58 | 93.42 |
| GBM-O | CD4+ | 3.70 | 2.30 | 3.09 | 0.08 | 0.26 | 0.80 | 0.13 | 0.25 | 1.39 | 0.22 | 2.97 | 0.30 | 0.13 | 11.60 | 2.44 | 2.56 | 2.84 | 1.92 | 21.80 | 0.26 | 1.15 | 1.06 | 0.40 | 5.83 | 67.76 |

## Table 14

The data of Table 14 shows that individual TCR VB are preferentially expanded in TIL. It is noted that clonality can only be addressed by sequencing. It is noted that the cytokine cocktail expands different TCR Vβ families in individual patients. This is true for patients with glioblastoma, as well as for patients with patients with pancreatic cancer. Note also that some TIL are composed for a single or two VB families showing a highly focused TCR VB expansion. suggestive of an antigen-driven T-cell expansion process. Some TIL that we have shown to be preferentially expanded were shown to be monoclonal. Thus, the cytokine cocktail of IL-2, IL-15 and IL-21 expands a focused T-cell response, that is directed against the patient's own tumor cells.

**Example 38 - Link of TCR usage in pancreatic cancer with recognition of autologous tumor cells**

Tumor tissue was obtained from pancreatic cancer patients. TILs were expanded from the tumor tissue with the cytokine cocktail of IL-2, IL-15 and IL-21 according to the protocol of Example 28.

Cells were stained by flow cytometry using CD3, CD4 and CD8 in combination with a TCR Vβ antibody. The panel that covers up to 75 % of the entire TCR VB repertoire. If certain T-cell families reside in the 25% that are not covered by this panel, they are not captured in this panel. The results are summarized in Table 15.

|  | Panc 1 | | Panc 2 | | Panc 3 | | Panc 4 | | Panc 5 | | Panc 6 | |
|---|---|---|---|---|---|---|---|---|---|---|---|---|
|  | CD4 | CD8 | CD4 | CD8 | CD4 | CD8 | CD4 | CD8 | CD4 | CD8 | CD4 | CD8 |
| Vβ1 | 2,8 | 77,1 | 3,2 | 2,3 | 3,4 | 0,8 | 0,2 | 2,1 | 1,5 | 0,2 | 0,7 | 1,1 |
| Vβ2 | 8,0 | 0,6 | 9,8 | 2,6 | 4,8 | 0,2 | 8,5 | 2,0 | 0,0 | 0,0 | 9,6 | 19,9 |
| Vβ3 | 2,1 | 0,2 | 4,8 | 1,9 | 26,7 | 0,5 | 4,8 | 8,3 | 0,0 | 6,1 | 4,2 | 0,3 |
| Vβ4 | 0,4 | 0,0 | 0,0 | 0,0 | 0,1 | 0,0 | 0,0 | 0,0 | 0,0 | 0,0 | 0,0 | 0,0 |
| Vβ5.1 | 0,4 | 0,4 | 0,7 | 0,5 | 0,3 | 0,0 | 0,1 | 0,0 | 0,0 | 0,3 | 9,0 | 0,3 |
| Vβ5.2 | 0,2 | 0,1 | 1,5 | 0,0 | 1,5 | 0,0 | 0,1 | 0,2 | 0,2 | 0,0 | 0,2 | 13,7 |
| Vβ5.3 | 0,1 | 0,1 | 1,6 | 0,4 | 1,9 | 0,0 | 0,0 | 0,0 | 0,0 | 0,0 | 0,3 | 0,1 |
| Vβ7.1 | 0,2 | 0,3 | 0,7 | 2,1 | 0,9 | 3,4 | 0,2 | 0,1 | 0,1 | 0,0 | 0,1 | 0,1 |

| | | | | | | | | | | | |
|---|---|---|---|---|---|---|---|---|---|---|---|
| Vβ7.2 | 6,6 | 0,0 | 0,3 | 0,1 | 0,9 | 1,7 | 0,2 | 0,1 | 0,0 | 0,0 | 1,1 | 0,1 |
| Vβ8 | 1,9 | 1,7 | 3,7 | 0,6 | 2,9 | 0,1 | 0,4 | 0,4 | **42,7** | 4,1 | 1,2 | 0,3 |
| Vβ9 | 1,6 | 0,0 | 0,8 | 0,3 | 0,3 | 0,1 | 0,1 | 0,1 | **40,5** | 0,1 | 1,7 | 0,0 |
| Vβ11 | 0,5 | 0,0 | 0,5 | 0,2 | 1,5 | 0,0 | 0,1 | 0,7 | 0,3 | 0,0 | 0,6 | 2,3 |
| Vβ12 | 3,2 | 0,9 | 1,9 | 0,9 | 2,5 | 0,1 | 0,7 | 1,8 | 0,0 | 0,0 | 0,6 | 0,3 |
| Vβ13.1 | 2,4 | 0,3 | 4,6 | 10,4 | 1,5 | 0,5 | 2,6 | 4,2 | 0,0 | 0,1 | 0,8 | 0,0 |
| Vβ13.2 | 3,1 | 0,2 | 1,4 | 0,4 | 1,8 | 0,2 | 0,2 | 0,1 | 0,0 | 0,0 | 0,2 | 0,6 |
| Vβ13.6 | 2,8 | 9,1 | 3,6 | 0,5 | 1,2 | 0,1 | 0,2 | 0,1 | 0,2 | 0,1 | 0,2 | 0,0 |
| Vβ14 | 1,9 | 1,4 | 2,0 | 3,6 | 1,3 | **63,3** | 0,4 | 0,7 | 0,0 | 0,0 | 1,0 | 5,3 |
| Vβ16 | 2,4 | 1,0 | 6,9 | 6,9 | 3,3 | 0,3 | 4,1 | 1,1 | 0,0 | 0,1 | 0,8 | 0,0 |
| Vβ17 | 4,6 | 0,1 | 2,9 | 1,8 | 0,8 | 0,0 | 0,2 | 0,0 | 0,0 | 0,0 | 1,5 | 0,0 |
| Vβ18 | 2,4 | 0,1 | 0,2 | 0,0 | 0,5 | 0,0 | 0,1 | 0,0 | 0,4 | 0,0 | 0,4 | 0,2 |
| Vβ20 | 7,0 | 5,9 | 6,6 | 13,8 | 4,6 | 0,1 | 3,7 | 0,1 | 0,0 | 0,4 | 0,7 | 0,2 |
| Vβ21.3 | 0,4 | 0,5 | 3,8 | 4,3 | 0,4 | 0,1 | 0,1 | 0,1 | 0,0 | 3,2 | 6,2 | 0,1 |
| Vβ22 | 11,1 | 0,2 | 3,3 | 1,7 | 2,8 | 0,2 | 6,2 | 1,8 | 0,1 | 0,0 | 15,1 | 2,1 |
| Vβ23 | 3,7 | 0,3 | 0,4 | 0,1 | 0,8 | 0,8 | 0,9 | 0,5 | 1,6 | 0,5 | 0,1 | 4,6 |

| | | Panc 7 | | Panc 8 | | Panc 9 | | Panc 10 | | Panc 11 | |
|---|---|---|---|---|---|---|---|---|---|---|---|
| | | CD4 | CD8 | CD4 | CD4 | CD8 | CD4 | CD8 | CD8 | CD4 | CD8 |
| Vβ1 | | 0,3 | 0,1 | 1,5 | 4,4 | 7,1 | 13,0 | 0,5 | 1,2 | 0,6 | 0,1 |
| Vβ2 | | 0,1 | 0,7 | 7,4 | 4,1 | **39,8** | 2,4 | 0,2 | 1,7 | 12,0 | 0,0 |
| Vβ3 | | 5,1 | 28,7 | 2,4 | 3,7 | 1,3 | 0,0 | 0,0 | 12,0 | 1,0 | 0,0 |
| Vβ4 | | 0,1 | 0,0 | 0,1 | 6,0 | 0,1 | 0,0 | 0,0 | 0,0 | 3,2 | 0,0 |
| Vβ5.1 | | **41,1** | 6,2 | 9,3 | 10,8 | 1,1 | 0,6 | 0,7 | 0,0 | 3,6 | 0,1 |
| Vβ5.2 | | 2,4 | 0,2 | 0,3 | 1,3 | 9,4 | 0,1 | 0,1 | 0,2 | 0,1 | 0,1 |
| Vβ5.3 | | 0,0 | 0,9 | 0,6 | 0,7 | 18,0 | 0,2 | 0,0 | 0,1 | 0,0 | 0,0 |
| Vβ7.1 | | 0,1 | 0,2 | 5,1 | 0,5 | 1,5 | 0,0 | 0,0 | 0,3 | 0,6 | 0,0 |
| Vβ7.2 | | 1,0 | 0,4 | 5,3 | 1,6 | 0,1 | 0,0 | 0,0 | 3,8 | 13,9 | 0,0 |
| Vβ8 | | 5,4 | 0,5 | 2,3 | 2,7 | 1,4 | 0,4 | 0,3 | 2,2 | 0,5 | 0,1 |
| Vβ9 | | 0,2 | 0,1 | 1,0 | 3,4 | 1,2 | 0,5 | 0,2 | 0,1 | 4,8 | 0,0 |
| Vβ11 | | 4,0 | 1,0 | 0,2 | 0,6 | 0,3 | 0,1 | 0,2 | 0,0 | 1,0 | 0,1 |
| Vβ12 | | 0,0 | 0,0 | 0,1 | 1,6 | 0,5 | 0,0 | 0,0 | 0,1 | 0,4 | 0,0 |
| Vβ13.1 | | 8,1 | 10,0 | 5,6 | 3,7 | 0,1 | **35,5** | 0,1 | 16,5 | 5,0 | 0,0 |
| Vβ13.2 | | 0,3 | 1,1 | 0,3 | 6,2 | 0,7 | 0,2 | 0,0 | 0,0 | 0,5 | **99,2** |
| Vβ13.6 | | 1,7 | 0,1 | 3,2 | 2,0 | 0,3 | 0,6 | 0,0 | 6,9 | 0,4 | 0,0 |
| Vβ14 | | 3,2 | 2,8 | 0,6 | 3,6 | 0,8 | 0,1 | 0,0 | 1,4 | 0,4 | 0,1 |
| Vβ16 | | 0,1 | 0,3 | 0,1 | 1,0 | 0,2 | 0,0 | 0,2 | 25,4 | 0,5 | 0,0 |

| | | | | | | | | | | |
|---|---|---|---|---|---|---|---|---|---|---|
| Vβ17 | | 1,0 | 32,9 | 1,3 | 7,5 | 0,3 | 0,1 | 12,3 | 0,1 | 12,4 | 0,0 |
| Vβ18 | | 1,0 | 0,2 | 3,6 | 0,5 | 0,2 | 0,1 | 0,2 | 0,0 | 0,4 | 0,0 |
| Vβ20 | | 0,1 | 0,0 | 1,4 | 2,5 | 0,5 | 0,1 | 0,1 | 0,4 | 0,1 | 0,0 |
| Vβ21.3 | | 2,4 | 0,0 | 0,6 | 2,4 | 0,1 | 0,9 | 0,0 | 0,3 | 0,5 | 0,0 |
| Vβ22 | | 0,2 | 2,6 | 1,4 | 3,4 | 1,0 | 3,6 | 0,1 | 0,6 | 1,9 | 0,7 |
| Vβ23 | | 0,1 | 0,1 | 0,2 | 4,5 | 0,7 | 0,6 | 0,1 | 6,7 | 0,1 | 0,0 |

| | Panc 12 | | Panc 13 | | Panc 14 | | Panc 15 | | Panc 16 | |
|---|---|---|---|---|---|---|---|---|---|---|
| | CD4 | CD8 | CD4 | CD8 | CD4 | CD8 | CD4 | CD8 | CD4 | CD8 |
| Vβ1 | 1,4 | **25,8** | 0,1 | 0,3 | 0,9 | 16,2 | 0,6 | 2,8 | 0,3 | 0,4 |
| Vβ2 | 7,5 | 4,8 | 1,5 | 0,6 | 5,1 | 0,7 | 29,3 | 1,5 | **48,9** | 1,0 |
| Vβ3 | 1,7 | 8,8 | 0,0 | 0,0 | 13,3 | 2,0 | 1,1 | 17,9 | 11,7 | 0,2 |
| Vβ4 | 2,2 | 0,4 | 4,8 | 0,0 | 0,9 | 0,1 | 8,3 | 1,2 | 0,1 | 0,1 |
| Vβ5.1 | 3,4 | 0,5 | 4,6 | 0,2 | 2,7 | 0,8 | 4,2 | 1,6 | 15,2 | 0,2 |
| Vβ5.2 | 0,3 | 1,8 | 0,1 | 0,1 | 0,4 | 0,1 | 2,3 | 0,8 | 1,2 | 0,1 |
| Vβ5.3 | 0,3 | 1,6 | 0,1 | 0,0 | 0,1 | 0,3 | 4,2 | 0,3 | 0,9 | 0,0 |
| Vβ7.1 | 1,6 | 2,8 | 0,1 | 0,3 | 0,3 | 6,3 | 0,9 | 1,8 | 0,3 | 2,2 |
| Vβ7.2 | 0,4 | 0,1 | 0,0 | 0,0 | 0,7 | 4,8 | 0,4 | 0,1 | 0,0 | 0,0 |
| Vβ8 | 3,6 | 1,8 | 1,1 | 0,5 | 1,7 | 0,8 | 1,9 | 4,1 | 0,5 | 0,3 |
| Vβ9 | 6,7 | 1,0 | 2,4 | 0,1 | 9,7 | 2,3 | 2,0 | 2,0 | 1,8 | 0,4 |
| Vβ11 | 0,8 | 0,3 | 0,2 | 0,2 | 0,3 | 0,2 | 1,4 | 0,7 | 0,4 | 0,2 |
| Vβ12 | 0,3 | 1,0 | 0,3 | 0,0 | 1,5 | 5,9 | 0,3 | 0,2 | 0,3 | 0,1 |
| Vβ13.1 | 9,2 | 0,3 | 0,5 | 0,2 | 4,3 | 19,6 | 0,7 | 0,8 | 2,6 | 1,1 |
| Vβ13.2 | 2,5 | 1,4 | 0,2 | 0,2 | 4,4 | 0,9 | 1,8 | 23,0 | 0,4 | 0,6 |
| Vβ13.6 | 1,5 | 0,2 | 0,5 | 0,2 | 0,3 | 1,9 | 0,6 | 0,9 | 0,3 | 0,4 |
| Vβ14 | 0,7 | 3,5 | 0,1 | 0,3 | 2,9 | 2,5 | 0,9 | 7,2 | 2,1 | 0,6 |
| Vβ16 | 0,1 | 0,1 | 0,0 | 0,2 | 0,2 | 0,8 | 0,2 | 0,2 | 0,1 | 0,0 |
| Vβ17 | 3,3 | 2,3 | **31,1** | 0,9 | 9,9 | 1,4 | 1,3 | 1,9 | 1,5 | 0,5 |
| Vβ18 | 0,3 | 0,3 | 0,5 | 0,1 | 0,9 | 3,1 | 3,3 | 3,6 | 0,4 | 3,3 |
| Vβ20 | 1,4 | 0,2 | 0,0 | 0,0 | 0,7 | 0,3 | 0,5 | 0,6 | 0,0 | 0,8 |
| Vβ21.3 | 0,5 | 8,0 | 0,5 | 0,0 | 1,2 | 0,5 | 0,4 | 0,4 | 0,2 | 1,6 |
| Vβ22 | 5,2 | 2,0 | **26,0** | **65,9** | 3,6 | 0,8 | 0,7 | 2,9 | 6,2 | 6,4 |
| Vβ23 | 0,2 | 0,4 | 0,3 | 1,0 | 0,5 | 0,7 | 1,8 | 7,4 | 0,3 | 0,3 |

## Table 15

IFNγ production is measured after 3 days after exposure of TIL to autologous tumor cells (single cell suspension). High level of IFNγ production is seen after with autologous tumor cells only (see Fig. 35) IFNγ production is complete blocked with the antibody against W6/32 (blocking CD8+ TIL). As a control, the antibody L243 blocking CD4+ TIL is not able to block reactivity, the reactive T-cells in expanded cell lymphocytes are all CD8+. The data show that the cytokine cocktail expands TIL that are very focused and specifically recognized autologous tumor cells from patients with pancreatic cancer. Monoclonal TCR families in TIL from patients with pancreatic cancer.: IL-2, IL-15 and II-21 expanded preferentially individual TCR VB families, such a preferential expansion of T-cell families is associated with antigen-specificy and focused anti-tumor reactivity. Some of these preferentially expanded VB families contain monoclonal T-cells, defined by a single TCR VB chain. Such monoclonal TCR expansion is indicative a focused, anti-cancer response. The functional response analyses showed that monoclonally expanded TIL recognize autologous tumor cells

**Example 39 - Analysis of the cytolytic response of expanded TIL from patients with glioblastoma against autologous tumor cells**

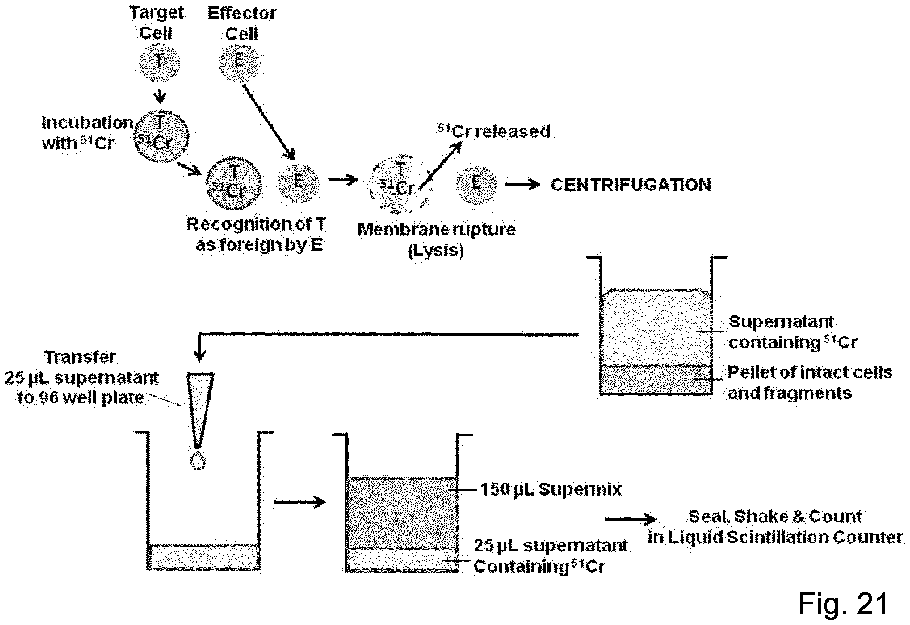

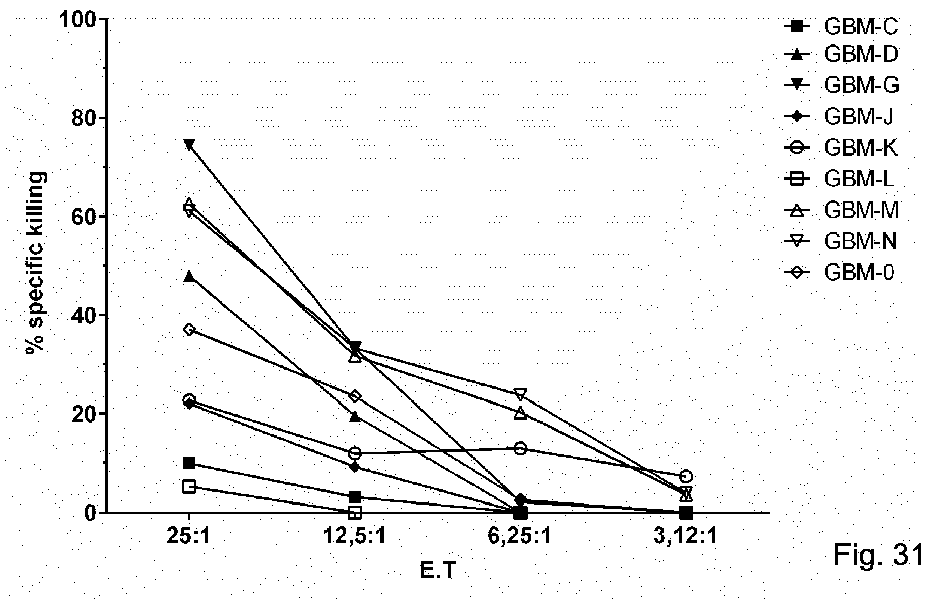

Tumor tissue was obtained from glioblastoma patients. TILs were expanded from the tumor tissue with the cytokine cocktail of IL-2, IL-15 and IL-21 according to the protocol of Example 28. Autologous tumor cells were generated according to the protocol in Example 29. Autologous tumor cells are labeled with radioactivity and cultured with expanded TIL for 4 h. Killing of tumor cells leads to release of radioactivity which is then measured. The principle of the method is shown in Fig. 21. The results are shown in Fig. 31 confirming a cytotoxic effect dependent on the ratio of TILs to tumor cells. The data show that the cytokine cocktail expands TIL that are strongly cytoxic against autologous tumor cells.

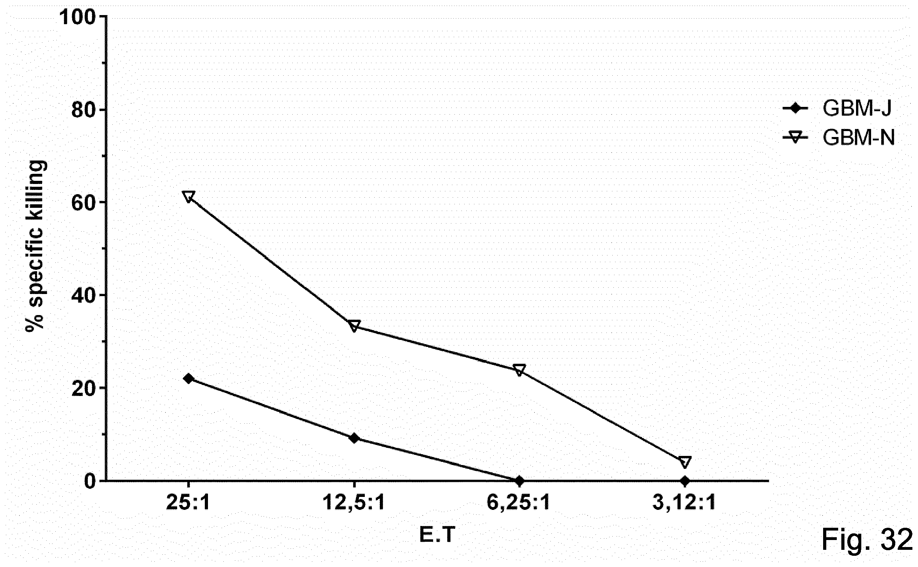

Alternatively, expanded monoclonal T-cells and/or preferentially expanded TIL were tested for their cytotoxicity with the same test. The results are shown in Fig. 32. Table 16 shows that these immune responses are specifically directed against the autologous tumor cells. The data shows that the cytokine cocktail expands TIL with a specific reactivity, including cytotoxic responses, against autologous tumor.

B)

In a parallel experiment the autologous tumor cells were incubated for three days with the TIL expanded from glioblastoma with the cytokine cocktail of IL-15, IL-21 and IL-2. After incubation IFN$\gamma$ production (pg/mL) was measured by ELISA. In order to identify whether CD4+ or CD8+ cells are responsible for the tumor responses antibodies blocking either MHC class I antigen responses (W6/32) affecting CD8+ T-cells, or blocking HLA-DR (MHC class II responses) affecting CD4+ T-cells were used. The results are summarized in Table 16.

Table 16

| Diagnosis | Label | autologous Tumor | autologous Tumor+W6/32 | autologous Tumor+L243 |
|---|---|---|---|---|
| GBM (grade IV) | GBM-A | 15,40 | 0,00 | 0,00 |
| PXA (grade II-III) | GBM-B | 251,19 | 109,75 | 0,00 |
| GBM (grade IV) | GBM-C | 13,32 | 30,94 | 0,00 |
| GBM (grade IV) | GBM-D | 1123,76 | 752,54 | 65,65 |
| GBM (grade IV) | GBM-E | 424,86 | 114,93 | 23,28 |
| GBM (grade IV) | GBM-F | 678,36 | 251,12 | 0,00 |
| GBM (grade IV) | GBM-G | 0,00 | 0,00 | 0,00 |
| GBM (grade IV) | GBM-H | 141,89 | 131,31 | 51,47 |
| GBM (grade IV) | GBM-I | 971,12 | 356,22 | 176,89 |
| GBM (grade IV) | GBM-J | 100,75 | 89,32 | 19,56 |
| GBM (grade IV) | GBM-K | 89,47 | 0,00 | 0,00 |
| AO (grade III) | GBM-L | 0,00 | 0,00 | 0,00 |
| GBM (grade IV) | GBM-M | 0,00 | 0,00 | 0,00 |
| O (grade II) | GBM-N | 0,00 | 0,00 | 0,00 |
| AE (grade III) | GBM-O | 0,00 | 0,00 | 0,00 |
| Relapse Necrosis | GBM-P | 36,26 | 0,21 | 0,00 |

The TIL that showed absent IFN$\gamma$ production were strongly cytotoxic, as measured in a standard CR51 release assay. The data show that TIL produce IFNgamma against autologous tumor cells in a specific fashion.

**Example 40 - Analysis of the cytolytic response of expanded TIL from patients with pancreas cancer against autologous tumor cells**

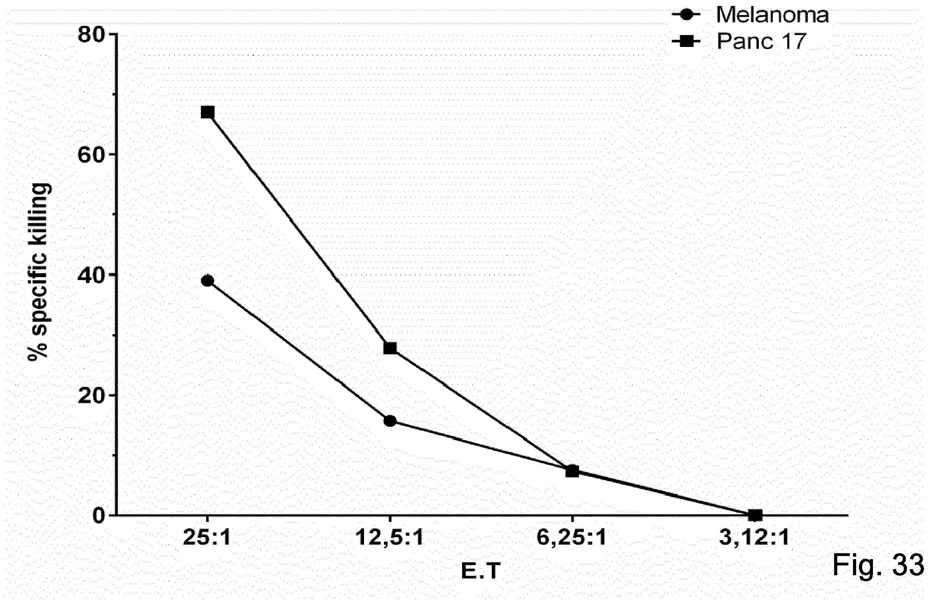

Tumor tissue was obtained from pancreatic cancer patients. TILs were expanded from the tumor tissue with the cytokine cocktail of IL-2, IL-15 and IL-21 according to the protocol of Example 28. Autologous tumor cells were generated according to the protocol in Example 29. Autologous tumor cells are labeled with radioactivity and cultured with expanded TIL for 4 h. Killing of tumor cells leads to release of radioactivity which is then measured. The principle of the method is shown in Fig. 7. For comparison and as a control, an autologous (melanoma) - TIL system was used. The results are shown in Fig. 33 confirming a cytotoxic effect. The data show that the cytokine cocktail expands TIL also from patients with pancreatic cancer. These TIL are highly focused - based on the TCR usage - and show a specific reactivity, including cytotoxic responses, against autologous tumor.

**Example 41 - Analysis of the CXCR3 Expression CD4+ cell distribution**

Tumor tissue was obtained from glioblastoma patients. TILs were expanded from the tumor tissue with the cytokine cocktail of IL-2, IL-15 and IL-21 according to the protocol of Example 27. Cells were analyzed by flow cytometry for marker expression that defined T-cell function and homing.

The results of the analysis are summarized in Table 17. The Th1 cells as well as the CXCR3 were less than 10% prior to expansion. The data show that the cytokine cocktail leads to a T-cell product in which the CD8+ cells consists essentially of CXCR3+ CD8+ T-cells. This phenotype is enabled to enter into the tumor tissue. The CD4+ T-cells almost all have a $T_{H1}$ profile. The $T_{H1}$ profile (IFNgamma and TNFalpha production) leads to improved anti-tumor responses.

Also CD3+CD4-CD8- (DN) T-cells are present, a T-cell subset, associated with strong-autoimmune and tumor responses, which also expresses the marker CXCR3 that enables better penetration into tumor tissue.

Table 17

| | | |
|---|---|---|
| **Lymphocytes** | | **76,3** |
| **CD3+** | | **84,3** |
| **CD8+** | **CD8+** | **25** |
| | **c-kit** | **0,49** |
| | **CD107a+** | **1,58** |
| | **CXCR3+** | **81,1** |
| **DP** | **DP** | **2,55** |
| **CD4+** | **CD4+** | **68,9** |
| | **c-kit** | **0,073** |
| | **CCR6+** **CCR6+** | **36,2** |
| | **TH1*** | **89,3** |
| | **TH17** | **0,047** |
| | **CCR6-** **CCR6-** | **63,8** |
| | **TH1** | **91,5** |
| | **TH2** | **0,038** |
| | **CD107a+** | **1** |
| **DN** | **DN** | **3,56** |
| | **c-kit** | **1,36** |
| | **CD107a+** | **1** |
| | **CXCR3+** | **59,1** |

**Example 42 - Recognition of commonly shared Tumor antigens by TIL from patients with pancreatic cancer**

TAAs as 15 overlapping peptides were incubated for three days with TIL and IFNγ production (pg/mL) was measured by ELISA. In addition, antigen responses were either blocked by an antibody blocking MHC class I antigen responses (W6/32) affecting CD8+ T-cells, or blocking HLA-DR (MHC class II responses, L243) affecting CD4+ T-cells. The IFNγ production (pg/mL) in each of the samples is shown in Table 18. IFNγ production in TIL that is antigen-specific, as shown by blocking with anti-MHC class I (blocking CD8+ T-cells) or with anti-MHC class II (blocking CD4+) T-cells. The results confirm that TIL produce IFNγ against commonly shared cancer antigens, particularly NY- ESO-1 or mesothelin from patients with pancreatic cancer.

Table 18

| | NY-ESO-1 | NY-ESO-1+N6/32 | NY-ESO-1 +LZ43 | Survivin | Survivin +W6/32 | Survivin +L243 | Mesothelin | Mesotelin +W6/32 | Mesothelin +L243 |
|---|---|---|---|---|---|---|---|---|---|
| Pane 1 | 6,97 | 0 | 0 | 0 | 0 | 0 | 262,23 | 15,26 | 0 |
| Panc 2 | 24,20 | 0 | 0 | 0 | 0 | 0 | 291,27 | 13,50 | 0 |
| Panc 6 | 51,02 | 31,96 | 33,74 | 77,99 | 49,24 | 36,72 | 44,46 | 34,34 | 27,21 |
| Pane 10 | 4,13 | 0 | 0 | 66,06 | 0 | 0 | 78,70 | 72,88 | 0 |
| Pane 11 | 0 | 0 | 0 | 0 | 0 | 0 | 131,16 | 0 | 0 |
| Pane 14 | 10,47 | 0 | 0 | 0 | 0 | 0 | 275,15 | n.d. | 249,95 |
| Pane 16 | 173,19 | 0 | 0 | 8,35 | 0 | 0 | 19,64 | 0 | n.d. |

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

**REFERENCES**

1. Dunn GP, Old LJ, Schreiber RD. The immunobiology of cancer immunosurveillance and immunoediting. Immunity. 2004;21 (2):137-48.

2. Rosenberg SA, Restifo NP, Yang JC, Morgan RA, Dudley ME. Adoptive cell transfer: a clinical path to effective cancer immunotherapy. Nature reviews Cancer. 2008;8(4):299-308.

3. Rosenberg SA, Packard BS, Aebersold PM, Solomon D, Topalian SL, Toy ST, et al. Use of tumor-infiltrating lymphocytes and interleukin-2 in the immunotherapy of patients with metastatic melanoma. A preliminary report. The New England journal of medicine. 1988;319(25):1676-80.

4. Robbins PF, Lu YC, El-Gamil M, Li YF, Gross C, Gartner J, et al. Mining exomic sequencing data to identify mutated antigens recognized by adoptively transferred tumor-reactive T cells. Nat Med. 2013;19(6):747-52.

5. Tran E, Turcotte S, Gros A, Robbins PF, Lu YC, Dudley ME, et al. Cancer immunotherapy based on mutation-specific CD4+ T cells in a patient with epithelial cancer. Science. 2014;344(6184):641-5.

6. Li Y, Liu S, Hernandez J, Vence L, Hwu P, Radvanyi L. MART-1-specific melanoma tumor-infiltrating lymphocytes maintaining CD28 expression have improved survival and expansion capability following antigenic restimulation in vitro. Journal of immunology. 2010;184(1):452-65.

7. June CH. Principles of adoptive T cell cancer therapy. The Journal of clinical investigation. 2007;117(5):1204-12.

8. Dudley ME, Wunderlich J, Nishimura MI, Yu D, Yang JC, Topalian SL, et al. Adoptive transfer of cloned melanoma-reactive T lymphocytes for the treatment of patients with metastatic melanoma. Journal of immunotherapy. 2001;24(4):363-73.

9. Dudley ME, Wunderlich JR, Shelton TE, Even J, Rosenberg SA. Generation of tumor-infiltrating lymphocyte cultures for use in adoptive transfer therapy for melanoma patients. Journal of immunotherapy. 2003;26(4):332-42.

10. Weber J, Atkins M, Hwu P, Radvanyi L, Sznol M, Yee C, et al. White paper on adoptive cell therapy for cancer with tumor-infiltrating lymphocytes: a report of the CTEP subcommittee on adoptive cell therapy. Clinical cancer research: an official journal of the American Association for Cancer Research. 2011;17(7):1664-73.

11. Tran KQ, Zhou J, Durflinger KH, Langhan MM, Shelton TE, Wunderlich JR, et al. Minimally cultured tumor-infiltrating lymphocytes display optimal characteristics for adoptive cell therapy. Journal of immunotherapy. 2008;31(8):742-51.

12. Nguyen LT, Yen PH, Nie J, Liadis N, Ghazarian D, Al- Habeeb A, et al. Expansion and characterization of human melanoma tumor-infiltrating lymphocytes (TILs). PloS one. 2010;5(11):e13940.

13. Zhou J, Dudley ME, Rosenberg SA, Robbins PF. Selective growth, in vitro and in vivo, of individual T cell clones from tumor-infiltrating lymphocytes obtained from patients with melanoma. Journal of immunology. 2004;173(12):7622-9.

14. Hunder NN, Wallen H, Cao J, Hendricks DW, Reilly JZ, Rodmyre R, et al.Treatment of metastatic melanoma with autologous CD4+ T cells against NY-ESO-1. The New England journal of medicine. 2008;358(25):2698-703.

15. Kagamu H, Shu S. Purification of L-selectin(low) cells promotes the generation of highly potent CD4 antitumor effector T lymphocytes. Journal of immunology. 1998;160(7):3444-52.

16. Xie Y, Akpinarli A, Maris C, Hipkiss EL, Lane M, Kwon EK, et al. Naive tumorspecific CD4(+) T cells differentiated in vivo eradicate established melanoma. The Journal of experimental medicine. 2010;207(3):651-67.

17. Mackensen A, Meidenbauer N, Vogl S, Laumer M, Berger J, Andreesen R. Phase I study of adoptive T-cell therapy using antigen-specific CD8+ T cells for the treatment of patients with metastatic melanoma. Journal of clinical oncology : official journal of the American Society of Clinical Oncology. 2006;24(31):5060-9.

18. Jager E, Gnjatic S, Nagata Y, Stockert E, Jager D, Karbach J, et al. Induction of primary NY-ESO-1 immunity: CD8+ T lymphocyte and antibody responses in peptidevaccinated patients with NY-ESO-1+ cancers. Proc Natl Acad Sci USA. 2000;97(22):12198-203.

19. Ho WY, Nguyen HN, Wolfl M, Kuball J, Greenberg PD. In vitro methods for generating CD8+ T-cell clones for immunotherapy from the naive repertoire. Journal of immunological methods. 2006;310(1-2):40-52.

20. Dudley ME, Wunderlich JR, Robbins PF, Yang JC, Hwu P, Schwartzentruber DJ, et al. Cancer regression and autoimmunity in patients after clonal repopulation with antitumor lymphocytes. Science. 2002;298(5594):850-4.

21. Lord GM, Matarese G, Howard JK, Baker RJ, Bloom SR, Lechler RI. Leptin modulates the T-cell immune response and reverses starvation-induced immunosuppression. Nature. 1998;394(6696):897-901.

22. Tanaka M, Suganami T, Kim-Saijo M, Toda C, Tsuiji M, Ochi K, et al. Role of central leptin signaling in the starvation-induced alteration of B-cell development. The Journal of neuroscience : the official journal of the Society for Neuroscience. 2011;31(23):8373-80.

23. Ravindran R, Khan N, Nakaya HI, Li S, Loebbermann J, Maddur MS, et al. Vaccine activation of the nutrient sensor GCN2 in dendritic cells enhances antigen presentation. Science. 2014;343(6168):313-7.

24. Pearson C, Silva A, Seddon B. Exogenous amino acids are essential for interleukin-7 induced CD8 T cell growth. [corrected]. PloS one. 2012;7(4):e33998.

25. Schwartzentruber DJ, Hom SS, Dadmarz R, White DE, Yannelli JR, SteinbergSM, et al. In vitro predictors of therapeutic response in melanoma patients receiving tumor- infiltrating lymphocytes and interleukin-2. Journal of clinical oncology : official journal of the American Society of Clinical Oncology. 1994;12(7):1475-83.

26. PittetMJ, Speiser DE, Valmori D, Cerottini JC, Romero P. Cutting edge: cytolytic effector function in human circulating CD8+ T cells closely correlates with CD56 surface expression. Journal of immunology. 2000;164(3):1148-52.

27. ShenX, Zhou J, Hathcock KS, Robbins P, Powell DJ, Jr., Rosenberg SA, et al.Persistence of tumor infiltrating lymphocytes in adoptive immunotherapy correlates with telomere length. Journal of immunotherapy. 2007;30(1):123-9.

28. Zhou J, Shen X, Huang J, Hodes RJ, Rosenberg SA, Robbins PF. Telomere length of transferred lymphocytes correlates with in vivo persistence and tumor regression in melanoma patients receiving cell transfer therapy. Journal of immunology. 2005;175(10):7046-52.

29. Inozume T, Hanada K, Wang QJ, Ahmadzadeh M, Wunderlich JR, Rosenberg SA, et al. Selection of CD8+PD-1+ lymphocytes in fresh human melanomas enriches for tumor-reactive T cells. Journal of immunotherapy. 2010;33(9):956- 64.

30. Zeng R, Spolski R, Finkelstein SE, Oh S, Kovanen PE, Hinrichs CS, et al. Synergy of IL-21 and IL-15 in regulating CD8+ T cell expansion and function. The Journal of experimental medicine. 2005;201(1):139-48.

31. Liu D, Song L, Wei J, Courtney AN, Gao X, Marinova E, et al. IL-15 protects NKT cells from inhibition by tumor-associated macrophages and enhances antimetastatic activity. The Journal of clinical investigation.

2012;122(6):2221-33.

32. Li Y, Bleakley M, Yee C. IL-21 influences the frequency, phenotype, and affinity of the antigen-specific CD8 T cell response. Journal of immunology. 2005;175(4): 2261-9.

33. Gattinoni L, Ji Y, Restifo NP. Wnt/beta-catenin signaling in T- cell immunity and cancer immunotherapy. Clinical cancer research an official journal of the American Association for Cancer Research. 2010;16(19):4695-701.

34. Gattinoni L, Zhong XS, Palmer DC, Ji Y, Hinrichs CS, Yu Z, et al. Wnt signaling arrests effector T cell differentiation and generates CD8+ memory stem cells. Nat Med. 2009;15(7):808-13.

35. Yi JS, Du M, Zajac AJ. A vital role for interleukin-21 in the control of a chronic viral infection. Science. 2009;324(5934):1572- 6.

36. Jager D, Karbach J, Pauligk C, Seil I, Frei C, Chen YT, et al. Humoral and cellular immune responses against the breast cancer antigen NY-BR-1: definition of two HLAA2 restricted peptide epitopes. Cancer immunity. 2005;5:11.

37. Jager E, Karbach J, Gnjatic S, Jager D, Maeurer M, Atmaca A, et al. Identification of a naturally processed NY-ESO-1 peptide recognized by CD8+ T cells in the context of HLA-B51. Cancer immunity. 2002;2:12.

38. Di Tomaso T, Mazzoleni S, Wang E, Sovena G, Clavenna D, Franzin A, et al.Immunobiological characterization of cancer stem cells isolated from glioblastoma patients. Clinical cancer research : an official journal of the American Association for Cancer Research. 2010;16(3):800-13.

39. Natsume A, Wakabayashi T, Tsujimura K, Shimato S, Ito M, Kuzushima K, et al. The DNA demethylating agent 5-aza-2'-deoxycytidine activates NY-ESO-1 antigenicity in orthotopic human glioma. International journal of cancer Journal international du cancer. 2008;122(11):2542-53.

40. Konkankit VV, Kim W, Koya RC, Eskin A, Dam MA, Nelson S, et al. Decitabine immunosensitizes human gliomas to NY-ESO-1 specific T lymphocyte targeting through the Fas/Fas ligand pathway. Journal of translational medicine. 2011;9:192.

41. Maeurer M, Hohn H, Castelli C, Salter RD, Necker A, Reichert T, et al. Antigen recognition by T cells: a strong sense of structure. Trends in immunology. 2001;22(11):599-601.

42. Maeurer MJ, Gollin SM, Martin D, Swaney W, Bryant J, Castelli C, et al. Tumor escape from immune recognition: lethal recurrent melanoma in a patient associated with downregulation of the peptide transporter protein TAP-1 and loss of expression of the immunodominant MART-1/Melan-A antigen. The Journal of clinical investigation. 1996;98(7):1633-41.

43. Maeurer MJ, Necker A, Salter RD, Castelli C, Hohn H, Karbach J, et al. Improved detection of melanoma antigen-specific T cells expressing low or high levels of CD8 by HLA-A2 tetramers presenting a Melan-A/Mart-1 peptide analogue. International journal of cancer Journal international du cancer. 2002;97(1):64-71.

44. Magalhaes I, Vudattu NK, Ahmed RK, Kuhlmann-Berenzon S, Ngo Y, Sizemore DR, et al. High content cellular immune profiling reveals differences between rhesus monkeys and men. Immunology. 2010;131(1):128-40.

The invention is further described by the following items:

1. Composition for expanding lymphocytes comprising at least two types of cytokines selected from interleukin 2 (IL-2), interleukin 15 (IL-15) and interleukin 21 (IL-21).

2. Composition according to item 1, comprising two or three types of cytokines.

3. Composition according to item 1 or 2, wherein the composition is in liquid form, in particular a cell culture medium.

4. Composition according to item 3, wherein the concentration of IL-2 in the liquid composition is in the range from 10 to 6000 U/ml, preferably in the range from 500 to 2000 U/ml, more preferably in the range from 800 to 1200 U/ml.

5. Composition according to item 3 or 4, wherein the concentration of IL-15 is in the range from 0.1 to 100 ng/ml, preferably in the range from 2 to 50 ng/ml, more preferably in the range from 5 to 20 ng/ml.

6. Composition according to any of items 3 to 5, wherein the concentration of IL-21 is in the range from 0.1 to 100 ng/ml, preferably in the range from 2 to 50 ng/ml, more preferably in the range from 5 to 20 ng/ml.

7. Method of preparing a population of clinically relevant lymphocytes, comprising the steps of:

- obtaining a body sample from a mammal in particular a tissue sample or body liquid sample, comprising at least one lymphocyte and optionally separating the cells in the body sample,
- culturing the body sample *in-vitro* to expand and/or stimulate lymphocytes in the sample wherein the culturing comprises using IL-2, IL-15 and/or IL-21,
- and optionally determining the presence of clinically relevant lymphocyte in the cultured sample.

8. Method according to item 7, wherein the clinically relevant lymphocytes are selected from tumor-reactive lymphocytes, pathogen reactive lymphocytes and autoimmune reactive lymphocytes, preferably tumor-reactive lymphocytes.

9. Method according to item 7 or 8, wherein the body sample is selected from the group consisting of peripheral blood, cord blood, bone marrow, lymph nodes liver, pleural effusion, thorax, abdominal cavity, synvial fluid, peritoneum, retroperitoneal space, thymus, and tumor.

10. Method according to item 9, wherein the body sample is selected from peripheral blood.

11. Method according to item any of items 7 to 10, wherein the mammal, in particular a human, is selected from a mammal with a tumor disease, a mammal at risk of developing a tumor disease, a mammal with an infectious disease, a mammal at risk of developing an infectious disease, a mammal with an auto immune disease, a mammal at risk of developing an autoimmune disease.

12. Method according to any of items 7 to 11, wherein the *in-vitro* culturing comprises a first expansion step comprising an incubation in culture medium comprising IL-2, IL-15 and IL-21 until lymphocytes become detectable.

13. Method according to item 12, wherein the time of incubation of the first expansion step is in the range from 6 hours to 180 days preferably in the range from 4 to 10, more preferably in the range from 6 to 8 days, most preferably about 7 days.

14. Method according to items 12 or 13, wherein the *in-vitro* culturing comprises a second expansion step comprising an incubation in culture medium comprising feeder cells and/or an antibody against CD3 in addition to IL-2, IL-15 and IL-21.

15. Method according to item 14, wherein the ratio of feeder cells to lymphocytes is in the range from 1:1 to 1:100, preferably in the range from 1:2 to 1 :50, more preferably in the range from 1:5 to 1:20, most preferably about 1:10.

16. Method according to any of items 12 to 15, wherein the first expansion step comprises adding IL-2, IL-15 and IL-21 at the same time point to the cell culture.

17. Method according to any of items 12 to 15, wherein the first expansion step comprises adding at least one of IL-2, IL-15 and IL-21 separately at a different time point to the cell culture.

18. Method according to item 17, wherein first IL-21 is added and in particular IL-15 is added second before adding IL-2.

19. Method according to item 17, wherein first IL-15 is added and in particular IL-21 is added second before adding IL-2.

20. Method according to any of items 7 to 19, wherein the clinically relevant lymphocyte population is either one of monoclonal, oligoclonal or polyclonal.

21. Method according to any of items 12 to 20, wherein the culture medium of the first and/or second expansion step comprises least one expansion antigen.

22. Method according to item 21, wherein expansion antigen is a fragment of TAA, in particular a peptide comprising at least eight continuous amino acids of an amino acid sequence that is at least 80 % identical to the amino acid sequences SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, or SEQ ID NO: 14

23. Method according to any of items 12 to 22, wherein the in-vitro culturing further comprises a refocusing step, comprising an incubation in culture medium comprising refocusing cells.

24. Method according to item 23, wherein the time of the refocusing step is in the range from 1 to 6 days, preferably 1 to 3 days.

25. Method according to item 23 or 24, wherein the ratio of refocusing cells to lymphocytes is in the range from 1:1 to 1:100, preferably in the range from 1:5 to 1:10.

26. Method according to any of items 7 to 25, wherein the culturing comprises the addition of a promoter compound to promote the expansion of a specific subgroup of lymphocytes, in particular gamma-delta T-cells ($\gamma\delta$-T-cells).

27. Method according to any of items 7 to 26, further comprising isolating the population of clinically relevant lymphocytes from the expanded cell culture.

28. Method according to any of items 7 to 27, wherein the testing for the presence of clinically relevant lymphocytes comprises using evaluation antigens.

29. Method according to item 28, wherein evaluation antigens are presented to the cultured sample in a form selected from cells, in particular tumor cells, derived from the same mammal as the cultured sample (autologous cells), at least partially genetically matched allogeneic cells, in particular tumor cells, or cells expressing the clinically relevant antigens as a transgene.

30. Method according to items 28 or 29, wherein the testing for the presence of clinically reactive lymphocytes comprises contacting the lymphocytes with at least one clinically relevant antigen and determining a change in either one of cytokine production, in particular INFy production, cell proliferation, cytotoxicity, signaling and/or intracellular phosphorylation.

31. Immunotherapy for treating or preventing an infectious disease, a tumor disease, or an auto-immune disease in a mammal, comprising the steps of:

- obtaining or generating a population of clinically relevant lymphocyte, according to the method of any of item 7 to 30, wherein the body sample is obtained from said mammal; and
- administering the population of clinically relevant lymphocytes to said mammal.

32. Immunotherapy according to items 31, wherein the tumor disease is selected from a glioblastoma and pancreas cancer.

33. Immunotherapy according to item 31 or 32, wherein the population of clinically relevant lymphocytes

- bring about regression of cancer cells in the mammal;
- interfere with the move from pre-malignant to malignant lesions;
- bring about fast senescence of tumor cells or pre-malignant cells;
- bring about removal of autoantigen-positive cells;
- bring about killing, growth arrest or containment of pathogens;
- interfere with cancer stem cells; and/or
- induce growth arrest of cancer cells, or cells expressing auto-antigens.

34. Composition according to any of items 1 to 6 for use in medical treatment, in particular for treating or preventing an infectious disease, an autoimmune disease or a tumor disease.

35. Composition for use according to item 34, wherein the use comprises a generation of a population of clinically relevant lymphocytes with the method according to any of items 7 to 31.

36. Kit for use in immunotherapy, in particular treatment of a tumor disease, wherein the kit comprises IL-2, IL-15, and IL-21 and optionally at least one of a component that stimulates the TCR, in particular OKT3, costimulatory molecules, feeder cells and a peptide comprising at the sequence of at least one clinically relevant antigen.

37. Clinically relevant lymphocyte obtained by a method according to any of items 7 to 30, wherein the clinically relevant lymphocyte is selected from a B-cell, an NK cell and T-cell wherein the T-cell is selected from a helper T-cell ($T_H$-cell or CD4$^+$-T-cell), in particular a $T_{H1}$-cell, a cytotoxic T-cell ($T_C$-cell or CD8$^+$-T-cell), in particular CD8$^+$CXCR3$^+$ T-cell, a memory T-cell, in particular a central memory T-cell ($T_{CM}$-cell), stem memory T-cell ($T_{SCM}$) or peripheral memory cell ($T_{PM}$-cell), a gamma-delta T-cell ($\gamma\delta$-T-cell), a NK-T-cell, a Mucosal-associated invariant T-cell (MAIT), a double-negative T-cell (CD3$^+$CD4$^-$CD8$^-$T-cell).

38. Clinically relevant lymphocyte according to item 36 or 37, wherein the lymphocyte is selected from

- a lymphocyte that expresses molecules that facilitate entry into tissues, in particular tumor- or infected or inflamed tissue (e.g. CXCR3); and
- a lymphocyte that is enriched for markers of any of long-term memory, in particular CD117 and c-kit), activation of antigen-specific immune responses, in particular 4-1BB, cytolytic immune cell responses, in particular CD107a.

39. Lymphocyte obtained by a method according to any of items 7 to 30,

- expressing molecules and cytokines promoting the formation of a combination of lymphocytes useful for medical application, comprising T-cell precursors, $T_{CM}$ and/or $T_{PM}$;
- expressing molecules and cytokines that promote the expansion of clinically relevant lymphocytes,
- producing cytokines selected from IFN$\gamma$, TNF$\alpha$, IL-2, IL-17 and any combination thereof.
- is a CD3+CD4-CD8- T-cell.

40. Population of clinically relevant lymphocytes obtained by a method according to any of items 7 to 30.

41. Population of lymphocytes obtained by a method according to any of items 7 to 30 comprising a population of clinically relevant lymphocytes.

42. Population of lymphocytes according to item 41, characterized by one or more of the following features:

- the percentage of $T_{reg}$ based on the total number of T cells is below 5 %, preferably below 3 %;
- the percentage of $T_{H1}$-cells based on the total number of $T_H$-cells is at least 50 %, preferably at least 70 %, more preferably at least 80 %,
- the percentage of CXCR3+ T-cells based on the total number of CD8$^+$ T-cells is at least 50 %, preferably at least 70 %, more preferably at least 80 %,
- the percentage of 4-1BB+ T-cells based on the total number of T-cells is at least 1 %, preferably at least 2 %, more preferably at least 2.5 %,
- the percentage of CD117+ T-cells based on the total number of T-cells is at least 1 %, preferably at least 2 %, more preferably at least 2.5 %,
- the percentage of CD3+CD4-CD8- cells based on the total number of T-cells is at least 1 %, preferably at least 3 %, more preferably at least 5 %; and
- the percentage of $\gamma\delta$T-cells based on the total number of T-cells is at least 1 %, preferably at least 3 %, more preferably at least 5 %.

43. Population of lymphocytes according to item 41 or 42, characterized by one or more of the following features:

- the percentage of precursor T-cells (CD45RA+CCR7+) based on the total number of T-cells is at least 1 %, preferably at least 2 %, more preferably at least 3 %;
- the percentage of central memory T-cells (CD45RA-CCR7+) based on the total number of T-cells is at least 2 %, preferably at least 5 %, more preferably at least 10%;
- the percentage of peripheral memory T-cells (CD45RA-CCR7-) based on the total number of T-cells is at least 2 %, preferably at least 5 %, more preferably at least 10 %; and
- the percentage of effector T-cells (CD45RA+CCR7-) based on the total number of T-cells is at least 1 %, preferably at least 3 % more preferably at least 5 %.

44. Population of lymphocytes according to any of items 41 to 43, characterized by one or more of the following features:

- the percentage of clinically relevant T-cells is at least 0.1 % based on the total number of T-cells determined by multimeric soluble MHC-peptide complexes or intracellular cytokine production;
- the intracellular cytokine production after antigen stimulation has a value that is at least 2-fold of the standard deviation without antigen stimulation; and
- the CD107a induction after antigen stimulation has a value that is at least 2-fold of the standard deviation without antigen stimulation.

**Claims**

1. A composition for expanding lymphocytes comprising interleukin 2 (IL-2), interleukin 15 (IL-15) and interleukin 21 (IL-21).

2. The composition according to claim 1, wherein the composition is in liquid form, in particular a cell culture medium, wherein the concentration of IL-2 in the liquid composition is in the range from 10 to 6000 U/ml, preferably in the range from 500 to 2000 U/ml, more preferably in the range from 800 to 1200 U/ml.

3. The composition according to claim 2, wherein the concentration of IL-15 is in the range from 0.1 to 100 ng/ml, preferably in the range from 2 to 50 ng/ml, more preferably in the range from 5 to 20 ng/ml and wherein the concentration of IL-21 is in the range from 0.1 to 100 ng/ml, preferably in the range from 2 to 50 ng/ml, more preferably in the range from 5 to 20 ng/ml.

4. A method of preparing a population of clinically relevant lymphocytes, comprising the steps of:

   - obtaining a body sample from a mammal in particular a tissue sample or body liquid sample, comprising at least one lymphocyte and optionally separating the cells in the body sample,
   - culturing the body sample *in-vitro* to expand and/or stimulate lymphocytes in the sample wherein the culturing comprises using IL-2, IL-15 and IL-21,
   - and optionally determining the presence of clinically relevant lymphocyte in the cultured sample;

   wherein the *in-vitro* culturing comprises a first expansion step comprising an incubation in culture medium comprising IL-2, IL-15 and IL-21 until lymphocytes become detectable.

5. The method according to claim 4, wherein the clinically relevant lymphocytes are selected from tumor-reactive lymphocytes, pathogen reactive lymphocytes and autoimmune reactive lymphocytes, preferably tumor-reactive lymphocytes.

6. The method according to claim 4 or 5, wherein the body sample is selected from the group consisting of peripheral blood, cord blood, bone marrow, lymph nodes liver, pleural effusion, thorax, abdominal cavity, synvial fluid, peritoneum, retroperitoneal space, thymus, and tumor, preferably peripheral blood or tumor.

7. The method according to any of claims 4 to 6, wherein the time of incubation of the first expansion step is in the range from 6 hours to 180 days preferably in the range from 4 to 10, more preferably in the range from 6 to 8 days, most preferably about 7 days.

8. The method according to any of claims 4 to 7, wherein the *in-vitro* culturing comprises a second expansion step comprising an incubation in culture medium comprising feeder cells and/or an antibody against CD3 in addition to IL-2, IL-15 and IL-21.

9. The method according to any of claims 4 to 8, wherein the first expansion step comprises adding IL-2, IL-15 and IL-21 at the same time point to the cell culture.

10. The method according to any of claims 4 to 8, wherein the first expansion step comprises adding at least one of IL-2, IL-15 and IL-21 separately at a different time point to the cell culture, preferably, first IL-21 is added and in particular IL-15 is added second before adding IL-2.

11. The method according to any of claims 7 to 10, wherein the culture medium of the first and/or second expansion step comprises least one expansion antigen, wherein expansion antigen is preferably a fragment of TAA, more preferably a peptide comprising at least eight continuous amino acids of an amino acid sequence that is at least 80 % identical to the amino acid sequences SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, or SEQ ID NO: 14.

12. The method according to any of claims 7 to 11, wherein the *in-vitro* culturing further comprises a refocusing step, comprising an incubation in culture medium comprising refocusing cells, wherein preferably the time of the refocusing step is in the range from 1 to 6 days, preferably 1 to 3 days.

13. The method according to any of claims 4 to 12, wherein the testing for the presence of clinically relevant lymphocytes comprises using evaluation antigens and the evaluation antigens are presented to the cultured sample in a form selected from cells, in particular tumor cells, derived from the same mammal as the cultured sample (autologous cells), at least partially genetically matched allogeneic cells, in particular tumor cells, or cells expressing the clinically relevant antigens as a transgene.

14. The method according to claim 14, wherein the testing for the presence of clinically reactive lymphocytes comprises contacting the lymphocytes with at least one clinically relevant antigen and determining a change in either one of cytokine production, in particular INF$\gamma$ production, cell proliferation, cytotoxicity, signaling and/or intracellular phosphorylation.

15. Population of lymphocytes obtained by a method according to any of claims 4 to 14 comprising a population of clinically relevant lymphocytes, **characterized by** one or more of the following features:

- the percentage of $T_{reg}$ based on the total number of T cells is below 5 %, preferably below 3 %;
- the percentage of $T_{H1}$-cells based on the total number of $T_H$-cells is at least 50 %, preferably at least 70 %, more preferably at least 80 %,
- the percentage of CXCR3+ T-cells based on the total number of CD8$^+$ T-cells is at least 50 %, preferably at least 70 %, more preferably at least 80 %,
- the percentage of 4-1BB+ T-cells based on the total number of T-cells is at least 1 %, preferably at least 2 %, more preferably at least 2.5 %,
- the percentage of CD117+ T-cells based on the total number of T-cells is at least 1 %, preferably at least 2 %, more preferably at least 2.5 %,
- the percentage of CD3+CD4-CD8- cells based on the total number of T-cells is at least 1 %, preferably at least 3 %, more preferably at least 5 %; and
- the percentage of $\gamma\delta$T-cells based on the total number of T-cells is at least 1 %, preferably at least 3 %, more preferably at least 5 %.

Day 1      Day 2      Day 7

3,68      5,16      48,2

IL-2/15/21
With Zolo

CD3 – ECD

TCRgamma/delta

Fig. 1

EP 3 838 288 A1

Fig. 2

Day 18 *post stimulation*

7,05 %

Day 1 *ex-vivo*

0,89%

Fig. 3

Fig. 4a

Fig. 4b

Fig. 4c

EP 3 838 288 A1

## 1,4% CD8s

## 0,56% DNs

## 0,34% CD8s

## 0,45% DNs

Fig. 5

Fig. 6a

Fig. 6b

Fig. 6c

Fig. 6d     Fig. 6e     Fig. 6f

CD107a

CD127

Cd117

Side Scatter

CD3+CD8+ T cells
●CD3+CD8+ dextramer +

EP 3 838 288 A1

Fig. 7a    Fig. 7b    Fig. 7c

Day0

Fig. 7d    Fig. 7e    Fig. 7f

T harvest –Day 18

medium affinity    high affinity    low affinity

CD8

Fig. 8b

Fig. 8d

APC-NYESO1

APC-NYESO1

1,85

9,25

T harvest –Day 18

T0

APC-Negative

APC-Negative

0,90

0

CD8

Fig. 8a

Fig. 8c

Fig. 9a — Patient 3 CD4+ T cells

Fig. 9b — Patient 3 Double Negative T cells

Fig. 9c — Patient 3 CD8+ T cells

Day zero

Day 18

CD45RA

effectors    precursor

CCR7

peripheral    central
: cells can
enter into
tumor

Fig. 10a

Fig. 10b

EP 3 838 288 A1

Fig. 11a    Fig. 11b

Fig. 12a
Fig. 12b
Fig. 12c

HPV L1 Peptide
PMA/Iono
Medium

2,23
23,6
0,77

CD107a-AF700

Ancestry
gating to
CD8 T-cells

Fig. 12d     Fig. 12e     Fig. 12f

Fig. 13

Fig. 14

EP 3 838 288 A1

Fig. 15

EP 3 838 288 A1

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

EP 3 838 288 A1

Panc 9 WBA-ELISA

Fig. 30

Fig. 31

EP 3 838 288 A1

Fig. 32

EP 3 838 288 A1

Fig. 33

EP 3 838 288 A1

Europäisches Patentamt
European Patent Office
Office européen des brevets

# EUROPEAN SEARCH REPORT

Application Number

EP 20 20 9411

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2008/066609 A1 (HUTCHINSON FRED CANCER RES [US]; RIDDELL STANLEY R [US]; BERGER CAROLI) 5 June 2008 (2008-06-05)<br>* page 2, line 27 - page 3, line 18 *<br>* page 4, line 3 - line 13 *<br>* page 5, line 13 - line 17 *<br>* page 19, line 28 - line 32 *<br>* page 24, line 28 - page 25, line 6 * | 1-15 | INV.<br>A61K38/20<br>A61K38/21<br>A61K39/00<br>A61K35/14<br>A61K35/26<br>C12N5/07<br>G01N33/50<br>A61P35/00 |
| Y | WO 2007/071390 A1 (SENTOCLONE THERAPEUTICS AB [SE]; WINQVIST OLA [SE]; THOERN MAGNUS [SE]) 28 June 2007 (2007-06-28)<br>* page 3, paragraph 1 - paragraph 2 *<br>* page 11, paragraph 3 - paragraph 4 *<br>* page 14, paragraph 3 *<br>* page 19, paragraph 3 - paragraph 5; claims 49-55 * | 1-15 | A61P37/00<br>A61P31/00<br>C12N5/0783 |
| Y | WO 2007/071409 A1 (SENTOCIONE THERAPEUTICS AB [SE]; WINQVIST OLA [SE]; THOERN MAGNUS [SE]) 28 June 2007 (2007-06-28)<br>* page 4, paragraph 1 - paragraph 2 *<br>* page 12, paragraph 3 *<br>* page 20, paragraph 2; claim 84 * | 1-15 | |

TECHNICAL FIELDS SEARCHED (IPC)

A61K
C12N
G01N
A61P

-/--

The present search report has been drawn up for all claims

| Place of search | Date of completion of the search | Examiner |
|---|---|---|
| Munich | 21 April 2021 | Zellner, Eveline |

Europäisches
Patentamt

European
Patent Office

Office européen
des brevets

# EUROPEAN SEARCH REPORT

Application Number

EP 20 20 9411

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HUARTE E ET AL: "Ex vivo expansion of tumor specific lymphocytes with IL-15 and IL-21 for adoptive immunotherapy in melanoma", CANCER LETTERS, NEW YORK, NY, US, vol. 285, no. 1, 18 November 2009 (2009-11-18), pages 80-88, XP026643980, ISSN: 0304-3835, DOI: 10.1016/J.CANLET.2009.05.003 [retrieved on 2009-06-06] * page 81, column 1, paragraph 4 - column 2, paragraph 4; table 1 * * page 85, column 1, paragraph 2 - page 86, column 1, paragraph 2 * ----- | 1-15 | |
| Y | SASKIA JAM SANTEGOETS ET AL: "IL-21 promotes the expansion of CD27+CD28+ tumor infiltrating lymphocytes with high cytotoxic potential and low collateral expansion of regulatory T cells", JOURNAL OF TRANSLATIONAL MEDICINE, BIOMED CENTRAL, LONDON, GB, vol. 11, no. 1, 12 February 2013 (2013-02-12), page 37, XP021146988, ISSN: 1479-5876, DOI: 10.1186/1479-5876-11-37 * abstract * ----- -/-- | 1-15 | TECHNICAL FIELDS SEARCHED (IPC) |

The present search report has been drawn up for all claims

| Place of search | Date of completion of the search | Examiner |
|---|---|---|
| Munich | 21 April 2021 | Zellner, Eveline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

page 2 of 3

Europäisches
Patentamt

European
Patent Office

Office européen
des brevets

# EUROPEAN SEARCH REPORT

Application Number

EP 20 20 9411

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | &NA;: "Abstracts for the 25th Annual Scientific Meeting of theInternational Society for Biological Therapy of Cancer", JOURNAL OF IMMUNOTHERAPY, vol. 33, no. 8, 1 October 2010 (2010-10-01), pages 859-920, XP055007199, ISSN: 1524-9557, DOI: 10.1097/CJI.0b013e3181f1e08d | 1,4-10, 14 | |
| Y | * page 867, column 2, paragraph 2 * | 1-15 | |
| Y | WO 2010/056144 A2 (INST DE MEDICINA MOLECULAR [PT]; UNIV LISBOA [PT] ET AL.) 20 May 2010 (2010-05-20) * page 16, line 9 - line 24; claim 15; example 1 * | 1-15 | |
| X | US 2012/121544 A1 (CHOI INPYO [KR] ET AL) 17 May 2012 (2012-05-17) | 1,4-6, 8-10,14, 15 | |
| Y | * paragraphs [0088], [0089], [0070], [0090], [0092]; examples 2,3 * | 1-15 | TECHNICAL FIELDS SEARCHED (IPC) |

The present search report has been drawn up for all claims

| Place of search | Date of completion of the search | Examiner |
|---|---|---|
| Munich | 21 April 2021 | Zellner, Eveline |

EPO FORM 1503 03.82 (P04C01)

## ANNEX TO THE EUROPEAN SEARCH REPORT
## ON EUROPEAN PATENT APPLICATION NO.

EP 20 20 9411

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2021

| Patent document cited in search report | | | Publication date | Patent family member(s) | | | Publication date |
|---|---|---|---|---|---|---|---|
| WO | 2008066609 | A1 | 05-06-2008 | AU | 2007325978 | A1 | 05-06-2008 |
| | | | | CA | 2670433 | A1 | 05-06-2008 |
| | | | | EP | 2099902 | A1 | 16-09-2009 |
| | | | | ES | 2685756 | T3 | 11-10-2018 |
| | | | | US | 2008131415 | A1 | 05-06-2008 |
| | | | | US | 2014356398 | A1 | 04-12-2014 |
| | | | | US | 2019359938 | A1 | 28-11-2019 |
| | | | | WO | 2008066609 | A1 | 05-06-2008 |
| WO | 2007071390 | A1 | 28-06-2007 | AU | 2006328945 | A1 | 28-06-2007 |
| | | | | US | 2009220472 | A1 | 03-09-2009 |
| | | | | WO | 2007071390 | A1 | 28-06-2007 |
| WO | 2007071409 | A1 | 28-06-2007 | US | 2009123443 | A1 | 14-05-2009 |
| | | | | WO | 2007071409 | A1 | 28-06-2007 |
| WO | 2010056144 | A2 | 20-05-2010 | AU | 2009314704 | A1 | 20-05-2010 |
| | | | | BR | PI0915255 | A2 | 06-02-2018 |
| | | | | CA | 2743502 | A1 | 20-05-2010 |
| | | | | CN | 102216448 | A | 12-10-2011 |
| | | | | EP | 2356222 | A2 | 17-08-2011 |
| | | | | JP | 2012508575 | A | 12-04-2012 |
| | | | | US | 2012114675 | A1 | 10-05-2012 |
| | | | | WO | 2010056144 | A2 | 20-05-2010 |
| US | 2012121544 | A1 | 17-05-2012 | CN | 102356154 | A | 15-02-2012 |
| | | | | KR | 20100045704 | A | 04-05-2010 |
| | | | | US | 2012121544 | A1 | 17-05-2012 |
| | | | | WO | 2010047475 | A2 | 29-04-2010 |

EPO FORM P0459

For more details about this annex : see Official Journal of the European Patent Office, No. 12/82

**REFERENCES CITED IN THE DESCRIPTION**

**Non-patent literature cited in the description**

- **NEEDLEMAN ; WUNSCH.** *J. Mol. Biol.,* 1970, vol. 48, 443-453
- **RICE.** EMBOSS: The European Molecular Biology Open Software Suite. *Trends Genet.,* 2000, vol. 16, 276-277
- **DUNN GP ; OLD LJ ; SCHREIBER RD.** The immunobiology of cancer immunosurveillance and immunoediting. *Immunity,* 2004, vol. 21 (2), 137-48
- **ROSENBERG SA ; RESTIFO NP ; YANG JC ; MORGAN RA ; DUDLEY ME.** Adoptive cell transfer: a clinical path to effective cancer immunotherapy. *Nature reviews Cancer,* 2008, vol. 8 (4), 299-308
- **ROSENBERG SA ; PACKARD BS ; AEBERSOLD PM ; SOLOMON D ; TOPALIAN SL ; TOY ST et al.** Use of tumor-infiltrating lymphocytes and interleukin-2 in the immunotherapy of patients with metastatic melanoma. A preliminary report. *The New England journal of medicine,* 1988, vol. 319 (25), 1676-80
- **ROBBINS PF ; LU YC ; EL-GAMIL M ; LI YF ; GROSS C ; GARTNER J et al.** Mining exomic sequencing data to identify mutated antigens recognized by adoptively transferred tumor-reactive T cells. *Nat Med.,* 2013, vol. 19 (6), 747-52
- **TRAN E ; TURCOTTE S ; GROS A ; ROBBINS PF ; LU YC ; DUDLEY ME et al.** Cancer immunotherapy based on mutation-specific CD4+ T cells in a patient with epithelial cancer. *Science,* 2014, vol. 344 (6184), 641-5
- **LI Y ; LIU S ; HERNANDEZ J ; VENCE L ; HWU P ; RADVANYI L.** MART-1-specific melanoma tumor-infiltrating lymphocytes maintaining CD28 expression have improved survival and expansion capability following antigenic restimulation in vitro. *Journal of immunology,* 2010, vol. 184 (1), 452-65
- **JUNE CH.** Principles of adoptive T cell cancer therapy. *The Journal of clinical investigation,* 2007, vol. 117 (5), 1204-12
- **DUDLEY ME ; WUNDERLICH J ; NISHIMURA MI ; YU D ; YANG JC ; TOPALIAN SL et al.** Adoptive transfer of cloned melanoma-reactive T lymphocytes for the treatment of patients with metastatic melanoma. *Journal of immunotherapy,* 2001, vol. 24 (4), 363-73
- **DUDLEY ME ; WUNDERLICH JR ; SHELTON TE ; EVEN J ; ROSENBERG SA.** Generation of tumor-infiltrating lymphocyte cultures for use in adoptive transfer therapy for melanoma patients. *Journal of immunotherapy,* 2003, vol. 26 (4), 332-42
- **WEBER J ; ATKINS M ; HWU P ; RADVANYI L ; SZNOL M ; YEE C et al.** White paper on adoptive cell therapy for cancer with tumor-infiltrating lymphocytes: a report of the CTEP subcommittee on adoptive cell therapy. *Clinical cancer research: an official journal of the American Association for Cancer Research,* 2011, vol. 17 (7), 1664-73
- **TRAN KQ ; ZHOU J ; DURFLINGER KH ; LANGHAN MM ; SHELTON TE ; WUNDERLICH JR et al.** Minimally cultured tumor-infiltrating lymphocytes display optimal characteristics for adoptive cell therapy. *Journal of immunotherapy,* 2008, vol. 31 (8), 742-51
- **NGUYEN LT ; YEN PH ; NIE J ; LIADIS N ; GHAZARIAN D ; AL- HABEEB A et al.** Expansion and characterization of human melanoma tumor-infiltrating lymphocytes (TILs). *PloS one,* 2010, vol. 5 (11), e13940
- **ZHOU J ; DUDLEY ME ; ROSENBERG SA ; ROBBINS PF.** Selective growth, in vitro and in vivo, of individual T cell clones from tumor-infiltrating lymphocytes obtained from patients with melanoma. *Journal of immunology,* 2004, vol. 173 (12), 7622-9
- **HUNDER NN ; WALLEN H ; CAO J ; HENDRICKS DW ; REILLY JZ ; RODMYRE R et al.** Treatment of metastatic melanoma with autologous CD4+ T cells against NY-ESO-1. *The New England journal of medicine,* 2008, vol. 358 (25), 2698-703
- **KAGAMU H ; SHU S.** Purification of L-selectin(low) cells promotes the generation of highly potent CD4 antitumor effector T lymphocytes. *Journal of immunology,* 1998, vol. 160 (7), 3444-52
- **XIE Y ; AKPINARLI A ; MARIS C ; HIPKISS EL ; LANE M ; KWON EK et al.** Naive tumorspecific CD4(+) T cells differentiated in vivo eradicate established melanoma. *The Journal of experimental medicine,* 2010, vol. 207 (3), 651-67
- **MACKENSEN A ; MEIDENBAUER N ; VOGL S ; LAUMER M ; BERGER J ; ANDREESEN R.** Phase I study of adoptive T-cell therapy using antigen-specific CD8+ T cells for the treatment of patients with metastatic melanoma. *Journal of clinical oncology : official journal of the American Society of Clinical Oncology,* 2006, vol. 24 (31), 5060-9

- **JAGER E ; GNJATIC S ; NAGATA Y ; STOCKERT E ; JAGER D ; KARBACH J et al.** Induction of primary NY-ESO-1 immunity: CD8+ T lymphocyte and antibody responses in peptidevaccinated patients with NY-ESO-1+ cancers. *Proc Natl Acad Sci USA.,* 2000, vol. 97 (22), 12198-203
- **HO WY ; NGUYEN HN ; WOLFL M ; KUBALL J ; GREENBERG PD.** In vitro methods for generating CD8+ T-cell clones for immunotherapy from the naive repertoire. *Journal of immunological methods,* 2006, vol. 310 (1-2), 40-52
- **DUDLEY ME ; WUNDERLICH JR ; ROBBINS PF ; YANG JC ; HWU P ; SCHWARTZENTRUBER DJ et al.** Cancer regression and autoimmunity in patients after clonal repopulation with antitumor lymphocytes. *Science,* 2002, vol. 298 (5594), 850-4
- **LORD GM ; MATARESE G ; HOWARD JK ; BAKER RJ ; BLOOM SR ; LECHLER RI.** Leptin modulates the T-cell immune response and reverses starvation-induced immunosuppression. *Nature,* 1998, vol. 394 (6696), 897-901
- **TANAKA M ; SUGANAMI T ; KIM-SAIJO M ; TODA C ; TSUIJI M ; OCHI K et al.** Role of central leptin signaling in the starvation-induced alteration of B-cell development. *The Journal of neuroscience : the official journal of the Society for Neuroscience,* 2011, vol. 31 (23), 8373-80
- **RAVINDRAN R ; KHAN N ; NAKAYA HI ; LI S ; LOEBBERMANN J ; MADDUR MS et al.** Vaccine activation of the nutrient sensor GCN2 in dendritic cells enhances antigen presentation. *Science,* 2014, vol. 343 (6168), 313-7
- **PEARSON C ; SILVA A ; SEDDON B.** Exogenous amino acids are essential for interleukin-7 induced CD8 T cell growth. [corrected. *PloS one,* 2012, vol. 7 (4), e33998
- **SCHWARTZENTRUBER DJ ; HOM SS ; DAD-MARZ R ; WHITE DE ; YANNELLI JR ; STEIN-BERGSM et al.** In vitro predictors of therapeutic response in melanoma patients receiving tumor- infiltrating lymphocytes and interleukin-2. *Journal of clinical oncology : official journal of the American Society of Clinical Oncology,* 1994, vol. 12 (7), 1475-83
- **PITTETMJ ; SPEISER DE ; VALMORI D ; CEROTTINI JC ; ROMERO P.** Cutting edge: cytolytic effector function in human circulating CD8+ T cells closely correlates with CD56 surface expression. *Journal of immunology,* 2000, vol. 164 (3), 1148-52
- **SHENX ; ZHOU J ; HATHCOCK KS ; ROBBINS P ; POWELL DJ, JR. ; ROSENBERG SA et al.** Persistence of tumor infiltrating lymphocytes in adoptive immunotherapy correlates with telomere length. *Journal of immunotherapy,* 2007, vol. 30 (1), 123-9
- **ZHOU J ; SHEN X ; HUANG J ; HODES RJ ; ROSENBERG SA ; ROBBINS PF.** Telomere length of transferred lymphocytes correlates with in vivo persistence and tumor regression in melanoma patients receiving cell transfer therapy. *Journal of immunology,* 2005, vol. 175 (10), 7046-52
- **INOZUME T ; HANADA K ; WANG QJ ; AHMAD-ZADEH M ; WUNDERLICH JR ; ROSENBERG SA et al.** Selection of CD8+PD-1+ lymphocytes in fresh human melanomas enriches for tumor-reactive T cells. *Journal of immunotherapy,* 2010, vol. 33 (9), 956-64
- **ZENG R ; SPOLSKI R ; FINKELSTEIN SE ; OH S ; KOVANEN PE ; HINRICHS CS et al.** Synergy of IL-21 and IL-15 in regulating CD8+ T cell expansion and function. *The Journal of experimental medicine,* 2005, vol. 201 (1), 139-48
- **LIU D ; SONG L ; WEI J ; COURTNEY AN ; GAO X ; MARINOVA E et al.** IL-15 protects NKT cells from inhibition by tumor-associated macrophages and enhances antimetastatic activity. *The Journal of clinical investigation,* 2012, vol. 122 (6), 2221-33
- **LI Y ; BLEAKLEY M ; YEE C.** IL-21 influences the frequency, phenotype, and affinity of the antigen-specific CD8 T cell response. *Journal of immunology,* 2005, vol. 175 (4), 2261-9
- **GATTINONI L ; JI Y ; RESTIFO NP.** Wnt/beta-catenin signaling in T- cell immunity and cancer immunotherapy. *Clinical cancer research an official journal of the American Association for Cancer Research,* 2010, vol. 16 (19), 4695-701
- **GATTINONI L ; ZHONG XS ; PALMER DC ; JI Y ; HINRICHS CS ; YU Z et al.** Wnt signaling arrests effector T cell differentiation and generates CD8+ memory stem cells. *Nat Med.,* 2009, vol. 15 (7), 808-13
- **YI JS ; DU M ; ZAJAC AJ.** A vital role for interleukin-21 in the control of a chronic viral infection. *Science,* 2009, vol. 324 (5934), 1572-6
- **JAGER D ; KARBACH J ; PAULIGK C ; SEIL I ; FREI C ; CHEN YT et al.** Humoral and cellular immune responses against the breast cancer antigen NY-BR-1: definition of two HLAA2 restricted peptide epitopes. *Cancer immunity,* 2005, vol. 5, 11
- **JAGER E ; KARBACH J ; GNJATIC S ; JAGER D ; MAEURER M ; ATMACA A et al.** Identification of a naturally processed NY-ESO-1 peptide recognized by CD8+ T cells in the context of HLA-B51. *Cancer immunity,* 2002, vol. 2, 12
- **DI TOMASO T ; MAZZOLENI S ; WANG E ; SOVENA G ; CLAVENNA D ; FRANZIN A et al.** Immunobiological characterization of cancer stem cells isolated from glioblastoma patients. *Clinical cancer research : an official journal of the American Association for Cancer Research,* 2010, vol. 16 (3), 800-13

- **NATSUME A ; WAKABAYASHI T ; TSUJIMURA K ; SHIMATO S ; ITO M ; KUZUSHIMA K et al.** The DNA demethylating agent 5-aza-2'-deoxycytidine activates NY-ESO-1 antigenicity in orthotopic human glioma. *International journal of cancer Journal international du cancer,* 2008, vol. 122 (11), 2542-53
- **KONKANKIT VV ; KIM W ; KOYA RC ; ESKIN A ; DAM MA ; NELSON S et al.** Decitabine immunosensitizes human gliomas to NY-ESO-1 specific T lymphocyte targeting through the Fas/Fas ligand pathway. *Journal of translational medicine,* 2011, vol. 9, 192
- **MAEURER M ; HOHN H ; CASTELLI C ; SALTER RD ; NECKER A ; REICHERT T et al.** Antigen recognition by T cells: a strong sense of structure. *Trends in immunology,* 2001, vol. 22 (11), 599-601
- **MAEURER MJ ; GOLLIN SM ; MARTIN D ; SWANEY W ; BRYANT J ; CASTELLI C et al.** Tumor escape from immune recognition: lethal recurrent melanoma in a patient associated with downregulation of the peptide transporter protein TAP-1 and loss of expression of the immunodominant MART-1/Melan-A antigen. *The Journal of clinical investigation,* 1996, vol. 98 (7), 1633-41
- **MAEURER MJ ; NECKER A ; SALTER RD ; CASTELLI C ; HOHN H ; KARBACH J et al.** Improved detection of melanoma antigen-specific T cells expressing low or high levels of CD8 by HLA-A2 tetramers presenting a Melan-A/Mart-1 peptide analogue. *International journal of cancer Journal international du cancer,* 2002, vol. 97 (1), 64-71
- **MAGALHAES I ; VUDATTU NK ; AHMED RK ; KUHLMANN-BERENZON S ; NGO Y ; SIZEMORE DR et al.** High content cellular immune profiling reveals differences between rhesus monkeys and men. *Immunology,* 2010, vol. 131 (1), 128-40

SEQUENCE LISTING

<110> PolyBioCept AB

<120> EXPANSION OF LYMPHOCYTES WITH A CYTOKINE COMPOSITION FOR ACTIVE
      CELLULAR IMMUNOTHERAPY

<130> 7027/P/1003-WO2

<150> DE 10 2014 211 167.6
<151> 2014-06-11

<160> 15

<170> PatentIn version 3.5

<210> 1
<211> 153
<212> PRT
<213> Homo sapiens

<400> 1

Met Tyr Arg Met Gln Leu Leu Ser Cys Ile Ala Leu Ser Leu Ala Leu
1               5                   10                  15


Val Thr Asn Ser Ala Pro Thr Ser Ser Ser Thr Lys Lys Thr Gln Leu
            20                  25                  30


Gln Leu Glu His Leu Leu Leu Asp Leu Gln Met Ile Leu Asn Gly Ile
            35                  40                  45


Asn Asn Tyr Lys Asn Pro Lys Leu Thr Arg Met Leu Thr Phe Lys Phe
        50                  55                  60


Tyr Met Pro Lys Lys Ala Thr Glu Leu Lys His Leu Gln Cys Leu Glu
65                  70                  75                  80


Glu Glu Leu Lys Pro Leu Glu Glu Val Leu Asn Leu Ala Gln Ser Lys
                85                  90                  95


Asn Phe His Leu Arg Pro Arg Asp Leu Ile Ser Asn Ile Asn Val Ile
            100                 105                 110


Val Leu Glu Leu Lys Gly Ser Glu Thr Thr Phe Met Cys Glu Tyr Ala
            115                 120                 125


Asp Glu Thr Ala Thr Ile Val Glu Phe Leu Asn Arg Trp Ile Thr Phe
        130                 135                 140


Cys Gln Ser Ile Ile Ser Thr Leu Thr
145                 150

<210> 2
<211> 162
<212> PRT
<213> Homo sapiens

<400> 2

Met Arg Ile Ser Lys Pro His Leu Arg Ser Ile Ser Ile Gln Cys Tyr
1               5                   10                  15

Leu Cys Leu Leu Leu Asn Ser His Phe Leu Thr Glu Ala Gly Ile His
            20                  25                  30

Val Phe Ile Leu Gly Cys Phe Ser Ala Gly Leu Pro Lys Thr Glu Ala
        35                  40                  45

Asn Trp Val Asn Val Ile Ser Asp Leu Lys Lys Ile Glu Asp Leu Ile
    50                  55                  60

Gln Ser Met His Ile Asp Ala Thr Leu Tyr Thr Glu Ser Asp Val His
65                  70                  75                  80

Pro Ser Cys Lys Val Thr Ala Met Lys Cys Phe Leu Leu Glu Leu Gln
                85                  90                  95

Val Ile Ser Leu Glu Ser Gly Asp Ala Ser Ile His Asp Thr Val Glu
            100                 105                 110

Asn Leu Ile Ile Leu Ala Asn Asn Ser Leu Ser Ser Asn Gly Asn Val
        115                 120                 125

Thr Glu Ser Gly Cys Lys Glu Cys Glu Glu Leu Glu Glu Lys Asn Ile
    130                 135                 140

Lys Glu Phe Leu Gln Ser Phe Val His Ile Val Gln Met Phe Ile Asn
145                 150                 155                 160

Thr Ser


<210> 3
<211> 155
<212> PRT
<213> Homo sapiens

<400> 3

Met Glu Arg Ile Val Ile Cys Leu Met Val Ile Phe Leu Gly Thr Leu
1               5                   10                  15

Val His Lys Ser Ser Ser Gln Gly Gln Asp Arg His Met Ile Arg Met

                    20                          25                          30


    Arg Gln Leu Ile Asp Ile Val Asp Gln Leu Lys Asn Tyr Val Asn Asp
            35                  40                  45


    Leu Val Pro Glu Phe Leu Pro Ala Pro Glu Asp Val Glu Thr Asn Cys
        50                  55                  60


    Glu Trp Ser Ala Phe Ser Cys Phe Gln Lys Ala Gln Leu Lys Ser Ala
    65                  70                  75                  80


    Asn Thr Gly Asn Asn Glu Arg Ile Ile Asn Val Ser Ile Lys Lys Leu
                85                  90                  95


    Lys Arg Lys Pro Pro Ser Thr Asn Ala Gly Arg Arg Gln Lys His Arg
                100                 105                 110


    Leu Thr Cys Pro Ser Cys Asp Ser Tyr Glu Lys Lys Pro Pro Lys Glu
            115                 120                 125


    Phe Leu Glu Arg Phe Lys Ser Leu Leu Gln Lys Met Ile His Gln His
            130                 135                 140


    Leu Ser Ser Arg Thr His Gly Ser Glu Asp Ser
    145                 150                 155


    <210>   4
    <211>   180
    <212>   PRT
    <213>   Homo sapiens

    <400>   4

    Met Gln Ala Glu Gly Arg Gly Thr Gly Gly Ser Thr Gly Asp Ala Asp
    1               5                   10                  15


    Gly Pro Gly Gly Pro Gly Ile Pro Asp Gly Pro Gly Gly Asn Ala Gly
                20                  25                  30


    Gly Pro Gly Glu Ala Gly Ala Thr Gly Gly Arg Gly Pro Arg Gly Ala
            35                  40                  45


    Gly Ala Ala Arg Ala Ser Gly Pro Gly Gly Gly Ala Pro Arg Gly Pro
        50                  55                  60


    His Gly Gly Ala Ala Ser Gly Leu Asn Gly Cys Cys Arg Cys Gly Ala
    65                  70                  75                  80


    Arg Gly Pro Glu Ser Arg Leu Leu Glu Phe Tyr Leu Ala Met Pro Phe

```
                    85                      90                      95

        Ala Thr Pro Met Glu Ala Glu Leu Ala Arg Arg Ser Leu Ala Gln Asp
                    100                     105                     110

        Ala Pro Pro Leu Pro Val Pro Gly Val Leu Leu Lys Glu Phe Thr Val
                    115                     120                     125

        Ser Gly Asn Ile Leu Thr Ile Arg Leu Thr Ala Ala Asp His Arg Gln
                    130                     135                     140

        Leu Gln Leu Ser Ile Ser Ser Cys Leu Gln Gln Leu Ser Leu Leu Met
        145                     150                     155                     160

        Trp Ile Thr Gln Cys Phe Leu Pro Val Phe Leu Ala Gln Pro Pro Ser
                    165                     170                     175

        Gly Gln Arg Arg
                    180


        <210>  5
        <211>  142
        <212>  PRT
        <213>  Homo sapiens

        <400>  5

        Met Gly Ala Pro Thr Leu Pro Pro Ala Trp Gln Pro Phe Leu Lys Asp
        1                   5                   10                      15

        His Arg Ile Ser Thr Phe Lys Asn Trp Pro Phe Leu Glu Gly Cys Ala
                    20                      25                      30

        Cys Thr Pro Glu Arg Met Ala Glu Ala Gly Phe Ile His Cys Pro Thr
                    35                      40                      45

        Glu Asn Glu Pro Asp Leu Ala Gln Cys Phe Phe Cys Phe Lys Glu Leu
                    50                      55                      60

        Glu Gly Trp Glu Pro Asp Asp Asp Pro Ile Glu Glu His Lys Lys His
        65                      70                      75                      80

        Ser Ser Gly Cys Ala Phe Leu Ser Val Lys Lys Gln Phe Glu Glu Leu
                    85                      90                      95

        Thr Leu Gly Glu Phe Leu Lys Leu Asp Arg Glu Arg Ala Lys Asn Lys
                    100                     105                     110

        Ile Ala Lys Glu Thr Asn Asn Lys Lys Lys Glu Phe Glu Glu Thr Ala
```

<pre>
                 115                      120                      125


        Lys Lys Val Arg Arg Ala Ile Glu Gln Leu Ala Ala Met Asp
            130                 135                 140


<210>   6
<211>   630
<212>   PRT
<213>   Homo sapiens


<400>   6

Met Ala Leu Pro Thr Ala Arg Pro Leu Leu Gly Ser Cys Gly Thr Pro
1                   5                   10                  15


Ala Leu Gly Ser Leu Leu Phe Leu Leu Phe Ser Leu Gly Trp Val Gln
            20                  25                  30


Pro Ser Arg Thr Leu Ala Gly Glu Thr Gly Gln Glu Ala Ala Pro Leu
            35                  40                  45


Asp Gly Val Leu Ala Asn Pro Pro Asn Ile Ser Ser Leu Ser Pro Arg
        50                  55                  60


Gln Leu Leu Gly Phe Pro Cys Ala Glu Val Ser Gly Leu Ser Thr Glu
65                  70                  75                  80


Arg Val Arg Glu Leu Ala Val Ala Leu Ala Gln Lys Asn Val Lys Leu
                85                  90                  95


Ser Thr Glu Gln Leu Arg Cys Leu Ala His Arg Leu Ser Glu Pro Pro
            100                 105                 110


Glu Asp Leu Asp Ala Leu Pro Leu Asp Leu Leu Leu Phe Leu Asn Pro
            115                 120                 125


Asp Ala Phe Ser Gly Pro Gln Ala Cys Thr Arg Phe Phe Ser Arg Ile
            130                 135                 140


Thr Lys Ala Asn Val Asp Leu Leu Pro Arg Gly Ala Pro Glu Arg Gln
145                 150                 155                 160


Arg Leu Leu Pro Ala Ala Leu Ala Cys Trp Gly Val Arg Gly Ser Leu
                165                 170                 175


Leu Ser Glu Ala Asp Val Arg Ala Leu Gly Gly Leu Ala Cys Asp Leu
            180                 185                 190


Pro Gly Arg Phe Val Ala Glu Ser Ala Glu Val Leu Leu Pro Arg Leu
</pre>

|     | 195 |     |     |     | 200 |     |     |     | 205 |     |     |     |     |     |
|-----|-----|-----|-----|-----|-----|-----|-----|-----|-----|-----|-----|-----|-----|-----|

Val Ser Cys Pro Gly Pro Leu Asp Gln Asp Gln Gln Glu Ala Ala Arg
210 215 220

Ala Ala Leu Gln Gly Gly Gly Pro Pro Tyr Gly Pro Pro Ser Thr Trp
225 230 235 240

Ser Val Ser Thr Met Asp Ala Leu Arg Gly Leu Leu Pro Val Leu Gly
245 250 255

Gln Pro Ile Ile Arg Ser Ile Pro Gln Gly Ile Val Ala Ala Trp Arg
260 265 270

Gln Arg Ser Ser Arg Asp Pro Ser Trp Arg Gln Pro Glu Arg Thr Ile
275 280 285

Leu Arg Pro Arg Phe Arg Arg Glu Val Glu Lys Thr Ala Cys Pro Ser
290 295 300

Gly Lys Lys Ala Arg Glu Ile Asp Glu Ser Leu Ile Phe Tyr Lys Lys
305 310 315 320

Trp Glu Leu Glu Ala Cys Val Asp Ala Ala Leu Leu Ala Thr Gln Met
325 330 335

Asp Arg Val Asn Ala Ile Pro Phe Thr Tyr Glu Gln Leu Asp Val Leu
340 345 350

Lys His Lys Leu Asp Glu Leu Tyr Pro Gln Gly Tyr Pro Glu Ser Val
355 360 365

Ile Gln His Leu Gly Tyr Leu Phe Leu Lys Met Ser Pro Glu Asp Ile
370 375 380

Arg Lys Trp Asn Val Thr Ser Leu Glu Thr Leu Lys Ala Leu Leu Glu
385 390 395 400

Val Asn Lys Gly His Glu Met Ser Pro Gln Ala Pro Arg Arg Pro Leu
405 410 415

Pro Gln Val Ala Thr Leu Ile Asp Arg Phe Val Lys Gly Arg Gly Gln
420 425 430

Leu Asp Lys Asp Thr Leu Asp Thr Leu Thr Ala Phe Tyr Pro Gly Tyr
435 440 445

```
Leu Cys Ser Leu Ser Pro Glu Glu Leu Ser Ser Val Pro Pro Ser Ser
    450             455             460

Ile Trp Ala Val Arg Pro Gln Asp Leu Asp Thr Cys Asp Pro Arg Gln
465             470             475             480

Leu Asp Val Leu Tyr Pro Lys Ala Arg Leu Ala Phe Gln Asn Met Asn
                485             490             495

Gly Ser Glu Tyr Phe Val Lys Ile Gln Ser Phe Leu Gly Gly Ala Pro
                500             505             510

Thr Glu Asp Leu Lys Ala Leu Ser Gln Gln Asn Val Ser Met Asp Leu
            515             520             525

Ala Thr Phe Met Lys Leu Arg Thr Asp Ala Val Leu Pro Leu Thr Val
        530             535             540

Ala Glu Val Gln Lys Leu Leu Gly Pro His Val Glu Gly Leu Lys Ala
545             550             555             560

Glu Glu Arg His Arg Pro Val Arg Asp Trp Ile Leu Arg Gln Arg Gln
                565             570             575

Asp Asp Leu Asp Thr Leu Gly Leu Gly Leu Gln Gly Gly Ile Pro Asn
            580             585             590

Gly Tyr Leu Val Leu Asp Leu Ser Met Gln Glu Ala Leu Ser Gly Thr
        595             600             605

Pro Cys Leu Leu Gly Pro Gly Pro Val Leu Thr Val Leu Ala Leu Leu
    610             615             620

Leu Ala Ser Thr Leu Ala
625             630
```

```
<210>  7
<211>  1210
<212>  PRT
<213>  Homo sapiens

<400>  7
```

```
Met Arg Pro Ser Gly Thr Ala Gly Ala Ala Leu Leu Ala Leu Leu Ala
1               5               10              15

Ala Leu Cys Pro Ala Ser Arg Ala Leu Glu Glu Lys Lys Val Cys Gln
        20              25              30
```

```
Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
        50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
        115                 120                 125

Lys Thr Gly Leu Lys Glu Leu Pro Met Arg Asn Leu Gln Glu Ile Leu
    130                 135                 140

His Gly Ala Val Arg Phe Ser Asn Asn Pro Ala Leu Cys Asn Val Glu
145                 150                 155                 160

Ser Ile Gln Trp Arg Asp Ile Val Ser Ser Asp Phe Leu Ser Asn Met
            165                 170                 175

Ser Met Asp Phe Gln Asn His Leu Gly Ser Cys Gln Lys Cys Asp Pro
            180                 185                 190

Ser Cys Pro Asn Gly Ser Cys Trp Gly Ala Gly Glu Glu Asn Cys Gln
            195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
            245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285
```

65

```
Ala Thr Cys Val Lys Lys Cys Pro Arg Asn Tyr Val Val Thr Asp His
    290                 295                 300

Gly Ser Cys Val Arg Ala Cys Gly Ala Asp Ser Tyr Glu Met Glu Glu
    305                 310                 315                 320

Asp Gly Val Arg Lys Cys Lys Lys Cys Glu Gly Pro Cys Arg Lys Val
                325                 330                 335

Cys Asn Gly Ile Gly Ile Gly Glu Phe Lys Asp Ser Leu Ser Ile Asn
            340                 345                 350

Ala Thr Asn Ile Lys His Phe Lys Asn Cys Thr Ser Ile Ser Gly Asp
        355                 360                 365

Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
            515                 520                 525

Val Ser Arg Gly Arg Glu Cys Val Asp Lys Cys Asn Leu Leu Glu Gly
    530                 535                 540
```

66

Glu Pro Arg Glu Phe Val Glu Asn Ser Glu Cys Ile Gln Cys His Pro
545                 550             555                 560

Glu Cys Leu Pro Gln Ala Met Asn Ile Thr Cys Thr Gly Arg Gly Pro
                565             570                 575

Asp Asn Cys Ile Gln Cys Ala His Tyr Ile Asp Gly Pro His Cys Val
            580             585             590

Lys Thr Cys Pro Ala Gly Val Met Gly Glu Asn Asn Thr Leu Val Trp
        595             600             605

Lys Tyr Ala Asp Ala Gly His Val Cys His Leu Cys His Pro Asn Cys
    610             615             620

Thr Tyr Gly Cys Thr Gly Pro Gly Leu Glu Gly Cys Pro Thr Asn Gly
625             630             635                 640

Pro Lys Ile Pro Ser Ile Ala Thr Gly Met Val Gly Ala Leu Leu Leu
        645             650             655

Leu Leu Val Val Ala Leu Gly Ile Gly Leu Phe Met Arg Arg Arg His
        660             665             670

Ile Val Arg Lys Arg Thr Leu Arg Arg Leu Leu Gln Glu Arg Glu Leu
        675             680             685

Val Glu Pro Leu Thr Pro Ser Gly Glu Ala Pro Asn Gln Ala Leu Leu
    690             695             700

Arg Ile Leu Lys Glu Thr Glu Phe Lys Lys Ile Lys Val Leu Gly Ser
705             710             715             720

Gly Ala Phe Gly Thr Val Tyr Lys Gly Leu Trp Ile Pro Glu Gly Glu
            725             730             735

Lys Val Lys Ile Pro Val Ala Ile Lys Glu Leu Arg Glu Ala Thr Ser
        740             745             750

Pro Lys Ala Asn Lys Glu Ile Leu Asp Glu Ala Tyr Val Met Ala Ser
        755             760             765

Val Asp Asn Pro His Val Cys Arg Leu Leu Gly Ile Cys Leu Thr Ser
    770             775             780

Thr Val Gln Leu Ile Thr Gln Leu Met Pro Phe Gly Cys Leu Leu Asp

67

785                    790                    795                    800

Tyr Val Arg Glu His Lys Asp Asn Ile Gly Ser Gln Tyr Leu Leu Asn
                805                    810                    815

Trp Cys Val Gln Ile Ala Lys Gly Met Asn Tyr Leu Glu Asp Arg Arg
                820                    825                    830

Leu Val His Arg Asp Leu Ala Ala Arg Asn Val Leu Val Lys Thr Pro
        835                    840                    845

Gln His Val Lys Ile Thr Asp Phe Gly Leu Ala Lys Leu Leu Gly Ala
        850                    855                    860

Glu Glu Lys Glu Tyr His Ala Glu Gly Gly Lys Val Pro Ile Lys Trp
865                    870                    875                    880

Met Ala Leu Glu Ser Ile Leu His Arg Ile Tyr Thr His Gln Ser Asp
                885                    890                    895

Val Trp Ser Tyr Gly Val Thr Val Trp Glu Leu Met Thr Phe Gly Ser
                900                    905                    910

Lys Pro Tyr Asp Gly Ile Pro Ala Ser Glu Ile Ser Ser Ile Leu Glu
        915                    920                    925

Lys Gly Glu Arg Leu Pro Gln Pro Pro Ile Cys Thr Ile Asp Val Tyr
        930                    935                    940

Met Ile Met Val Lys Cys Trp Met Ile Asp Ala Asp Ser Arg Pro Lys
945                    950                    955                    960

Phe Arg Glu Leu Ile Ile Glu Phe Ser Lys Met Ala Arg Asp Pro Gln
                965                    970                    975

Arg Tyr Leu Val Ile Gln Gly Asp Glu Arg Met His Leu Pro Ser Pro
        980                    985                    990

Thr Asp Ser Asn Phe Tyr Arg Ala Leu Met Asp Glu Glu Asp Met Asp
        995                    1000                   1005

Asp Val Val Asp Ala Asp Glu Tyr Leu Ile Pro Gln Gln Gly Phe
        1010                   1015                   1020

Phe Ser Ser Pro Ser Thr Ser Arg Thr Pro Leu Leu Ser Ser Leu
        1025                   1030                   1035

```
Ser Ala  Thr Ser Asn Asn Ser  Thr Val Ala Cys Ile  Asp Arg Asn
    1040                1045                1050

Gly Leu  Gln Ser Cys Pro Ile  Lys Glu Asp Ser Phe  Leu Gln Arg
    1055                1060                1065

Tyr Ser  Ser Asp Pro Thr Gly  Ala Leu Thr Glu Asp  Ser Ile Asp
    1070                1075                1080

Asp Thr  Phe Leu Pro Val Pro  Glu Tyr Ile Asn Gln  Ser Val Pro
    1085                1090                1095

Lys Arg  Pro Ala Gly Ser Val  Gln Asn Pro Val Tyr  His Asn Gln
    1100                1105                1110

Pro Leu  Asn Pro Ala Pro Ser  Arg Asp Pro His Tyr  Gln Asp Pro
    1115                1120                1125

His Ser  Thr Ala Val Gly Asn  Pro Glu Tyr Leu Asn  Thr Val Gln
    1130                1135                1140

Pro Thr  Cys Val Asn Ser Thr  Phe Asp Ser Pro Ala  His Trp Ala
    1145                1150                1155

Gln Lys  Gly Ser His Gln Ile  Ser Leu Asp Asn Pro  Asp Tyr Gln
    1160                1165                1170

Gln Asp  Phe Phe Pro Lys Glu  Ala Lys Pro Asn Gly  Ile Phe Lys
    1175                1180                1185

Gly Ser  Thr Ala Glu Asn Ala  Glu Tyr Leu Arg Val  Ala Pro Gln
    1190                1195                1200

Ser Ser  Glu Phe Ile Gly Ala
    1205                1210


<210>  8
<211>  510
<212>  PRT
<213>  Homo sapiens

<400>  8

Met Glu Ser Arg Gly Arg Arg Cys Pro Glu Met Ile Ser Val Leu Gly
1                5                10                15

Pro Ile Ser Gly His Val Leu Lys Ala Val Phe Ser Arg Gly Asp Thr
            20                25                30
```

69

```
Pro Val Leu Pro His Glu Thr Arg Leu Leu Gln Thr Gly Ile His Val
    35                  40                  45

Arg Val Ser Gln Pro Ser Leu Ile Leu Val Ser Gln Tyr Thr Pro Asp
    50                  55                  60

Ser Thr Pro Cys His Arg Gly Asp Asn Gln Leu Gln Val Gln His Thr
65              70                  75                      80

Tyr Phe Thr Gly Ser Glu Val Glu Asn Val Ser Val Asn Val His Asn
                85                  90                  95

Pro Thr Gly Arg Ser Ile Cys Pro Ser Gln Glu Pro Met Ser Ile Tyr
            100                 105                 110

Val Tyr Ala Leu Pro Leu Lys Met Leu Asn Ile Pro Ser Ile Asn Val
        115                 120                 125

His His Tyr Pro Ser Ala Ala Glu Arg Lys His Arg His Leu Pro Val
    130                 135                 140

Ala Asp Ala Val Ile His Ala Ser Gly Lys Gln Met Trp Gln Ala Arg
145                 150                 155                 160

Leu Thr Val Ser Gly Leu Ala Trp Thr Arg Gln Gln Asn Gln Trp Lys
                165                 170                 175

Glu Pro Asp Val Tyr Tyr Thr Ser Ala Phe Val Phe Pro Thr Lys Asp
            180                 185                 190

Val Ala Leu Arg His Val Val Cys Ala His Glu Leu Val Cys Ser Met
        195                 200                 205

Glu Asn Thr Arg Ala Thr Lys Met Gln Val Ile Gly Asp Gln Tyr Val
    210                 215                 220

Lys Val Tyr Leu Glu Ser Phe Cys Glu Asp Val Pro Ser Gly Lys Leu
225                 230                 235                 240

Phe Met His Val Thr Leu Gly Ser Asp Val Glu Glu Asp Leu Thr Met
                245                 250                 255

Thr Arg Asn Pro Gln Pro Phe Met Arg Pro His Glu Arg Asn Gly Phe
            260                 265                 270

Thr Val Leu Cys Pro Lys Asn Met Ile Ile Lys Pro Gly Lys Ile Ser
    275                 280                 285
```

70

```
His Ile Met Leu Asp Val Ala Phe Thr Ser His Glu His Phe Gly Leu
    290             295             300

Leu Cys Pro Lys Ser Ile Pro Gly Leu Ser Ile Ser Gly Asn Leu Leu
    305             310             315             320

Met Asn Gly Gln Gln Ile Phe Leu Glu Val Gln Ala Ile Arg Glu Thr
                325             330             335

Val Glu Leu Arg Gln Tyr Asp Pro Val Ala Ala Leu Phe Phe Phe Asp
            340             345             350

Ile Asp Leu Leu Leu Gln Arg Gly Pro Gln Tyr Ser Glu His Pro Thr
        355             360             365

Phe Thr Ser Gln Tyr Arg Ile Gln Gly Lys Leu Glu Tyr Arg His Thr
    370             375             380

Trp Asp Arg His Asp Glu Gly Ala Ala Gln Gly Asp Asp Asp Val Trp
385             390             395             400

Thr Ser Gly Ser Asp Ser Asp Glu Glu Leu Val Thr Thr Glu Arg Lys
            405             410             415

Thr Pro Arg Val Thr Gly Gly Gly Ala Met Ala Gly Ala Ser Thr Ser
            420             425             430

Ala Gly Arg Lys Arg Lys Ser Ala Ser Ser Ala Thr Ala Cys Thr Ser
        435             440             445

Gly Val Met Thr Arg Gly Arg Leu Lys Ala Glu Ser Thr Val Ala Pro
    450             455             460

Glu Glu Asp Thr Asp Glu Asp Ser Asp Asn Glu Ile His Asn Pro Ala
465             470             475             480

Val Phe Thr Trp Pro Pro Trp Gln Ala Gly Ile Leu Ala Arg Asn Leu
            485             490             495

Val Pro Met Val Ala Thr Val Gln Gly Gln Asn Leu Lys Tyr
            500             505             510
```

```
<210>  9
<211>  944
<212>  PRT
<213>  Homo sapiens

<400>  9
```

```
Met Asp Lys Asp Arg Pro Gly Pro Pro Ala Leu Asp Asp Asn Met Glu
1               5               10              15

Glu Glu Val Pro Ser Thr Ser Val Val Gln Glu Gln Val Ser Ala Gly
            20              25              30

Asp Trp Glu Asn Val Leu Ile Glu Leu Ser Asp Ser Ser Ser Glu Lys
        35              40              45

Glu Ala Glu Asp Ala His Leu Glu Pro Ala Gln Lys Gly Thr Lys Arg
    50              55              60

Lys Arg Val Asp His Asp Ala Gly Gly Ser Ala Pro Ala Arg Pro Met
65              70              75              80

Leu Pro Pro Gln Pro Asp Leu Pro Gly Arg Glu Ala Ile Leu Arg Arg
            85              90              95

Phe Pro Leu Asp Leu Arg Thr Leu Leu Gln Ala Ile Gly Ala Ala Ala
        100             105             110

Thr Arg Ile Asp Thr Arg Ala Ile Asp Gln Phe Phe Gly Ser Gln Ile
        115             120             125

Ser Asn Thr Glu Met Tyr Ile Met Tyr Ala Met Ala Ile Arg Gln Ala
    130             135             140

Ile Arg Asp Arg Arg Arg Asn Pro Ala Ser Arg Arg Asp Gln Ala Lys
145             150             155             160

Trp Arg Leu Gln Thr Leu Ala Ala Gly Trp Pro Met Gly Tyr Gln Ala
            165             170             175

Tyr Ser Ser Trp Met Tyr Ser Tyr Thr Asp His Gln Thr Thr Pro Thr
            180             185             190

Phe Val His Leu Gln Ala Thr Leu Gly Cys Thr Gly Gly Arg Arg Cys
        195             200             205

His Val Thr Phe Ser Ala Gly Thr Phe Lys Leu Pro Arg Cys Thr Pro
    210             215             220

Gly Asp Arg Gln Trp Leu Tyr Val Gln Ser Ser Val Gly Asn Ile Val
225             230             235             240

Gln Ser Cys Asn Pro Arg Tyr Ser Ile Phe Phe Asp Tyr Met Ala Ile
            245             250             255
```

72

His Arg Ser Leu Thr Lys Ile Trp Glu Glu Val Leu Thr Pro Asp Gln
         260                  265             270

Arg Val Ser Phe Met Glu Phe Leu Gly Phe Leu Gln Arg Thr Asp Leu
         275                  280             285

Ser Tyr Ile Lys Ser Phe Val Ser Asp Ala Leu Gly Thr Thr Ser Ile
         290                  295             300

Gln Thr Pro Trp Ile Asp Asp Asn Pro Ser Thr Glu Thr Ala Gln Ala
305                  310             315            320

Trp Asn Ala Gly Phe Leu Arg Gly Arg Ala Tyr Gly Ile Asp Leu Leu
         325                  330             335

Arg Thr Glu Gly Glu His Val Glu Gly Ala Thr Gly Glu Thr Arg Glu
         340                  345             350

Glu Ser Glu Asp Thr Glu Ser Asp Gly Asp Asp Glu Asp Leu Pro Cys
         355                  360             365

Ile Val Ser Arg Gly Gly Pro Lys Val Lys Arg Pro Pro Ile Phe Ile
         370                  375             380

Arg Arg Leu His Arg Leu Leu Leu Met Arg Ala Gly Lys Arg Thr Glu
385                  390             395           400

Gln Gly Lys Glu Val Leu Glu Lys Ala Arg Gly Ser Thr Tyr Gly Thr
            405                410            415

Pro Arg Pro Pro Val Pro Lys Pro Arg Pro Glu Val Pro Gln Ser Asp
         420                  425            430

Glu Thr Ala Thr Ser His Gly Ser Ala Gln Val Pro Glu Pro Pro Thr
         435                  440            445

Ile His Leu Ala Ala Gln Gly Met Ala Tyr Pro Leu His Glu Gln His
         450                  455            460

Gly Met Ala Pro Cys Pro Val Ala Gln Ala Pro Pro Thr Pro Leu Pro
465                  470             475           480

Pro Val Ser Pro Gly Asp Gln Leu Pro Gly Val Phe Ser Asp Gly Arg
         485                  490            495

Val Ala Cys Ala Pro Val Pro Ala Pro Ala Gly Pro Ile Val Arg Pro

                        500                         505                         510

Trp Glu Pro Ser Leu Thr Gln Ala Ala Gly Gln Ala Phe Ala Pro Val
        515                 520                 525

Arg Pro Gln His Met Pro Val Glu Pro Val Pro Val Pro Thr Val Ala
        530                 535                 540

Leu Glu Arg Pro Val Tyr Pro Lys Pro Val Arg Pro Ala Pro Pro Lys
545                 550                 555                 560

Ile Ala Met Gln Gly Pro Gly Glu Thr Ser Gly Ile Arg Arg Ala Arg
                565                 570                 575

Glu Arg Trp Arg Pro Ala Pro Trp Thr Pro Asn Pro Pro Arg Ser Pro
                580                 585                 590

Ser Gln Met Ser Val Arg Asp Arg Leu Ala Arg Leu Arg Ala Glu Ala
            595                 600                 605

Gln Val Lys Gln Ala Ser Val Glu Val Gln Pro Pro Gln Leu Thr Gln
        610                 615                 620

Val Ser Pro Gln Gln Pro Met Glu Gly Pro Leu Val Pro Glu Gln Gln
625                 630                 635                 640

Met Phe Pro Gly Ala Pro Phe Ser Gln Val Ala Asp Val Val Arg Ala
                645                 650                 655

Pro Gly Val Pro Ala Met Gln Pro Gln Tyr Phe Asp Leu Pro Leu Ile
                660                 665                 670

Gln Pro Ile Ser Gln Gly Ala Pro Val Ala Pro Leu Arg Ala Ser Met
        675                 680                 685

Gly Pro Val Pro Pro Val Pro Ala Thr Gln Pro Gln Tyr Phe Asp Ile
        690                 695                 700

Pro Leu Thr Glu Pro Ile Asn Gln Gly Ala Ser Ala Ala His Phe Leu
705                 710                 715                 720

Pro Gln Gln Pro Met Glu Gly Pro Leu Val Pro Glu Gln Trp Met Phe
                725                 730                 735

Pro Gly Ala Ala Leu Ser Gln Ser Val Arg Pro Gly Val Ala Gln Ser
                740                 745                 750

```
Gln Tyr Phe Asp Leu Pro Leu Thr Gln Pro Ile Asn His Gly Ala Pro
        755                 760             765

Ala Ala His Phe Leu His Gln Pro Pro Met Glu Gly Pro Trp Val Pro
        770             775             780

Glu Gln Trp Met Phe Gln Gly Ala Pro Pro Ser Gln Gly Thr Asp Val
785             790             795                 800

Val Gln His Gln Leu Asp Ala Leu Gly Tyr Thr Leu His Gly Leu Asn
            805             810             815

His Pro Gly Val Pro Val Ser Pro Ala Val Asn Gln Tyr His Leu Ser
        820             825             830

Gln Ala Ala Phe Gly Leu Pro Ile Asp Glu Asp Glu Ser Gly Glu Gly
        835             840             845

Ser Asp Thr Ser Glu Pro Cys Glu Ala Leu Asp Leu Ser Ile His Gly
    850             855             860

Arg Pro Cys Pro Gln Ala Pro Glu Trp Pro Val Gln Glu Glu Gly Gly
865             870             875                 880

Gln Asp Ala Thr Glu Val Leu Asp Leu Ser Ile His Gly Arg Pro Arg
            885             890             895

Pro Arg Thr Pro Glu Trp Pro Val Gln Gly Glu Gly Gly Gln Asn Val
        900             905             910

Thr Gly Pro Glu Thr Arg Arg Val Val Val Ser Ala Val Val His Met
        915             920             925

Cys Gln Asp Asp Glu Phe Pro Asp Leu Gln Asp Pro Pro Asp Glu Ala
    930             935             940
```

```
<210>   10
<211>   641
<212>   PRT
<213>   Homo sapiens

<400>   10
```

```
Met Ser Asp Glu Gly Pro Gly Thr Gly Pro Gly Asn Gly Leu Gly Glu
1               5               10              15

Lys Gly Asp Thr Ser Gly Pro Glu Gly Ser Gly Gly Ser Gly Pro Gln
        20              25              30
```

Arg Arg Gly Gly Asp Asn His Gly Arg Gly Arg Gly Arg Gly Arg Gly
        35              40              45

Arg Gly Gly Gly Arg Pro Gly Ala Pro Gly Gly Ser Gly Ser Gly Pro
        50              55              60

Arg His Arg Asp Gly Val Arg Arg Pro Gln Lys Arg Pro Ser Cys Ile
65              70              75              80

Gly Cys Lys Gly Thr His Gly Gly Thr Gly Ala Gly Ala Gly Ala Gly
            85              90              95

Gly Ala Gly Ala Gly Gly Ala Gly Ala Gly Gly Gly Ala Gly Ala Gly
        100             105             110

Gly Gly Ala Gly Gly Ala Gly Gly Ala Gly Gly Ala Gly Ala Gly Gly
        115             120             125

Gly Ala Gly Ala Gly Gly Gly Ala Gly Gly Ala Gly Gly Ala Gly Ala
        130             135             140

Gly Gly Gly Ala Gly Ala Gly Gly Gly Ala Gly Gly Ala Gly Ala Gly
145             150             155             160

Gly Gly Ala Gly Gly Ala Gly Gly Ala Gly Ala Gly Gly Gly Ala Gly
            165             170             175

Ala Gly Gly Gly Ala Gly Gly Ala Gly Ala Gly Gly Gly Ala Gly Gly
            180             185             190

Ala Gly Gly Ala Gly Ala Gly Gly Gly Ala Gly Ala Gly Gly Ala Gly
            195             200             205

Gly Ala Gly Gly Ala Gly Ala Gly Gly Ala Gly Ala Gly Gly Gly Ala
        210             215             220

Gly Gly Ala Gly Gly Ala Gly Ala Gly Gly Ala Gly Ala Gly Gly Ala
225             230             235             240

Gly Ala Gly Gly Ala Gly Ala Gly Gly Ala Gly Gly Ala Gly Ala Gly
            245             250             255

Gly Ala Gly Gly Ala Gly Ala Gly Gly Ala Gly Gly Ala Gly Ala Gly
            260             265             270

Gly Gly Ala Gly Gly Ala Gly Ala Gly Gly Gly Ala Gly Gly Ala Gly
            275             280             285

```
Ala Gly Gly Ala Gly Gly Ala Gly Ala Gly Gly Ala Gly Gly Ala Gly
    290             295             300

Ala Gly Gly Ala Gly Gly Ala Gly Ala Gly Gly Gly Ala Gly Ala Gly
305             310             315             320

Gly Ala Gly Ala Gly Gly Gly Gly Arg Gly Arg Gly Gly Ser Gly Gly
            325             330             335

Arg Gly Arg Gly Gly Ser Gly Gly Arg Gly Arg Gly Gly Ser Gly Gly
            340             345             350

Arg Arg Gly Arg Gly Arg Glu Arg Ala Arg Gly Gly Ser Arg Glu Arg
            355             360             365

Ala Arg Gly Arg Gly Arg Gly Arg Gly Glu Lys Arg Pro Arg Ser Pro
    370             375             380

Ser Ser Gln Ser Ser Ser Ser Gly Ser Pro Pro Arg Arg Pro Pro Pro
385             390             395             400

Gly Arg Arg Pro Phe Phe His Pro Val Gly Glu Ala Asp Tyr Phe Glu
            405             410             415

Tyr His Gln Glu Gly Gly Pro Asp Gly Glu Pro Asp Val Pro Pro Gly
            420             425             430

Ala Ile Glu Gln Gly Pro Ala Asp Asp Pro Gly Glu Gly Pro Ser Thr
    435             440             445

Gly Pro Arg Gly Gln Gly Asp Gly Gly Arg Arg Lys Lys Gly Gly Trp
    450             455             460

Phe Gly Lys His Arg Gly Gln Gly Gly Ser Asn Pro Lys Phe Glu Asn
465             470             475             480

Ile Ala Glu Gly Leu Arg Ala Leu Leu Ala Arg Ser His Val Glu Arg
            485             490             495

Thr Thr Asp Glu Gly Thr Trp Val Ala Gly Val Phe Val Tyr Gly Gly
            500             505             510

Ser Lys Thr Ser Leu Tyr Asn Leu Arg Arg Gly Thr Ala Leu Ala Ile
    515             520             525

Pro Gln Cys Arg Leu Thr Pro Leu Ser Arg Leu Pro Phe Gly Met Ala
    530             535             540
```

77

Pro Gly Pro Gly Pro Gln Pro Gly Pro Leu Arg Glu Ser Ile Val Cys
545                 550                 555                 560

Tyr Phe Met Val Phe Leu Gln Thr His Ile Phe Ala Glu Val Leu Lys
                565                 570                 575

Asp Ala Ile Lys Asp Leu Val Met Thr Lys Pro Ala Pro Thr Cys Asn
            580                 585                 590

Ile Arg Val Thr Val Cys Ser Phe Asp Asp Gly Val Asp Leu Pro Pro
            595                 600                 605

Trp Phe Pro Pro Met Val Glu Gly Ala Ala Ala Glu Gly Asp Asp Gly
    610                 615                 620

Asp Asp Gly Asp Glu Gly Gly Asp Gly Asp Glu Gly Glu Glu Gly Gln
625                 630                 635                 640

Glu

<210>  11
<211>  505
<212>  PRT
<213>  Homo sapiens

<400>  11

Met Ser Leu Trp Leu Pro Ser Glu Ala Thr Val Tyr Leu Pro Pro Val
1               5                   10                  15

Pro Val Ser Lys Val Val Ser Thr Asp Glu Tyr Val Ala Arg Thr Asn
            20                  25                  30

Ile Tyr Tyr His Ala Gly Thr Ser Arg Leu Leu Ala Val Gly His Pro
            35                  40                  45

Tyr Phe Pro Ile Lys Lys Pro Asn Asn Asn Lys Ile Leu Val Pro Lys
    50                  55                  60

Val Ser Gly Leu Gln Tyr Arg Val Phe Arg Ile His Leu Pro Asp Pro
65                  70                  75                  80

Asn Lys Phe Gly Phe Pro Asp Thr Ser Phe Tyr Asn Pro Asp Thr Gln
                85                  90                  95

Arg Leu Val Trp Ala Cys Val Gly Val Glu Val Gly Arg Gly Gln Pro
                100                 105                 110

Leu Gly Val Gly Ile Ser Gly His Pro Leu Leu Asn Lys Leu Asp Asp
115 120 125

Thr Glu Asn Ala Ser Ala Tyr Ala Ala Asn Ala Gly Val Asp Asn Arg
130 135 140

Glu Cys Ile Ser Met Asp Tyr Lys Gln Thr Gln Leu Cys Leu Ile Gly
145 150 155 160

Cys Lys Pro Pro Ile Gly Glu His Trp Gly Lys Gly Ser Pro Cys Thr
165 170 175

Asn Val Ala Val Asn Pro Gly Asp Cys Pro Pro Leu Glu Leu Ile Asn
180 185 190

Thr Val Ile Gln Asp Gly Asp Met Val Asp Thr Gly Phe Gly Ala Met
195 200 205

Asp Phe Thr Thr Leu Gln Ala Asn Lys Ser Glu Val Pro Leu Asp Ile
210 215 220

Cys Thr Ser Ile Cys Lys Tyr Pro Asp Tyr Ile Lys Met Val Ser Glu
225 230 235 240

Pro Tyr Gly Asp Ser Leu Phe Phe Tyr Leu Arg Arg Glu Gln Met Phe
245 250 255

Val Arg His Leu Phe Asn Arg Ala Gly Ala Val Gly Glu Asn Val Pro
260 265 270

Asp Asp Leu Tyr Ile Lys Gly Ser Gly Ser Thr Ala Asn Leu Ala Ser
275 280 285

Ser Asn Tyr Phe Pro Thr Pro Ser Gly Ser Met Val Thr Ser Asp Ala
290 295 300

Gln Ile Phe Asn Lys Pro Tyr Trp Leu Gln Arg Ala Gln Gly His Asn
305 310 315 320

Asn Gly Ile Cys Trp Gly Asn Gln Leu Phe Val Thr Val Val Asp Thr
325 330 335

Thr Arg Ser Thr Asn Met Ser Leu Cys Ala Ala Ile Ser Thr Ser Glu
340 345 350

Thr Thr Tyr Lys Asn Thr Asn Phe Lys Glu Tyr Leu Arg His Gly Glu

```
                355                      360                      365


        Glu Tyr Asp Leu Gln Phe Ile Phe Gln Leu Cys Lys Ile Thr Leu Thr
            370                 375                 380


        Ala Asp Val Met Thr Tyr Ile His Ser Met Asn Ser Thr Ile Leu Glu
        385                 390                 395                 400


        Asp Trp Asn Phe Gly Leu Gln Pro Pro Pro Gly Gly Thr Leu Glu Asp
                        405                 410                 415


        Thr Tyr Arg Phe Val Thr Ser Gln Ala Ile Ala Cys Gln Lys His Thr
                        420                 425                 430


        Pro Pro Ala Pro Lys Glu Asp Pro Leu Lys Lys Tyr Thr Phe Trp Glu
                    435                 440                 445


        Val Asn Leu Lys Glu Lys Phe Ser Ala Asp Leu Asp Gln Phe Pro Leu
                450                 455                 460


        Gly Arg Lys Phe Leu Leu Gln Ala Gly Leu Lys Ala Lys Pro Lys Phe
        465                 470                 475                 480


        Thr Leu Gly Lys Arg Lys Ala Thr Pro Thr Thr Ser Ser Thr Ser Thr
                        485                 490                 495


        Thr Ala Lys Arg Lys Lys Arg Lys Leu
                    500                 505


        <210>  12
        <211>  1718
        <212>  PRT
        <213>  Homo sapiens

        <400>  12

        Met Asn Gln Asn Thr Thr Glu Pro Val Ala Ala Thr Glu Thr Leu Ala
        1                   5                   10                  15


        Glu Val Pro Glu His Val Leu Arg Gly Leu Pro Glu Glu Val Arg Leu
                    20                  25                  30


        Phe Pro Ser Ala Val Asp Lys Thr Arg Ile Gly Val Trp Ala Thr Lys
                    35                  40                  45


        Pro Ile Leu Lys Gly Lys Lys Phe Gly Pro Phe Val Gly Asp Lys Lys
                50                  55                  60


        Lys Arg Ser Gln Val Lys Asn Asn Val Tyr Met Trp Glu Val Tyr Tyr
```

```
            65                      70                      75                      80

            Pro Asn Leu Gly Trp Met Cys Ile Asp Ala Thr Asp Pro Glu Lys Gly
                            85                  90                  95

            Asn Trp Leu Arg Tyr Val Asn Trp Ala Cys Ser Gly Glu Glu Gln Asn
                            100                 105                 110

            Leu Phe Pro Leu Glu Ile Asn Arg Ala Ile Tyr Tyr Lys Thr Leu Lys
                            115                 120                 125

            Pro Ile Ala Pro Gly Glu Glu Leu Leu Val Trp Tyr Asn Gly Glu Asp
                    130                 135                 140

            Asn Pro Glu Ile Ala Ala Ala Ile Glu Glu Glu Arg Ala Ser Ala Arg
            145                 150                 155                 160

            Ser Lys Arg Ser Ser Pro Lys Ser Arg Lys Gly Lys Lys Lys Ser Gln
                            165                 170                 175

            Glu Asn Lys Asn Lys Gly Asn Lys Ile Gln Asp Ile Gln Leu Lys Thr
                            180                 185                 190

            Ser Glu Pro Asp Phe Thr Ser Ala Asn Met Arg Asp Ser Ala Glu Gly
                    195                 200                 205

            Pro Lys Glu Asp Glu Glu Lys Pro Ser Ala Ser Ala Leu Glu Gln Pro
                    210                 215                 220

            Ala Thr Leu Gln Glu Val Ala Ser Gln Glu Val Pro Pro Glu Leu Ala
            225                 230                 235                 240

            Thr Pro Ala Pro Ala Trp Glu Pro Gln Pro Glu Pro Asp Glu Arg Leu
                            245                 250                 255

            Glu Ala Ala Ala Cys Glu Val Asn Asp Leu Gly Glu Glu Glu Glu Glu
                    260                 265                 270

            Glu Glu Glu Glu Asp Glu Glu Glu Glu Glu Asp Asp Asp Asp Glu
                    275                 280                 285

            Leu Glu Asp Glu Gly Glu Glu Glu Ala Ser Met Pro Asn Glu Asn Ser
                    290                 295                 300

            Val Lys Glu Pro Glu Ile Arg Cys Asp Glu Lys Pro Glu Asp Leu Leu
            305                 310                 315                 320
```

```
Glu Glu Pro Lys Thr Thr Ser Glu Glu Thr Leu Glu Asp Cys Ser Glu
            325             330             335

Val Thr Pro Ala Met Gln Ile Pro Arg Thr Lys Glu Glu Ala Asn Gly
            340             345             350

Asp Val Phe Glu Thr Phe Met Phe Pro Cys Gln His Cys Glu Arg Lys
            355             360             365

Phe Thr Thr Lys Gln Gly Leu Glu Arg His Met His Ile His Ile Ser
    370             375             380

Thr Val Asn His Ala Phe Lys Cys Lys Tyr Cys Gly Lys Ala Phe Gly
385             390             395             400

Thr Gln Ile Asn Arg Arg Arg His Glu Arg Arg His Glu Ala Gly Leu
            405             410             415

Lys Arg Lys Pro Ser Gln Thr Leu Gln Pro Ser Glu Asp Leu Ala Asp
            420             425             430

Gly Lys Ala Ser Gly Glu Asn Val Ala Ser Lys Asp Asp Ser Ser Pro
            435             440             445

Pro Ser Leu Gly Pro Asp Cys Leu Ile Met Asn Ser Glu Lys Ala Ser
    450             455             460

Gln Asp Thr Ile Asn Ser Ser Val Val Glu Glu Asn Gly Glu Val Lys
465             470             475             480

Glu Leu His Pro Cys Lys Tyr Cys Lys Lys Val Phe Gly Thr His Thr
            485             490             495

Asn Met Arg Arg His Gln Arg Arg Val His Glu Arg His Leu Ile Pro
            500             505             510

Lys Gly Val Arg Arg Lys Gly Gly Leu Glu Glu Pro Gln Pro Pro Ala
            515             520             525

Glu Gln Ala Gln Ala Thr Gln Asn Val Tyr Val Pro Ser Thr Glu Pro
            530             535             540

Glu Glu Glu Gly Glu Ala Asp Asp Val Tyr Ile Met Asp Ile Ser Ser
545             550             555             560

Asn Ile Ser Glu Asn Leu Asn Tyr Tyr Ile Asp Gly Lys Ile Gln Thr
            565             570             575
```

```
Asn Asn Asn Thr Ser Asn Cys Asp Val Ile Glu Met Glu Ser Ala Ser
            580             585             590

Ala Asp Leu Tyr Gly Ile Asn Cys Leu Leu Thr Pro Val Thr Val Glu
            595             600             605

Ile Thr Gln Asn Ile Lys Thr Thr Gln Val Pro Val Thr Glu Asp Leu
            610             615             620

Pro Lys Glu Pro Leu Gly Ser Thr Asn Ser Glu Ala Lys Lys Arg Arg
625             630             635             640

Thr Ala Ser Pro Pro Ala Leu Pro Lys Ile Lys Ala Glu Thr Asp Ser
            645             650             655

Asp Pro Met Val Pro Ser Cys Ser Leu Ser Leu Pro Leu Ser Ile Ser
            660             665             670

Thr Thr Glu Ala Val Ser Phe His Lys Glu Lys Ser Val Tyr Leu Ser
            675             680             685

Ser Lys Leu Lys Gln Leu Leu Gln Thr Gln Asp Lys Leu Thr Pro Ala
            690             695             700

Gly Ile Ser Ala Thr Glu Ile Ala Lys Leu Gly Pro Val Cys Val Ser
705             710             715             720

Ala Pro Ala Ser Met Leu Pro Val Thr Ser Ser Arg Phe Lys Arg Arg
            725             730             735

Thr Ser Ser Pro Pro Ser Ser Pro Gln His Ser Pro Ala Leu Arg Asp
            740             745             750

Phe Gly Lys Pro Ser Asp Gly Lys Ala Ala Trp Thr Asp Ala Gly Leu
            755             760             765

Thr Ser Lys Lys Ser Lys Leu Glu Ser His Ser Asp Ser Pro Ala Trp
            770             775             780

Ser Leu Ser Gly Arg Asp Glu Arg Glu Thr Val Ser Pro Pro Cys Phe
785             790             795             800

Asp Glu Tyr Lys Met Ser Lys Glu Trp Thr Ala Ser Ser Ala Phe Ser
            805             810             815

Ser Val Cys Asn Gln Gln Pro Leu Asp Leu Ser Ser Gly Val Lys Gln
            820             825             830
```

```
Lys Ala Glu Gly Thr Gly Lys Thr Pro Val Gln Trp Glu Ser Val Leu
        835                 840                 845


Asp Leu Ser Val His Lys Lys His Cys Ser Asp Ser Glu Gly Lys Glu
        850                 855                 860


Phe Lys Glu Ser His Ser Val Gln Pro Thr Cys Ser Ala Val Lys Lys
865                 870                 875                 880


Arg Lys Pro Thr Thr Cys Met Leu Gln Lys Val Leu Leu Asn Glu Tyr
                885                 890                 895


Asn Gly Ile Asp Leu Pro Val Glu Asn Pro Ala Asp Gly Thr Arg Ser
            900                 905                 910


Pro Ser Pro Cys Lys Ser Leu Glu Ala Gln Pro Asp Pro Asp Leu Gly
        915                 920                 925


Pro Gly Ser Gly Phe Pro Ala Pro Thr Val Glu Ser Thr Pro Asp Val
        930                 935                 940


Cys Pro Ser Ser Pro Ala Leu Gln Thr Pro Ser Leu Ser Ser Gly Gln
945                 950                 955                 960


Leu Pro Pro Leu Leu Ile Pro Thr Asp Pro Ser Ser Pro Pro Pro Cys
                965                 970                 975


Pro Pro Val Leu Thr Val Ala Thr Pro Pro Pro Pro Leu Leu Pro Thr
                980                 985                 990


Val Pro Leu Pro Ala Pro Ser Ser Ser Ala Ser Pro His Pro Cys Pro
        995                 1000                1005


Ser Pro Leu Ser Asn Ala Thr Ala Gln Ser Pro Leu Pro Ile Leu
    1010                1015                1020


Ser Pro Thr Val Ser Pro Ser Pro Ser Pro Ile Pro Pro Val Glu
    1025                1030                1035


Pro Leu Met Ser Ala Ala Ser Pro Gly Pro Pro Thr Leu Ser Ser
    1040                1045                1050


Ser Ser Ser Ser Ser Ser Ser Ser Ser Ser Phe Ser Ser Ser Ser
    1055                1060                1065


Ser Ser Ser Ser Pro Ser Pro Pro Pro Leu Ser Ala Ile Ser Ser
```

|      | 1070 |     |     |     | 1075 |     |     |     | 1080 |     |     |     |
|------|------|-----|-----|-----|------|-----|-----|-----|------|-----|-----|-----|

Val Val Ser Ser Gly Asp Asn Leu Glu Ala Ser Leu Pro Met Ile
    1085           1090          1095

Ser Phe Lys Gln Glu Glu Leu Glu Asn Glu Gly Leu Lys Pro Arg
    1100           1105          1110

Glu Glu Pro Gln Ser Ala Ala Glu Gln Asp Val Val Val Gln Glu
    1115           1120          1125

Thr Phe Asn Lys Asn Phe Val Cys Asn Val Cys Glu Ser Pro Phe
    1130           1135          1140

Leu Ser Ile Lys Asp Leu Thr Lys His Leu Ser Ile His Ala Glu
    1145           1150          1155

Glu Trp Pro Phe Lys Cys Glu Phe Cys Val Gln Leu Phe Lys Asp
    1160           1165          1170

Lys Thr Asp Leu Ser Glu His Arg Phe Leu Leu His Gly Val Gly
    1175           1180          1185

Asn Ile Phe Val Cys Ser Val Cys Lys Lys Glu Phe Ala Phe Leu
    1190           1195          1200

Cys Asn Leu Gln Gln His Gln Arg Asp Leu His Pro Asp Lys Val
    1205           1210          1215

Cys Thr His His Glu Phe Glu Ser Gly Thr Leu Arg Pro Gln Asn
    1220           1225          1230

Phe Thr Asp Pro Ser Lys Ala His Val Glu His Met Gln Ser Leu
    1235           1240          1245

Pro Glu Asp Pro Leu Glu Thr Ser Lys Glu Glu Glu Glu Leu Asn
    1250           1255          1260

Asp Ser Ser Glu Glu Leu Tyr Thr Thr Ile Lys Ile Met Ala Ser
    1265           1270          1275

Gly Ile Lys Thr Lys Asp Pro Asp Val Arg Leu Gly Leu Asn Gln
    1280           1285          1290

His Tyr Pro Ser Phe Lys Pro Pro Pro Phe Gln Tyr His His Arg
    1295           1300          1305

```
Asn Pro Met Gly Ile Gly Val    Thr Ala Thr Asn Phe    Thr Thr His
    1310            1315                1320

Asn Ile Pro Gln Thr Phe Thr    Thr Ala Ile Arg Cys    Thr Lys Cys
    1325            1330                1335

Gly Lys Gly Val Asp Asn Met    Pro Glu Leu His Lys    His Ile Leu
    1340            1345                1350

Ala Cys Ala Ser Ala Ser Asp    Lys Lys Arg Tyr Thr    Pro Lys Lys
    1355            1360                1365

Asn Pro Val Pro Leu Lys Gln    Thr Val Gln Pro Lys    Asn Gly Val
    1370            1375                1380

Val Val Leu Asp Asn Ser Gly    Lys Asn Ala Phe Arg    Arg Met Gly
    1385            1390                1395

Gln Pro Lys Arg Leu Asn Phe    Ser Val Glu Leu Ser    Lys Met Ser
    1400            1405                1410

Ser Asn Lys Leu Lys Leu Asn    Ala Leu Lys Lys Lys    Asn Gln Leu
    1415            1420                1425

Val Gln Lys Ala Ile Leu Gln    Lys Asn Lys Ser Ala    Lys Gln Lys
    1430            1435                1440

Ala Asp Leu Lys Asn Ala Cys    Glu Ser Ser Ser His    Ile Cys Pro
    1445            1450                1455

Tyr Cys Asn Arg Glu Phe Thr    Tyr Ile Gly Ser Leu    Asn Lys His
    1460            1465                1470

Ala Ala Phe Ser Cys Pro Lys    Lys Pro Leu Ser Pro    Pro Lys Lys
    1475            1480                1485

Lys Val Ser His Ser Ser Lys    Lys Gly Gly His Ser    Ser Pro Ala
    1490            1495                1500

Ser Ser Asp Lys Asn Ser Asn    Ser Asn His Arg Arg    Arg Thr Ala
    1505            1510                1515

Asp Ala Glu Ile Lys Met Gln    Ser Met Gln Thr Pro    Leu Gly Lys
    1520            1525                1530

Thr Arg Ala Arg Ser Ser Gly    Pro Thr Gln Val Pro    Leu Pro Ser
    1535            1540                1545
```

86

```
Ser  Ser  Phe  Arg  Ser  Lys  Gln  Asn  Val  Lys  Phe  Ala  Ala  Ser  Val
     1550                1555               1560

Lys  Ser  Lys  Lys  Pro  Ser  Ser  Ser  Ser  Leu  Arg  Asn  Ser  Ser  Pro
     1565                1570               1575

Ile  Arg  Met  Ala  Lys  Ile  Thr  His  Val  Glu  Gly  Lys  Lys  Pro  Lys
     1580                1585               1590

Ala  Val  Ala  Lys  Asn  His  Ser  Ala  Gln  Leu  Ser  Ser  Lys  Thr  Ser
     1595                1600               1605

Arg  Ser  Leu  His  Val  Arg  Val  Gln  Lys  Ser  Lys  Ala  Val  Leu  Gln
     1610                1615               1620

Ser  Lys  Ser  Thr  Leu  Ala  Ser  Lys  Lys  Arg  Thr  Asp  Arg  Phe  Asn
     1625                1630               1635

Ile  Lys  Ser  Arg  Glu  Arg  Ser  Gly  Gly  Pro  Val  Thr  Arg  Ser  Leu
     1640                1645               1650

Gln  Leu  Ala  Ala  Ala  Ala  Asp  Leu  Ser  Glu  Asn  Lys  Arg  Glu  Asp
     1655                1660               1665

Gly  Ser  Ala  Lys  Gln  Glu  Leu  Lys  Asp  Phe  Ser  Tyr  Ser  Leu  Arg
     1670                1675               1680

Leu  Ala  Ser  Arg  Cys  Ser  Pro  Pro  Ala  Ala  Pro  Tyr  Ile  Thr  Arg
     1685                1690               1695

Gln  Tyr  Arg  Lys  Val  Lys  Ala  Pro  Ala  Ala  Ala  Gln  Phe  Gln  Gly
     1700                1705               1710

Pro  Phe  Phe  Lys  Glu
     1715


<210>  13
<211>  230
<212>  PRT
<213>  Homo sapiens

<400>  13

Met  Glu  Gly  Gln  Arg  Trp  Leu  Pro  Leu  Glu  Ala  Asn  Pro  Glu  Val  Thr
1                   5                   10                  15

Asn  Gln  Phe  Leu  Lys  Gln  Leu  Gly  Leu  His  Pro  Asn  Trp  Gln  Phe  Val
          20                  25                  30
```

```
Asp Val Tyr Gly Met Asp Pro Glu Leu Leu Ser Met Val Pro Arg Pro
        35              40              45

Val Cys Ala Val Leu Leu Leu Phe Pro Ile Thr Glu Lys Tyr Glu Val
        50              55              60

Phe Arg Thr Glu Glu Glu Glu Lys Ile Lys Ser Gln Gly Gln Asp Val
65              70              75              80

Thr Ser Ser Val Tyr Phe Met Lys Gln Thr Ile Ser Asn Ala Cys Gly
                85              90              95

Thr Ile Gly Leu Ile His Ala Ile Ala Asn Asn Lys Asp Lys Met His
            100             105             110

Phe Glu Ser Gly Ser Thr Leu Lys Lys Phe Leu Glu Glu Ser Val Ser
        115             120             125

Met Ser Pro Glu Glu Arg Ala Arg Tyr Leu Glu Asn Tyr Asp Ala Ile
    130             135             140

Arg Val Thr His Glu Thr Ser Ala His Glu Gly Gln Thr Glu Ala Pro
145             150             155             160

Ser Ile Asp Glu Lys Val Asp Leu His Phe Ile Ala Leu Val His Val
            165             170             175

Asp Gly His Leu Tyr Glu Leu Asp Gly Arg Lys Pro Phe Pro Ile Asn
            180             185             190

His Gly Glu Thr Ser Asp Glu Thr Leu Leu Glu Asp Ala Ile Glu Val
        195             200             205

Cys Lys Lys Phe Met Glu Arg Asp Pro Asp Glu Leu Arg Phe Asn Ala
    210             215             220

Ile Ala Leu Ser Ala Ala
225             230
```

```
<210>  14
<211>  244
<212>  PRT
<213>  Homo sapiens

<400>  14
```

```
Met Asn Gly Pro Ala Asp Gly Glu Val Asp Tyr Lys Lys Lys Tyr Arg
1               5               10              15
```

Asn Leu Lys Arg Lys Leu Lys Phe Leu Ile Tyr Glu His Glu Cys Phe
20 25 30

Gln Glu Glu Leu Arg Lys Ala Gln Arg Lys Leu Leu Lys Val Ser Arg
35 40 45

Asp Lys Ser Phe Leu Leu Asp Arg Leu Leu Gln Tyr Glu Asn Val Asp
50 55 60

Glu Asp Ser Ser Asp Ser Asp Ala Thr Ala Ser Ser Asp Asn Ser Glu
65 70 75 80

Thr Glu Gly Thr Pro Lys Leu Ser Asp Thr Pro Ala Pro Lys Arg Lys
85 90 95

Arg Ser Pro Pro Leu Gly Gly Ala Pro Ser Pro Ser Ser Leu Ser Leu
100 105 110

Pro Pro Ser Thr Gly Phe Pro Leu Gln Ala Ser Gly Val Pro Ser Pro
115 120 125

Tyr Leu Ser Ser Leu Ala Ser Ser Arg Tyr Pro Pro Phe Pro Ser Asp
130 135 140

Tyr Leu Ala Leu Gln Leu Pro Glu Pro Ser Pro Leu Arg Pro Lys Arg
145 150 155 160

Glu Lys Arg Pro Arg Leu Pro Arg Lys Leu Lys Met Ala Val Gly Pro
165 170 175

Pro Asp Cys Pro Val Gly Gly Pro Leu Thr Phe Pro Gly Arg Gly Ser
180 185 190

Gly Ala Gly Val Gly Thr Thr Leu Thr Pro Leu Pro Pro Pro Lys Met
195 200 205

Pro Pro Pro Thr Ile Leu Ser Thr Val Pro Arg Gln Met Phe Ser Asp
210 215 220

Ala Gly Ser Gly Asp Asp Ala Leu Asp Gly Asp Asp Asp Leu Val Ile
225 230 235 240

Asp Ile Pro Glu

<210> 15
<211> 271

<212> PRT
<213> Homo sapiens

<400> 15

Met Asn Leu Leu Gly Ser Arg Arg Val Phe Ser Lys Lys Cys Arg Leu
1               5                   10                  15

Val Lys Phe Ser Met Val Ala Leu Val Ser Ala Thr Met Ala Val Thr
            20                  25                  30

Thr Val Thr Leu Glu Asn Thr Ala Leu Ala Arg Gln Thr Gln Val Ser
        35                  40                  45

Asn Asp Val Val Leu Asn Asp Gly Ala Ser Lys Tyr Leu Asn Glu Ala
    50                  55                  60

Leu Ala Trp Thr Phe Asn Asp Ser Pro Asn Tyr Tyr Lys Thr Leu Gly
65                  70                  75                  80

Thr Ser Gln Ile Thr Pro Ala Leu Phe Pro Lys Ala Gly Asp Ile Leu
            85                  90                  95

Tyr Ser Lys Leu Asp Glu Leu Gly Arg Thr Arg Thr Ala Arg Gly Thr
            100                 105                 110

Leu Thr Tyr Ala Asn Val Glu Gly Ser Tyr Gly Val Arg Gln Ser Phe
        115                 120                 125

Gly Lys Asn Gln Asn Pro Ala Gly Trp Thr Gly Asn Pro Asn His Val
    130                 135                 140

Lys Tyr Lys Ile Glu Trp Leu Asn Gly Leu Ser Tyr Val Gly Asp Phe
145                 150                 155                 160

Trp Asn Arg Ser His Leu Ile Ala Asp Ser Leu Gly Gly Asp Ala Leu
            165                 170                 175

Arg Val Asn Ala Val Thr Gly Thr Arg Thr Gln Asn Val Gly Gly Arg
            180                 185                 190

Asp Gln Lys Gly Gly Met Arg Tyr Thr Glu Gln Arg Ala Gln Glu Trp
    195                 200                 205

Leu Glu Ala Asn Arg Asp Gly Tyr Leu Tyr Tyr Glu Val Ala Pro Ile
    210                 215                 220

Tyr Asn Ala Asp Glu Leu Ile Pro Arg Ala Val Val Val Ser Met Gln
225                 230                 235                 240

90

```
Ser Ser Asp Asn Thr Ile Asn Glu Lys Val Leu Val Tyr Asn Thr Ala
            245                 250                      255


Asn Gly Tyr Thr Ile Asn Tyr His Asn Gly Thr Pro Thr Gln Lys
        260                 265                 270
```